# FASCICULE DE BREVET EUROPEEN

(11) **EP 2 859 008 B1**
(45) Date de publication et mention de la délivrance du brevet: **22.02.2017**
(21) Numéro de dépôt: 13716756.5
(22) Date de dépôt: 04.04.2013
(51) Int. Cl.: C07H 21/00, C07H 21/04, G01N 33/50

(54) **OLIGONUCLEOTIDES MODIFIES COMPENANT DES FONCTIONS THIOL ET LEUR UTILISATION POUR LA DETECTION D'ACIDES NUCLEIQUES**
MODIFIZIERTE OLIGONUKLEOTIDE MIT THIOL-FUNKTIONEN UND VERWENDUNG DAVON ZUM NACHWEIS VON NUKLEINSÄUREN
MODIFIED OLIGONUCLEOTIDES COMPRISING THIOL FUNCTIONS AND THE USE OF SAME FOR THE DETECTION OF NUCLEIC ACIDS

(30) Priorité: 04.04.2012 FR 1253122
(43) Date de publication de la demande: 15.04.2015
(73) Titulaire: Etablissement Français Du Sang (EFS), 93218 La Plaine Saint Denis (FR); Centre National de la Recherche Scientifique (C.N.R.S.), 75794 Paris Cedex 16 (FR); Universite de Montpellier 1, 34967 Montpellier Cedex 2 (FR); Université Claude Bernard Lyon 1, 69622 Villeurbanne, Cedex (FR)
(72) Inventeur: FOURNIER-WIRTH, Chantal, F-93218 La Plaine Saint Denis Cedex (FR); LEREAU, Myriam, F-93218 La Plaine Saint Denis Cedex (FR); CANTALOUBE, Jean-François, F-93218 La Plaine Saint Denis Cedex (FR); VASSEUR, Jean-Jacques, F-34980 Combaillaux (FR); MORVAN, François, F-34170 Castelnau Le Lez (FR); MEYER, Albert, F-34470 Perols (FR); MAYEN, Julie, F-84270 Vedene (FR); CHAIX, Carole, F-69970 Chaponnay (FR); FARRE, Carole, F-69003 Lyon (FR)
(74) Mandataire: Hirsch & Associés
(86) Numéro de dépôt international: PCT/EP2013/057150
(87) Numéro de publication internationale: WO 2013/150122

(56) Documents cités:
- EP-A1- 0 523 978
- WO-A1-2005/065405
- JP-A- 6 041 183
- US-B2- 7 601 848

## Description

### DOMAINE TECHNIQUE

La présente invention concerne un oligonucléotide modifié possédant deux ou plusieurs fonctions thiol tel que défini à la revendication 1, apte à être immobilisé sur une surface d'or ou sur une surface greffée, notamment une surface comportant au moins une double liaison carbone-carbone ou une triple liaison carbone-carbone ou des fonctions halogénoacétamide, de préférence des fonctions maléimide ou acrylamide. L'invention concerne également une méthode de détection d'un acide nucléique dans un échantillon biologique comprenant une étape de détection d'hybridation entre un oligonucléotide modifié et un acide nucléique cible amplifié à partir de l'échantillon biologique. L'invention concerne plus particulièrement une méthode de détection, de génotypage ou de séquençage d'un organisme pathogène, de préférence un virus.

### ETAT DE LA TECHNIQUE

En raison de leur stabilité physicochimique et de la spécificité conférée par l'agencement successif des différents nucléotides qui les composent, les acides nucléiques constituent des molécules extrêmement bien adaptées pour une utilisation dans les procédés de détection et d'identification spécifiques d'organismes humains, animaux, végétaux, bactériens ou viraux.

Ces propriétés ont conduit au développement de biocapteurs (en anglais « biosensors ») fiables et sensibles, qui se composent classiquement d'un élément de reconnaissance moléculaire et d'un transducteur. L'élément de reconnaissance moléculaire, appelé « sonde », est généralement immobilisé à la surface du transducteur et présente une spécificité et une sensibilité élevées pour une molécule d'acide nucléique « cible ». Le transducteur a pour rôle de convertir l'évènement de reconnaissance moléculaire, c'est-à-dire l'hybridation entre les séquences nucléotidiques de la sonde et de la cible, en un signal facilement mesurable. Les biocapteurs exploitent ainsi les capacités d'appariement de deux séquences nucléotidiques complémentaires contenues dans la sonde et la cible, et engendrent un signal lorsque l'hybridation des deux séquences se produit.

Les biocapteurs se caractérisent par le mode de transduction utilisé pour révéler l'hybridation des séquences sonde et cible.

Les biocapteurs à transduction directe permettent de détecter l'hybridation des séquences sans recourir à un marquage particulier. Ils comprennent par exemple les systèmes électrochimiques, dans lesquels la transduction s'effectue par des réactions de transfert d'électrons, tels que l'ampérométrie, basée sur la détection de changements de courant pour un potentiel constant, la conductimétrie, reposant sur la détection de changements de conductivité entre deux électrodes, ou la potentiométrie, basée sur la détection de changements de potentiel, pour un courant constant. Les biocapteurs à transduction directe comprennent également les systèmes gravimétriques, basés sur des technologies piézoélectriques, et utilisant par exemple les propriétés d'un cristal de quartz, dont la fréquence change en réponse à des modifications qui interviennent à sa surface. Généralement rapides et spécifiques, les biocapteurs à transduction directe présentent néanmoins une sensibilité limitée (10⁻⁹ à 10⁻¹² M). Les biocapteurs à transduction directe sont bien adaptés pour la réalisation de systèmes de diagnostic peu onéreux, à usage unique et portables.

Les biocapteurs à transduction indirecte nécessitent, quant à eux, le marquage des séquences d'acides nucléiques cibles, et requièrent la mise en oeuvre d'étapes de lavage avant détection. Les biocapteurs à transduction indirecte se présentent généralement sous la forme de systèmes optiques, dans lesquels la détection est effectuée dans un lecteur couplé à un microscope confocal, sur la base d'une émission lumineuse par un ou plusieurs marqueurs fluorescents en réponse à une excitation induite par un laser. Les biocapteurs à transduction indirecte requièrent donc généralement de lourds instruments, compliqués à manipuler et nécessitant des personnes qualifiées, mais ils présentent une sensibilité de détection élevée (10⁻¹² à 10⁻¹⁵ M). Les biocapteurs à transduction indirecte constituent la technologie la plus employée dans le domaine du diagnostic à base d'acides nucléiques.

Les biocapteurs, à transduction directe ou indirecte, permettent de détecter des milliers ou dizaines de milliers d'hybridations de séquences d'acides nucléiques simultanément et sont généralement agencés sous la forme d'un arrangement ordonné de sondes immobilisées sous forme de points sur un support solide. Chaque point contient environ 10⁶ sondes identiques possédant la même séquence nucléotidique, et la séquence nucléotidique des sondes diffère d'un point à un autre.

Les « microréseaux » (en anglais « microarrays ») sont des biocapteurs de haute complexité, comprenant 1000 à 10⁶ points d'une taille d'environ 20 à 50 µm. Ils sont très sophistiqués et onéreux et sont principalement destinés au séquençage de l'ADN, à l'étude des maladies génétiques et aux analyses de polymorphisme. Ce type de biocapteur est notamment développé par les compagnies Affymetrix, Illumina et Agilent. Les « macroréseaux » (en anglais « macroarrays ») sont, pour leur part, des biocapteurs de faible complexité comprenant 1000 à 10000 points d'une taille supérieure ou égale à 200 µm.

L'immobilisation des sondes sur les biocapteurs peut être effectuée par couplage covalent ou de manière non covalente. Le couplage covalent de la sonde avec le transducteur peut être obtenu par exemple par photolithographie ou par réaction d'un oligonucléotide aminé avec une surface revêtue de silane fonctionnalisé. La sonde peut également être immobilisée sur une surface métallique, et notamment sur une surface d'or, par exemple par l'intermédiaire d'une liaison entre un atome d'or et un atome de soufre. Cependant, la liaison Au-S est modérément forte, et une seule liaison or-soufre ne suffit pas à immobiliser une sonde sur la surface d'or de façon très stable. En effet, les procédés de détection comprennent des étapes, notamment de lavage, qui occasionnent de fortes contraintes mécaniques susceptibles de déstabiliser la liaison Au-S.

La sonde peut également être couplée avec le transducteur de manière non-covalente, par exemple par adsorption électrostatique. Le couplage non covalent peut par exemple résulter de l'interaction entre les phosphates chargés négativement de l'ADN de la sonde et la surface du transducteur revêtue de γ-amino propylsilane ou d'une couche de poly-L-lysine. Les interactions non covalentes entre la sonde et le transducteur résistent mal aux étapes de lavage mises en oeuvre au cours des procédures de détection, en particulier lorsque la détection est effectuée par transduction indirecte puisque la détection de l'hybridation nécessite, après l'étape d'hybridation elle-même, d'éliminer les séquences de nucléotides cibles marquées ne s'étant pas liées à la séquence nucléotidique de la sonde. Le couplage non covalent ne permet en outre pas de modifier significativement les conditions opératoires pour augmenter la stringence des étapes d'hybridation ou de lavage et les rendre ainsi plus spécifiques.

Le document EP 0 523 978 divulgue des composés phosphoramidite ou phosphonate utilisables pour la production d'oligonucléotides modifiés par des thiols. Ces composés ne peuvent toutefois être introduits qu'une seule fois et uniquement sur l'extrémité 5' d'un oligonucléotide.

Le document US 7,601,848 divulgue un composé polyfonctionnel comprenant deux atomes de soufre destiné à être incorporé dans des oligomères afin de créer au moins deux liaisons or-soufre et ainsi stabiliser l'oligonucléotide sur la surface d'or. Certains de ces composés comportent une fonction phosphoramidite. Les composés utilisés dans ce document sont néanmoins fabriqués à partir de composés très coûteux et le couplage du composé polyfonctionnel sur les oligonucléotides n'a pas un rendement satisfaisant en raison de l'encombrement stérique de ce composé. Dans ce document, un agent de liaison est nécessaire afin de lier les composés thiol entre eux et ainsi effectuer une multiple introduction de composés thiol dans un oligonucléotide.

Les documents JP 6 041 183 et US 7,601,848 décrivent un oligonucléotide modifié par des fonctions thiol. Le document EP 0 523 978 décrit un oligonucléotide greffé par une fonction phosphoramidite. Le document WO 2005/065405 décrit un ionophore activé par thiosulfonate qui comprend un ionophore, un groupe espaceur et un groupe fonctionnel d'alkylthiosulfonate.

Le but de l'invention est par conséquent de développer une sonde formée par un oligonucléotide possédant au moins deux fonctions thiol, susceptible d'être greffée de manière simple et efficace sur des supports variés comprenant aussi bien les surfaces revêtues de métal (par exemple l'or) que les surfaces greffées comportant au moins une double liaison carbone-carbone ou une triple liaison carbone-carbone ou des fonctions halogénoacétamide, de préférence des fonctions maléimide ou acrylamide. La sonde de l'invention pallie au moins partiellement les inconvénients précités et permet d'augmenter la spécificité et la sensibilité de détection de séquences nucléotidiques cibles, indépendamment du type de transduction (directe ou indirecte) mis en oeuvre lors de la détection.

L'invention vise également à fournir une méthode de détection, de séquençage et/ou de génotypage d'acides nucléiques d'organismes pathogènes ou infectieux ou de gènes responsables ou impliqués dans des maladies, qui soit économique, rapide, sensible, plus flexible et plus facile à automatiser que les méthodes actuelles.

### RESUME DE L'INVENTION

La présente invention décrit un oligonucléotide modifié répondant à la formule (XIIb):

(XIIb) N₁-N₂-...-Nₙ₋₁-Nₙ-(I'b)_{y}-(M₁-...-Mₘ₋₁-Mₘ)ₚ-(I'b)_{y'}

ou à la formule (XIIIb):

(XIIIb) (Ic')-(I'b)_{y-1}-N₁-N₂-...-Nₙ₋₁-Nₙ-(I'b)_{y'}-(M₁-M₂-...-Mₘ₋₁-Mₘ)ₚ-(I'b)_{y"}

dans lesquelles,
N₁, ..., Nₙ représentent indépendamment les uns des autres un nucléotide,
M₁, ..., Mₘ représentent indépendamment les uns des autres un nucléotide,
(I'b) représente un composé de formule : 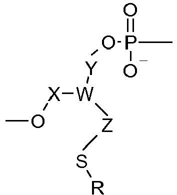
(Ic') représente un composé de formule : 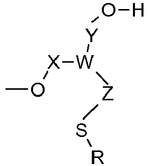
n est un nombre entier allant de 4 à 100,
m est un nombre entier allant de 4 à 100,
y est un nombre entier allant de 2 à 12,
p représente 0 ou 1,
y' est un nombre entier allant de 0 à 12 si p vaut 1 et y' est égal à 0 si p vaut 0,
y" est un nombre entier allant de 0 à 12 si p vaut 1 et si p vaut 0 alors y" vaut 0,
la somme des nombres entiers (y + y') ou (y + y' + y") étant comprise entre 2 et 12,
X est choisi parmi les groupements alkyles linéaires ou ramifiés en C1-C12, aminoalkyles en C1-C12, alkoxy en C1-C12, cycloalkyles en C3-C12, cyclohétéroalkyles oxygénés ou azotés en C3-C12,
Y est choisi parmi les groupements alkyles linéaires ou ramifiés en C1-C12, aminoalkyles en C1-C12, alkoxy en C1-C12, cycloalkyles en C3-C12, cyclohétéroalkyles oxygénés ou azotés en C3-C12,
Z est choisi parmi les groupements alkoxy en C1-C12, cyclohétéroalkyles oxygénés ou azotés en C3-C12, NCO-alkyles en C1-C12, CON-alkyles en C1-C12,
W est choisi parmi les groupements alcanes tri-yles en C1-C12, les groupements aryles tri-yles en C6-C18 et les groupements aralcanes tri-yles en C6-C18, de préférence un groupement choisi parmi CH, CCH₃, CCH₂CH₃, un cyclohexane tri-yle et benzène tri-yle,
R est H ou est choisi parmi les groupements acyles en C1-C12, S-alkyles en C1-C12, S-aryles en C6-C12, S-2-pyridine, S-hétéroalkyles oxygénés ou azotés en C1-C12, S-cycloalkyles en C3-C12, S-cyclohétéroalkyles oxygénés ou azotés en C3-C12, et
R1 est choisi parmi les groupements 2-cyanoéthyle ou R'₁R'₂R'₃SiCH₂CH₂, dans lequel R'₁, R'₂ et R'₃ sont identiques ou différents et représentent un groupement choisi parmi les alkyles linéaires ou ramifiés comportant de 1 à 12 atomes de carbone et les aryles en C6-C12.

L'oligonucléotide modifié de l'invention répond à la formule : 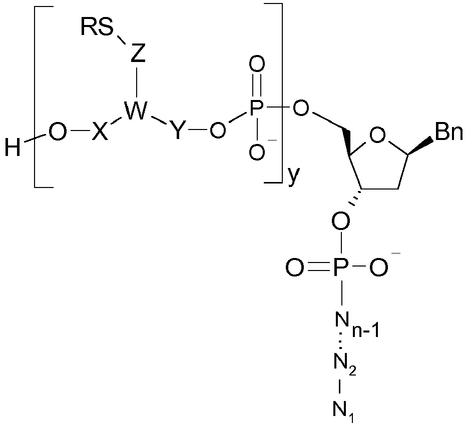 dans laquelle n, y, N₁,..., Nₙ₋₁, X, Y, Z, W et R ont la même définition que ci-dessus, et Bₙ représente la base du n-ième nucléotide.

Selon un autre mode de réalisation de l'invention, l'oligonucléotide modifié répond à la formule : 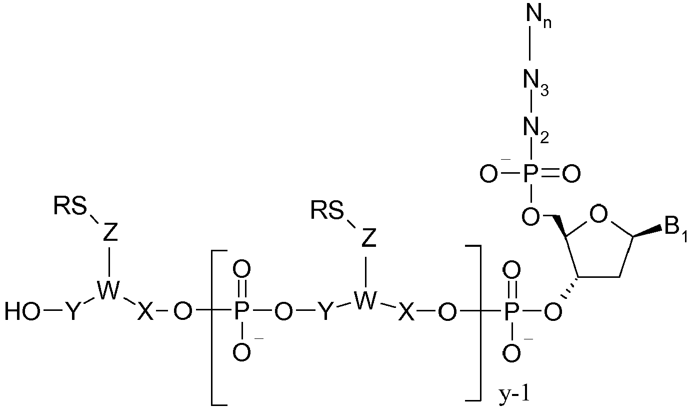 dans laquelle n, y, N₂,..., Nₙ, X, Y, Z, W et R ont la même définition que ci-dessus, et B₁ représente la base du 1er nucléotide.

Selon un mode de réalisation de l'invention, l'oligonucléotide modifié tel que décrit ci-dessus, comprend une séquence de nucléotides (N₁-N₂-...-Nₙ₋₁-Nₙ) et, éventuellement, une séquence de nucléotides (M₁-M₂-...-Mₘ₋₁-Mₘ) qui sont spécifiques d'un virus, d'une bactérie ou d'un gène responsable ou impliqué dans une maladie.

Selon un mode de réalisation de l'invention, la séquence de nucléotides (N₁-N₂-...-Nₙ₋₁-Nₙ) et, éventuellement, la séquence de nucléotides (M₁-M₂-...-Mₘ₋₁-Mₘ) sont choisies parmi :
- les séquences de SEQ ID NO : 1, de SEQ ID NO : 4, de SEQ ID NO : 27, de SEQ ID NO : 28, de SEQ ID NO : 35 ou de SEQ ID NO : 36 spécifiques du virus de l'hépatite C (VHC),
- les séquences de SEQ ID NO : 16, de SEQ ID NO : 17 ou de SEQ ID NO : 40, spécifiques des flavivirus,
- la séquence de SEQ ID NO : 18 ou de SEQ ID NO : 41, spécifique du virus de la Dengue, ou
- la séquence de SEQ ID NO : 19, spécifique du West Nile virus (WNV).

Selon un mode de réalisation de l'invention, la séquence de nucléotides (N₁-N₂-...-Nₙ₋₁-Nₙ) et, éventuellement, la séquence de nucléotides (M₁-M₂-...-Mₘ₋₁-Mₘ) ont une structure de type anomère alpha, anomère béta, linéaire, ou tige-boucle (« snail »).

Un autre objet de l'invention concerne un substrat greffé par au moins un oligonucléotide modifié tel que décrit ci-dessus, ledit substrat comportant au moins une zone de réception revêtue d'une substance tolérant le greffage dudit oligonucléotide modifié.

Selon un mode de réalisation de l'invention, ladite zone de réception dudit substrat greffé est revêtue d'un film d'or ou de platine, et ledit substrat est en métal, de préférence en cuivre, ou ladite zone de réception comporte à sa surface au moins une double liaison carbone-carbone (fonction alcényle) ou une triple liaison carbone-carbone (fonction alcynyle) ou des fonctions halogénoacétamide, de préférence des fonctions maléimide ou acrylamide, et ledit substrat est en plastique, de préférence en polystyrène. Selon un mode de réalisation de l'invention, les substrats utilisés sont des polymères non conducteurs. Selon un autre mode de réalisation de l'invention, les substrats utilisés pour mettre en oeuvre l'invention sont conducteurs.

Selon un mode de réalisation de l'invention, le substrat greffé plan (en partie ou dans sa totalité) ou courbe, et peut avantageusement prendre une forme sphérique. Selon un mode de réalisation, le substrat est non planaire, par exemple sous forme de micorparticules ou nanoparticules, et est de préférence magnétiques.

Un autre objet de l'invention concerne une méthode de détection d'au moins un acide nucléique cible dans un échantillon biologique, comprenant une étape de détection dudit acide nucléique cible avec au moins une sonde de détection formée par un oligonucléotide modifié tel que décrit ci-dessus.

Selon un mode de réalisation de l'invention, la méthode de détection de l'invention comprend les étapes :
- d'obtention d'au moins un acide nucléique source à partir d'un échantillon biologique,
- de production d'un amplicon par amplification dudit acide nucléique cible à partir de l'acide nucléique source, et
- de détection de l'hybridation dudit amplicon avec au moins une sonde de détection formée par un oligonucléotide modifié tel que décrit ci-dessus.

Selon un mode de réalisation de l'invention, la méthode de détection de l'invention permet de déterminer le génotype et/ou le sous-type d'un virus présent dans un échantillon biologique, et comprend :
- la génération d'un amplicon par amplification d'une séquence nucléotidique cible, correspondant à une région génomique du virus portant des informations relatives au génotype et/ou au sous-type viral, et
- la détection de l'hybridation dudit amplicon avec au moins une sonde de détection formée par un oligonucléotide modifié tel que décrit ci-dessus avec une sonde spécifique d'un génotype et/ou d'un sous-type viral.

Selon un mode de réalisation de l'invention, l'étape de production de l'amplicon de la méthode de détection de l'invention est réalisée avec un mélange d'amorces nucléotidiques, de préférence choisies parmi les couples d'amorces :
- SEQ ID NO : 8 et SEQ ID NO : 9, lorsque l'amplicon est généré à partir du VHC, quelque soit le génotype viral impliqué. Ce couple d'amorces est générique et permet d'amplifier un amplicon « long » de 401nt à partir de tout génotype de VHC ;
- SEQ ID NO : 10 et SEQ ID NO : 9, couple permettant la génération d'amplicons « courts » de 191 nt spécifiques du génotype 1a/1b ;
- SEQ ID NO : 29 et SEQ ID NO : 9, couple permettant la génération d'amplicons « courts » de 108 nt spécifiques du génotype 2 ;
- SEQ ID NO : 8 et SEQ ID NO : 11, couple permettant la génération d'amplicons « courts » de 143 nt spécifiques du génotype 3a ;
- SEQ ID NO : 8 et SEQ ID NO : 30, couple permettant la génération d'amplicons « courts » de 175 nt spécifiques du génotype 4a/4d ; et
- SEQ ID NO : 20 et SEQ ID NO : 21, et/ou SEQ ID NO : 22 et SEQ ID NO : 21 lorsque l'amplicon est généré à partir d'un flavivirus.

Un autre objet de l'invention concerne un kit de détection d'au moins un acide nucléique cible dans un échantillon biologique, comprenant :
- au moins un oligonucléotide modifié tel que décrit ci-dessus et au moins un substrat comportant au moins une zone de réception revêtue d'une substance tolérant le greffage dudit oligonucléotide modifié, ladite zone de réception étant de préférence revêtue d'or, de platine ou comporte au moins une double liaison carbone-carbone ou une triple liaison carbone-carbone ou des fonctions halogénoacétamide, de préférence des fonctions maléimide ou acrylamide
- au moins un substrat greffé tel que décrit ci-dessus.

La présente invention décrit un oligonucléotide possédant une séquence nucléotidique choisie parmi les séquences SEQ ID NO : 1, SEQ ID NO : 4, SEQ ID
NO : 27, SEQ ID NO : 28, SEQ ID NO : 16, SEQ ID NO : 17, SEQ ID NO : 18, SEQ ID NO : 19, SEQ ID NO : 35, SEQ ID NO : 36, SEQ ID NO : 40 et SEQ ID NO : 41.

Un autre objet de l'invention concerne l'utilisation d'un oligonucléotide ou d'un oligonucléotide modifié tels que décrits ci-dessus ou d'un substrat greffé tel que décrit ci-dessus pour détecter au moins un acide nucléique cible dans un échantillon biologique.

Selon un mode de réalisation de l'invention, ladite utilisation permet le diagnostic ou le génotypage de souches virales, de préférence du VHC, des virus de la Dengue ou du West Nile.

Les avantages de la présente invention sont les suivants :
- l'oligonucléotide modifié de l'invention portant les fonctions thiol est peu coûteux à produire,
- l'oligonucléotide modifié de l'invention peut être immobilisé de manière stable sur une surface d'or, ou sur une surface comportant au moins une double liaison carbone-carbone ou une triple liaison carbone-carbone ou des fonctions halogénoacétamide, de préférence des fonctions maléimide ou acrylamide,
- l'utilisation de l'oligonucléotide modifié de l'invention dans des méthodes de détection, de séquençage et/ou de génotypage d'acides nucléiques permet de détecter spécifiquement des séquences nucléotidiques cibles de plusieurs centaines de nucléotides,
- la séquence nucléotidique de l'oligonucléotide modifié de l'invention est avantageusement plus courte que celles des sondes utilisées dans les méthodes de détection et/ou de génotypage connues, tout en présentant une meilleure sensibilité et spécificité de détection.

D'autres caractéristiques et avantages de l'invention apparaîtront à la lecture de la description qui suit d'un mode de réalisation préféré de l'invention, donnée à titre d'exemple et en référence aux dessins annexés.

### BREVE DESCRIPTION DES FIGURES

La figure 1 représente un schéma décrivant un procédé de synthèse des composés (I).
Les figures 2A et 2B représentent respectivement un schéma décrivant un procédé de synthèse d'un oligomère des composés de l'invention à partir de composés (Ia) et à partir de composés (Ib).
La figure 3 représente un schéma décrivant un procédé de synthèse d'un composé (XIIc) oligonucléotidique greffé par un oligomère de (I) à son extrémité 5'.
La figure 4 représente un schéma exposant un procédé de synthèse d'un composé (XIIIc) oligonucléotidique greffé par un oligomère de (I) à son extrémité 3'.
La figure 5 représente un histogramme du taux de greffage d'oligonucléotides modifiés sur une surface d'or.
La figure 6 représente un schéma exposant la stabilité du greffage d'oligonucléotides modifiés sur une surface d'or en fonction du temps à 60°C.
La figure 7 représente un schéma exposant la stabilité du greffage d'oligonucléotides modifiés sur une surface d'or en fonction du temps à 80°C.
Les figures 8 et 9 représentent des diagrammes schématisant les résultats de tests ELOSA avec détection par fluorescence, réalisés avec une sonde tétrathiol de type 3a.
La figure 10 représente un diagramme schématisant les résultats de tests ELOSA avec détection par fluorescence, réalisés avec une sonde tétrathiol de type 1a/1b.
La figure 11 représente un diagramme schématisant les résultats de tests ELOSA avec détection par fluorescence, réalisés avec des sondes de type 1a/1b portant 1, 2, 4, 6 ou 8 groupements thiol.
La figure 12 représente un diagramme schématisant les résultats de tests ELOSA avec détection de fluorescence, réalisés avec une sonde de type 1a/1b monothiol à différentes densités de greffage.
La figure 13 représente un diagramme schématisant les résultats de tests ELOSA avec détection de fluorescence, réalisés avec une sonde de type 1a/1b dithiol à différentes densités de greffage.
La figure 14 représente un diagramme schématisant les résultats de tests ELOSA avec détection de fluorescence, réalisés avec une sonde de type 1a/1b tétrathiol à différentes densités de greffage.
La figure 15 représente les résultats d'un test d'hybridation sonde/cible avec une sonde tétrathiol greffée sur surface d'or.
La figure 16a représente les réactions entre l'oligonucléotide modifié selon l'invention et la surface greffée par des groupements alcényles ou alcynyles activés.
La figure 16b représente les réactions entre l'oligonucléotide modifié selon l'invention et la surface greffée par des groupements alcényles ou alcynyles avec activation lumineuse (λ= 265 nm).
La figure 17 représente un diagramme schématisant les résultats de tests ELOSA avec détection par fluorescence, réalisés avec une sonde VHC tétrathiol de type 1a/1b.
La figure 18 représente un diagramme schématisant les résultats de tests ELOSA avec détection par fluorescence, réalisés avec un mélange de deux sondes VHC tétrathiol de types 2a/2c et /2b.
La figure 19 représente un diagramme schématisant les résultats de tests ELOSA avec détection par fluorescence, réalisés avec une sonde VHC tétrathiol de type 3a.
La figure 20 représente un diagramme schématisant les résultats de tests ELOSA avec détection par fluorescence, réalisés avec une sondeVHC tétrathiol de type 4a/4d.
La figure 21 représente un diagramme schématisant les résultats de tests ELOSA avec détection par fluorescence, réalisés avec une sonde tétrathiol générique pour les flavivirus.
La figure 22 représente un diagramme schématisant les résultats de tests ELOSA avec détection par fluorescence, réalisés avec une sonde tétrathiol spécifique du sérotype 4 de la Dengue.
La figure 23 représente un diagramme schématisant les résultats de tests ELOSA avec détection par fluorescence, réalisés avec une sonde tétrathiol générique pour le virus West Nile.

### EXPOSE DES MODES DE REALISATION DE L'INVENTION

La présente demande décrit la préparation de composés de structure phosphoramidite, H-phosphonate ou de composés reliés à un support solide présentant une fonction thiol protégée. Ces composés thiol sont destinés à être introduits dans des oligonucléotides pour former les oligonucléotides modifiés selon l'invention. Les oligonucléotides modifiés ainsi obtenus peuvent présenter plusieurs fonctions thiol.

La présente invention concerne donc les oligonucléotides modifiés susceptibles d'être obtenus par le procédé décrit ci-après et comprenant de 2 à 12 fonctions thiol. Un autre objet de l'invention concerne l'utilisation de tels oligonucléotides modifiés pour détecter au moins un acide nucléique cible dans un échantillon biologique.

### Composé thiol

Les composés thiol destinés à être introduits dans les oligonucléotides modifiés de la présente invention répondent à la formule (I) suivante : 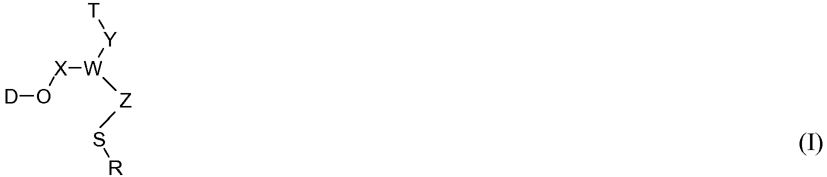 dans laquelle :
T est un groupement choisi parmi -O-P(OR₁)N(R₂)₂, -O-PH(O)O⁻, -OC(O)J
   C(O)NH-,
   o R₁ est choisi parmi les groupements 2-cyanoéthyle, R'₁R'₂R'₃SiCH₂CH₂, et R'₁, R'₂, R'₃, identiques ou différents, représentent un groupement choisi parmi les alkyles linéaires ou ramifiés comportant de 1 à 12 atomes de carbone et les aryles en C6-C12,
   o R₂ est choisi parmi les groupements alkyles linéaires ou ramifiés comportant de 1 à 12 atomes de carbone, pyrrolidine,
   o J est choisi parmi une simple liaison, un groupement -CH₂-, -CH₂CH₂-, -CH₂OCH₂-, -CH₂OPhOCH₂- où Ph est un benzyle,
   ∘ représente un support solide,
D est un groupement protecteur des alcools,
W est choisi parmi les groupements alcanes tri-yle en C1-C12, les groupements aryles tri-yles en C6-C18 et les groupements aralcanes tri-yles en C6-C18,
Z est choisi parmi les groupements alkoxy en C1-C12, cyclohétéroalkyles oxygéné ou azoté en C3-C12, NCO-alkyles en C1-C12, CON-alkyles en C1-C12,
Y est choisi parmi les groupements alkyles linéaires ou ramifiés en C1-C12, aminoalkyles en C1-C12, alkoxy en C1-C12, cycloalkyles en C3-C12, cyclohétéroalkyles oxygénés ou azotés en C3-C12,
X est choisi parmi les groupements alkyles linéaires ou ramifiés en C1-C12, aminoalkyles en C1-C12, alkoxy en C1-C12, cycloalkyles en C3-C12, cyclohétéroalkyles oxygénés ou azotés en C3-C12,
R est choisi parmi les groupements acyles en C1-C12, S-alkyles en C1-C12, S-aryles en C6-C12, S-2-pyridine, S-hétéroalkyles oxygénés ou azotés en C1-C12, S-cycloalkyles en C3-C12, S-cyclohétéroalkyles oxygénés ou azotés en C3-C12.

Au sens de la présente invention, par « alcane tri-yle », on entend les alcanes tri-yle linéaires, ramifiés ou cycliques, éventuellement substitués par un ou plusieurs groupement alkyles. Parmi les groupements aryles tri-yles qui peuvent être présents dans le composé selon l'invention, on peut citer le benzène tri-yle et le naphtalène tri-yle. Parmi les groupements aralcanes, on peut citer le 1,3,5-triméthylbenzène tri-yle et le triméthylnaphtalène tri-yle.

Le composé (I) peut être distingué en trois sous-composés (Ia), (Ib) et (Ic) répondant aux formules (Ia), (Ib) et (Ic) ci-dessous dans lesquelles les paramètres X, Y, Z, R, R₁, R₂ et D ont la même définition qu'exposée ci-dessus pour la formule (I) :

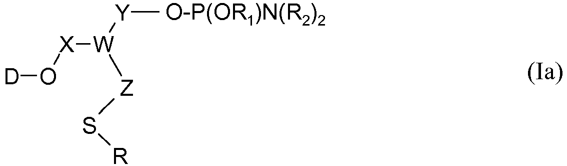

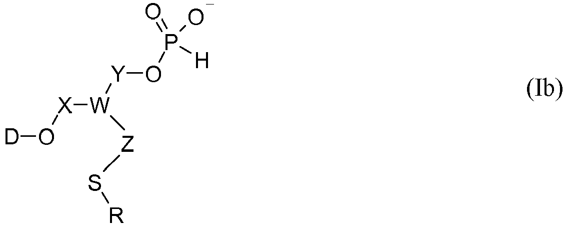

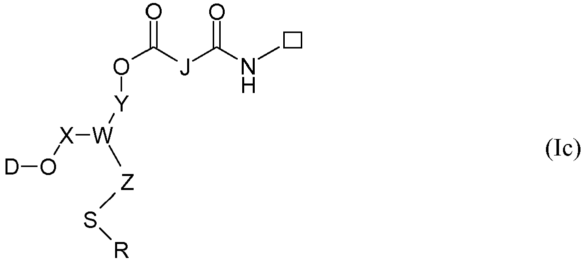

De préférence, R₁ est choisi parmi les groupements 2-cyanoéthyle et R'₁R'₂R'₃SiCH₂CH₂, et R'₁, R'₂, R'₃ identiques ou différents représentent un groupement choisi parmi les groupements alkyles linéaires ou ramifiés comportant de 1 à 6 atomes de carbone, le phényl ; préférentiellement R₁ est choisi parmi les groupements 2-cyanoéthyle et R'₁R'₂R'₃SiCH₂CH₂, et R'₁, R'₂, R'₃ identiques ou différents représentent un groupement choisi parmi les groupements alkyles linéaires ou ramifiés comportant de 1 à 3 atomes de carbone, le phényl ; encore plus préférentiellement R₁ est choisi parmi les groupements 2-cyanoéthyle, 2-(triméthylsilyl)éthyle, 2-(triphénylsilyl)éthyle, 2-(diphénylméthylsilyl)éthyle.

De préférence, R₂ est choisi parmi les groupements alkyles linéaires ou ramifiés comportant de 1 à 6 atomes de carbone. De préférence, R₂ est un groupement isopropyle (iPr).

De préférence, le support solide □ est choisi parmi les résines, en particulier parmi les résines à base de polystyrène, polyacrylamide, polyéthylène glycol, cellulose, polyéthylène, polyester, latex, polyamide, polydiméthylacrylamide, les polymères hydrophiles synthétiques ou naturels, les billes de verre, les gels de silice.

De préférence, W est choisi parmi les groupements alcanes tri-yle en C1-C6, un groupement aryle tri-yle en C6-C12, un groupement aralcane tri-yle en C6-C12, plus particulièrement parmi les groupements CH, CCH₃, CCH₂CH₃, les cyclohexanes tri-yle et les benzènes tri-yle.

De préférence, D est choisi parmi les groupements protecteurs des alcools qui permettent une déprotection orthogonale par rapport aux autres groupements du composé (I). Plus particulièrement, D est choisi parmi le 4,4'-diméthoxytrityle (DMTr), le 9-phenylxanthen-9-yl (pixyl) ou le Fluorenylmethyloxycarbonyl (Fmoc). Le groupement protecteur pixyl est décrit notamment dans le document Chattopadhyaya et Reese, Chem. Soc. Chem. Comm., 1978, 639-640. Un autre groupement protecteur des alcools peut être un groupement tert-butyl-diméthylsilyl, dans ce cas un support en polystyrène sera particulièrement préféré.

De préférence, Z est choisi parmi les groupements aminoalkyles en C1-C6, alkoxy en C1-C6, cyclohétéroalkyles oxygénés ou azotés en C3-C6, NCO-alkyles en C1-C6, CON-alkyles en C1-C6.

De préférence, Y est choisi parmi les groupements alkyles linéaires ou ramifiés en C1-C6, aminoalkyles en C1-C6, alkoxy en C1-C6, cycloalkyles en C3-C6, cyclohétéroalkyles oxygénés ou azotés en C3-C6.

De préférence, X est choisi parmi les groupements alkyles linéaires ou ramifiés en C1-C6, aminoalkyles en C1-C6, alkoxy en C1-C6, cycloalkyles en C3-C6, cyclohétéroalkyles oxygénés ou azotés en C3-C6.

De préférence, R est choisi parmi les groupements acyles en C1-C12, S-alkyles en C1-C6, S-aryles en C6, S-hétéroalkyles oxygénés ou azotés en C6, S-cycloalkyles en C6, S-cyclohétéroalkyles oxygénés ou azotés en C6.

Selon un mode de réalisation, les alkyles linéaires ou ramifiés sont choisis parmi les groupements méthyle, éthyle, propyle, butyle, pentyle, hexyle, heptyle, octyle, nonyle, décyle, undécyle, dodécyle, isopropyle, isobutyle, *tert*-butyle.

Selon un mode de réalisation, les aminoalkyles sont choisis parmi les groupements aminométhyle, aminoéthyle, aminopropyle, aminobutyle, aminopentyle, aminohexyle, aminoheptyle, aminooctyle, aminononyle, aminodécyle, aminoundécyle, aminododécyle, aminoisopropyle, aminoisobutyle, amino-*tert*-butyle comprenant un ou plusieurs atomes d'azote.

Selon un mode de réalisation, les alkoxy sont choisis parmi les groupements méthoxy, éthoxy, propyloxy, oxybutyloxy, pentyloxy, hexyloxy, heptyloxy, octyloxy, nonyloxy, décyloxy, undécyloxy, dodécyloxy, isopropyloxy, isobutyloxy, *tert-*butyloxy comprenant un ou plusieurs atomes d'oxygène.

Selon un mode de réalisation, les cycloalkyles sont choisis parmi les cycles, comprenant éventuellement une ou plusieurs insaturations, comprenant entre 3 et 12 atomes de carbone, de préférence 6 atomes de carbone.

Selon un mode de réalisation, les cyclohétéroalkyles sont choisis parmi les cycles substitués par un ou plusieurs atomes d'azote et/ou d'oxygène, comprenant éventuellement une ou plusieurs insaturations et comprenant entre 3 et 12 atomes de carbone, de préférence 5 atomes de carbone et un atome d'azote ou d'oxygène.

Selon un mode de réalisation, les NCO-alkyles et CON-alkyles sont des groupements dans lesquels les alkyles peuvent être des alkyles linéaires ou ramifiés choisis parmi les groupements méthyle, éthyle, propyle, butyle, pentyle, hexyle, heptyle, octyle, nonyle, décyle, undécyle, dodécyle, isopropyle, isobutyle, *tert*-butyle.

Selon un mode de réalisation, R₂ est un groupement isopropyle (iPr) et/ou R₁ est un groupement cyanoéthyle.

Selon un mode de réalisation préféré, le composé thiol (Ia) est le composé (VI) répondant à la formule suivante : 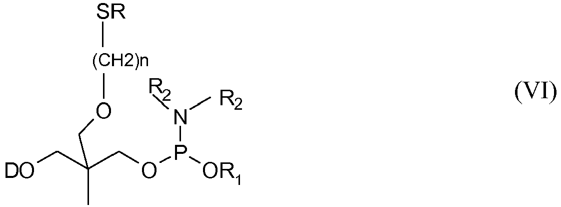 dans laquelle,
n est un nombre entier compris entre 1 et 12, de préférence compris entre 1 et 6,
R, R₁, R₂ et D ont la même définition que ci-dessus pour (Ia).

De préférence, R₂ est un groupement isopropyle (iPr) et R₁ est un groupement cyanoéthyle.

De préférence, R est un groupement acétyle.

De préférence, D est le 4,4'-diméthoxytrityle.

Selon un autre mode de réalisation, le composé thiol (Ia) est le composé (VII) répondant à la formule suivante : 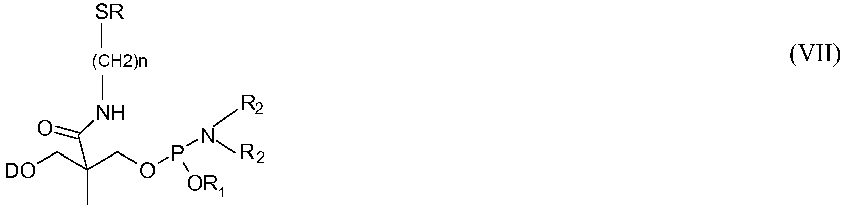 dans laquelle,
n est un nombre entier compris entre 1 et 12, de préférence compris entre 1 et 6,
R, R₁, R₂ et D ont la même définition que ci-dessus pour (Ia).

De préférence, R₂ est un groupement isopropyle (iPr) et R₁ est un groupement cyanoéthyle.

De préférence, R est un groupement acétyle.

De préférence, D est le 4,4'-diméthoxytrityle.

Selon un mode de réalisation, le composé thiol (Ic) est le composé (VIII) répondant à la formule suivante : 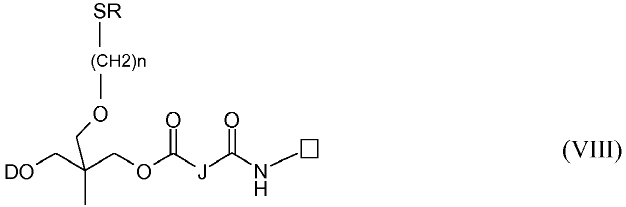 dans laquelle,
n est un nombre entier compris entre 1 et 12, de préférence entre 1 et 6,
R et □ ont la même définition que ci-dessus pour (Ic).

De préférence, R est un groupement acétyle. De préférence, J est un groupement éthyle. De préférence, D est le 4,4'-diméthoxytrityle.

Selon un mode de réalisation, le composé thiol (Ia) est le composé (IX) de formule : 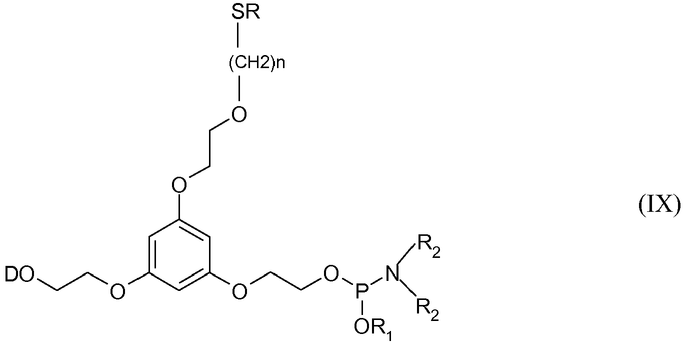 dans laquelle,
n est un nombre entier compris entre 1 et 12, de préférence entre 1 et 6,
R, R₁, R₂ et D ont la même définition que ci-dessus pour (Ia).

De préférence, R₂ est un groupement isopropyle (iPr) et R₁ est un groupement cyanoéthyle.

De préférence, R est un groupement acétyle.

De préférence, D est le 4,4'-diméthoxytrityle.

Selon un mode de réalisation, le composé thiol (Ia) est le composé (X) de formule : 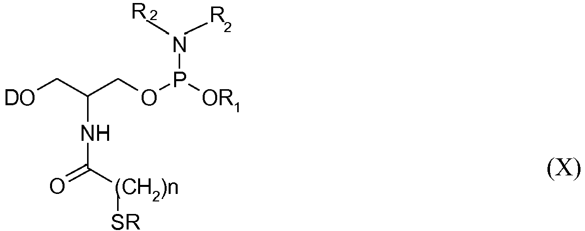 dans laquelle,
n est un nombre entier compris entre 1 et 12, de préférence entre 1 et 6,
R, R₁, R₂ et D ont la même définition que ci-dessus pour (Ia).

De préférence, R₂ est un groupement isopropyle (iPr) et R₁ est un groupement cyanoéthyle.

De préférence, R est un groupement acétyle.

De préférence, D est le 4,4'-diméthoxytrityle.

De préférence, R₂ est un groupement isopropyle (iPr) et R₁ est un groupement cyanoéthyle.

Selon un mode de réalisation, le composé thiol (Ia) est le composé (XI) de formule : 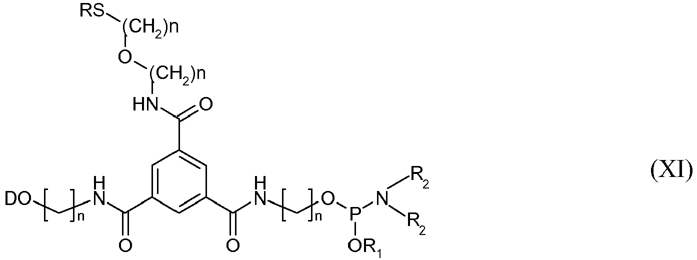 dans laquelle,
n est un nombre entier compris entre 1 et 12, de préférence compris entre 1 et 6,
R, R₁, R₂ et D ont la même définition que ci-dessus pour (Ia).

De préférence, R₂ est un groupement isopropyle (iPr) et R₁ est un groupement cyanoéthyle.

De préférence, R est un groupement acétyle.

De préférence, D est le 4,4'-diméthoxytrityle.

### Procédé de fabrication

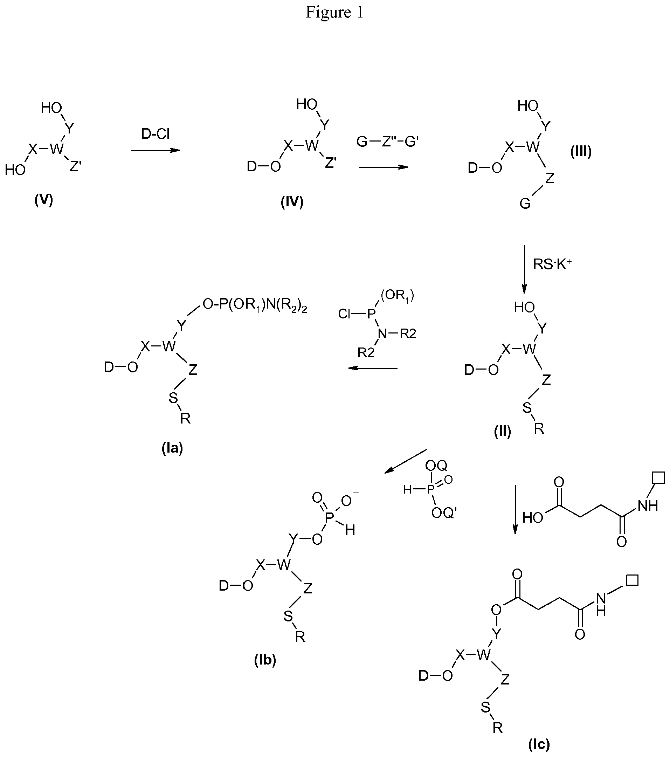

Le procédé de fabrication des composés (Ia), (Ib) et (Ic) est représenté sur le schéma de la figure 1.

Les composés (Ia), (Ib) et (Ic) sont obtenus à partir du même composé (II) présentant la formule suivante : 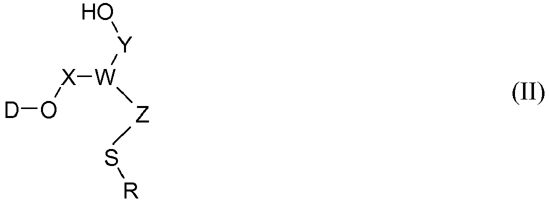 dans laquelle, D, X, W, Y, Z et R ont la même définition que dans le composé thiol (I).

Le composé de formule (Ia) peut être obtenu selon la réaction représentée sur le schéma suivant : 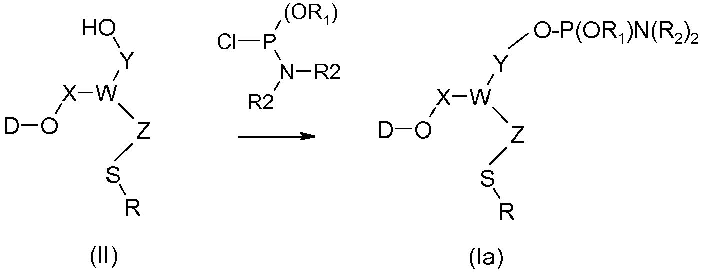 ou selon la réaction représentée sur le schéma suivant, de préférence en présence du sel de diisopropylamine tétrazolure :

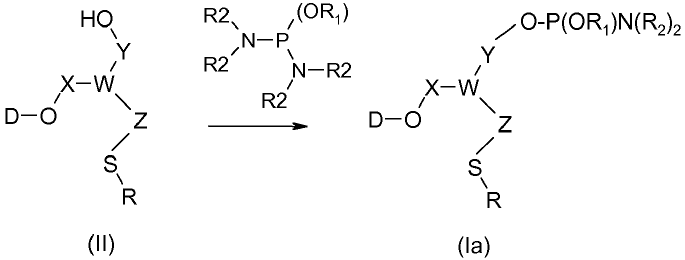

Le composé de formule (Ib) peut être obtenu selon la réaction représentée sur le schéma suivant : 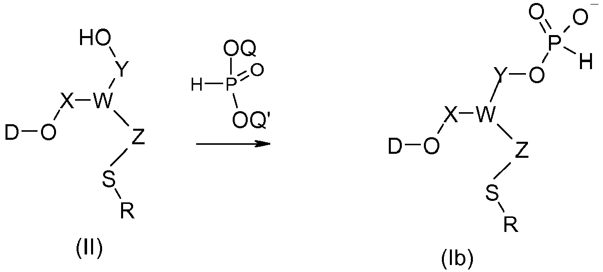 dans laquelle, Q et Q' représentent, indépendamment l'un de l'autre, un groupement benzène substitué ou non.

La réaction précédente d'obtention du composé (Ia) ou (Ib) s'effectue à partir du composé (II), de préférence en présence d'une base, par exemple la diisopropyléthylamine (DIEA), dans un solvant anhydre, tel que le dichlorométhane anhydre.

Le composé de formule (Ic) est également obtenu à partir du composé (II) mais de préférence selon l'étape de réaction représentée sur le schéma suivant :

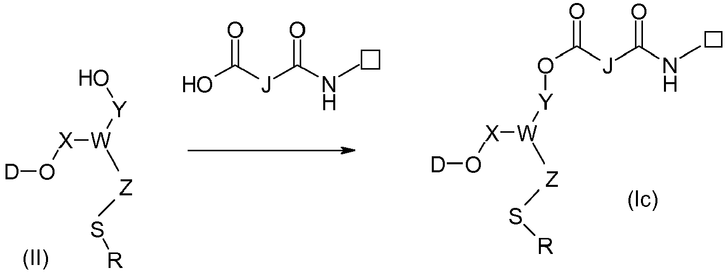

La réaction précédente d'obtention du composé (Ic) s'effectue de préférence dans un solvant anhydre, tel que la pyridine, en présence d'une base, telle que la triéthylamine.

On peut également obtenir le composé (Ic) selon le schéma réactionnel suivant :

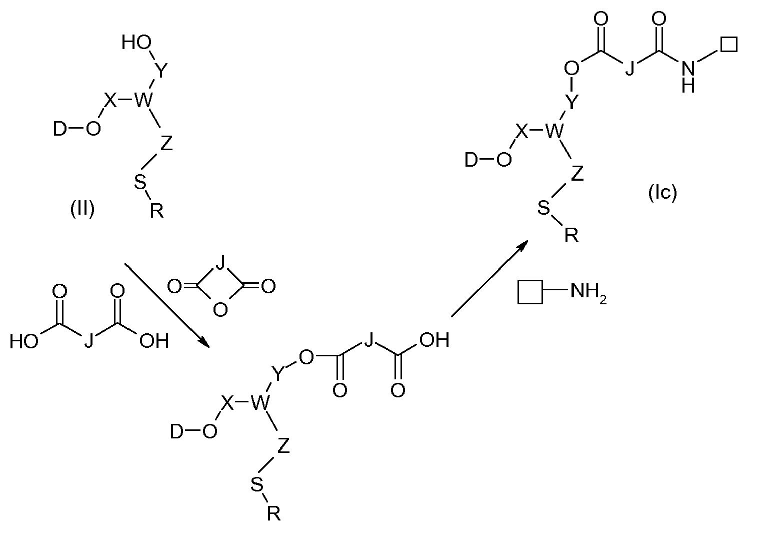

Le composé (II) de formule ci-dessus, dans laquelle les groupements X, W, Z, Y et R ont la même définition que dans le composé (I), correspond à un mode de réalisation.

Le composé de formule (II) peut être obtenu à partir du composé (III) selon l'étape de réaction décrite ci-dessous : 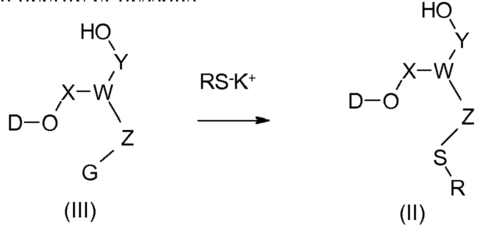 dans laquelle G est un halogène, de préférence le brome ou l'iode.

La réaction décrite ci-dessus s'effectue de préférence dans un solvant anhydre, tel que le toluène anhydre et en présence d'un éther couronne.

Le composé (III) peut être obtenu à partir du composé (IV) selon l'étape de réaction décrite ci-dessous: 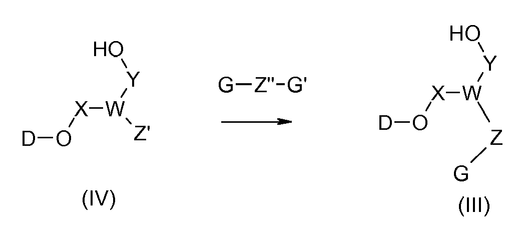 dans laquelle,
G et G' sont des atomes d'halogène identiques ou différents, de préférence G et G' sont des atomes de brome ou d'iode,
Z' est un groupement aminoalkyle en C1-C12, alkoxy en C1-C12, cyclohétéroalkyle oxygéné ou azoté en C3-C12, NCO-alkyle en C1-C12, CON-alkyle en C1-C12,
Z" est un groupement alkyle linéaire ou ramifié en C1-C12, aminoalkyle en C1-C12, alkoxy en C1-C12, cycloalkyle en C3-C12, cyclohétéroalkyle oxygéné ou azoté en C3-C12, NCO-alkyle en C1-C12, CON-alkyle en C1-C12,
le composé dihalogéné G-Z"-G' étant destiné à réagir avec le groupement Z' du compose (IV) pour conduire à la formation du groupement Z-G du composé (III).

L'étape d'obtention du composé (III) s'effectue de préférence en présence d'un hydrure alcalin, tel que NaH.

Le composé (IV) peut être obtenu à partir du composé commercial (V) par protection de la fonction alcool, selon l'étape de réaction suivante :

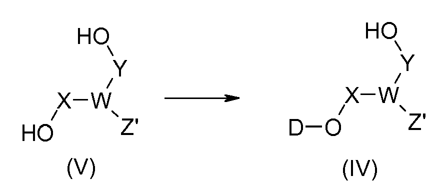

Cette étape de protection de la fonction alcool est effectuée dans des conditions bien connues de l'homme du métier, en fonction du choix de D.

Selon un mode de réalisation, le composé (IV) est obtenu à partir du composé (V) par réaction avec le 4,4'-chlorure de diméthoxytrityle (DMTr-Cl) de préférence dans un solvant, tel que la pyridine afin de protéger la fonction alcool.

Selon un autre mode de réalisation, le composé (IV) est obtenu à partir du composé (V) à partir du chlorure de 9-phénylxantèn-9-yle (pixyl-Cl) dans les conditions décrites dans le document Chattopadhyaya et Reese, Chem. Soc. Chem. Comm., 1978, 639-640.

Selon un autre mode de réalisation, le composé (IV) est obtenu à partir du composé (V) par réaction avec le chlorure de fluorenylmethyloxycarbonyle (Fmoc-Cl) dans des conditions bien connues de l'homme du métier.

Dans les formules précédentes (II) à (V), X, Y, W, Z, D, R, R₁, R₂ ont les mêmes définitions que dans la définition du composé (I) indiquée ci-dessus.

De préférence, le composé (V) de départ est le 1,1,1-tris(hydroxyméthyl)éthane ou l'acide 2,2-bis(hydroxyméthyl) propionique ou le 1,3,5-tris(hydroxyethoxy)benzène ou le 1,3,5-tris(hydroxyméthyl)cyclohexane ou le 2-amino-1,3-propanediol.

### Oligomère du composé thiol

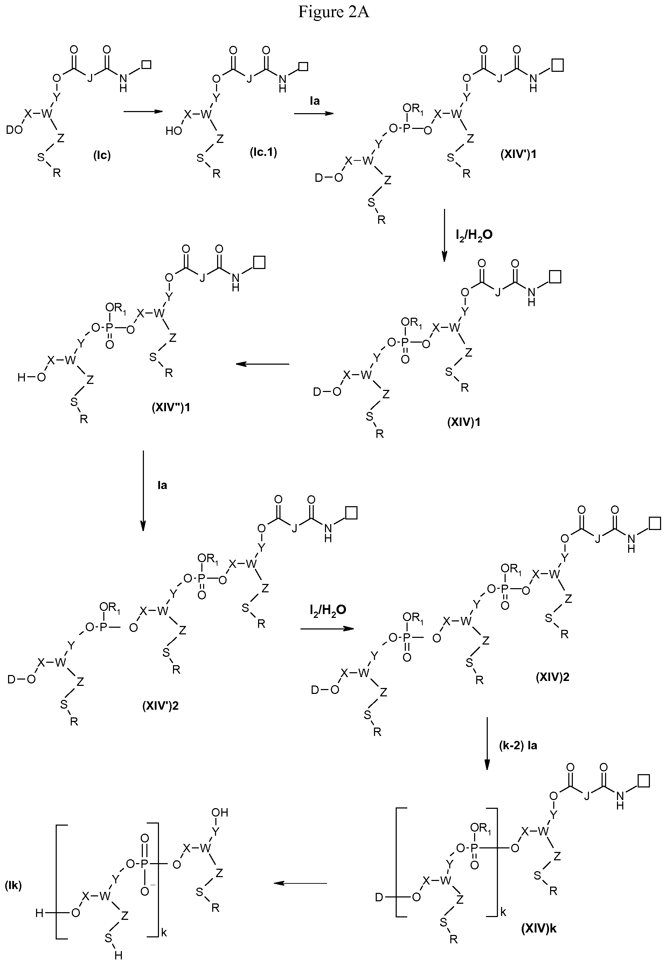

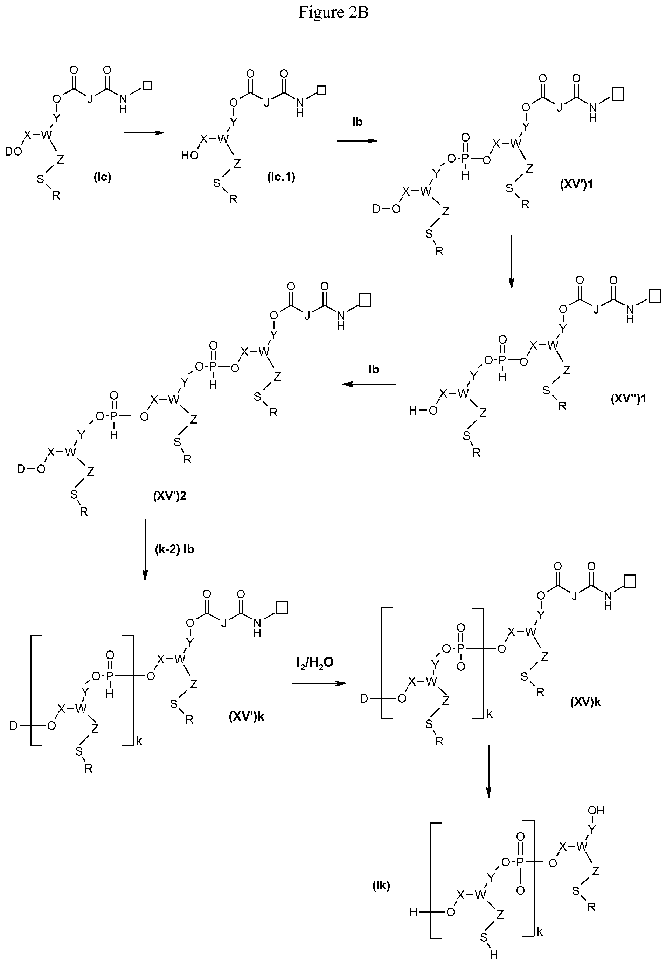

Un oligomère peut être formé à partir de composés thiol de formule (I) précédemment décrits. Le procédé de synthèse de tels oligomères est décrit sur le schéma de la figure 2A pour l'oligomérisation de composés de formule (Ia) et sur le schéma de la figure 2B pour l'oligomérisation de composés de formule (Ib).

Dans une première étape, la fonction alcool du composé (Ic) est déprotégée pour conduire au composé (Ic.1). Cette étape de déprotection s'effectue par des moyens bien connus de l'homme du métier, de préférence en présence de l'acide di ou trichloroacétique pour les groupements DMTr et Pixyl et de pipéridine pour le groupement Fmoc.

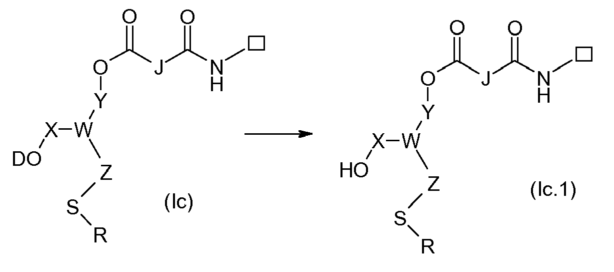

Ensuite, le composé (Ic.1) réagit avec le composé (Ia) ou (Ib) pour conduire respectivement au composé phosphitetriester (XIV')1 ou H-phosphonate diester (XV')1.

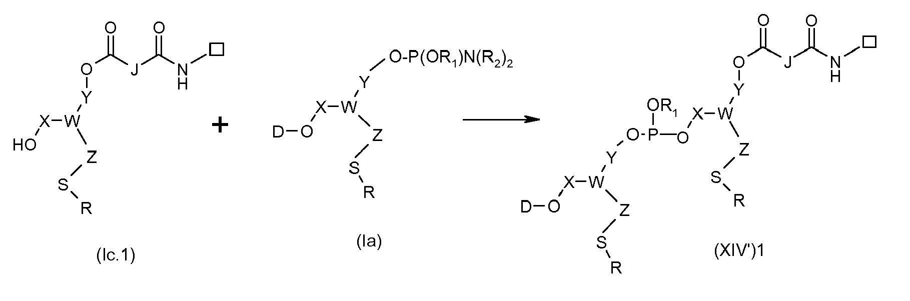

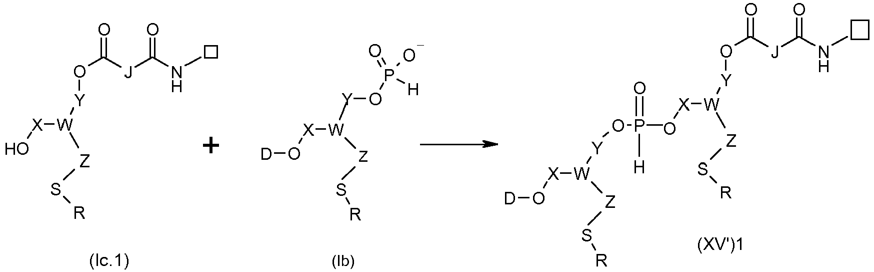

Puis, le composé (XIV')1 est oxydé de préférence en présence de diiode, le diiode étant ensuite déplacé par de l'eau apportant l'atome d'oxygène du lien phosphate triester, pour conduire au composé phosphotriester (XIV)1. Cette étape d'oxydation s'effectue après chaque étape de couplage entre un composé (XIV)i et un composé (Ia).

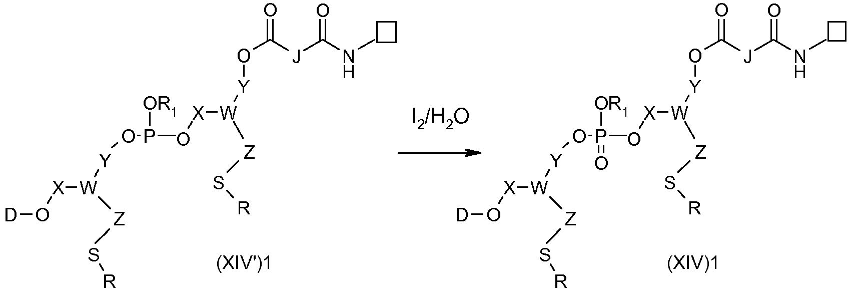

Puis, de la même manière, le composé (XIV)1 réagit avec (k-1) composés (Ia) pour conduire, après (k-1) oxydations, au composé (XIV)ₖ : 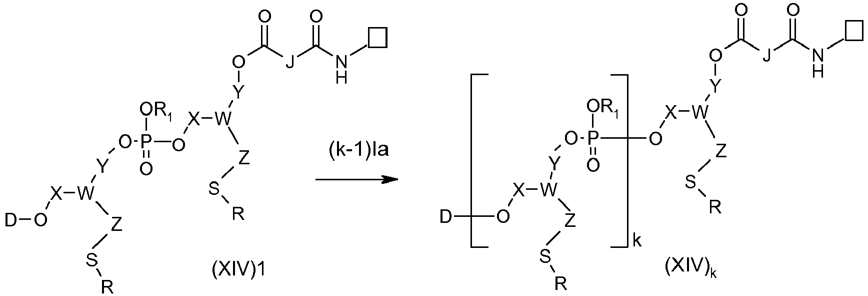 et le composé (XV')1 est déprotégé sur sa fonction alcool pour donner (XV")1 qui réagit avec (k-1) composés (Ib) pour conduire au composé (XV')ₖ :

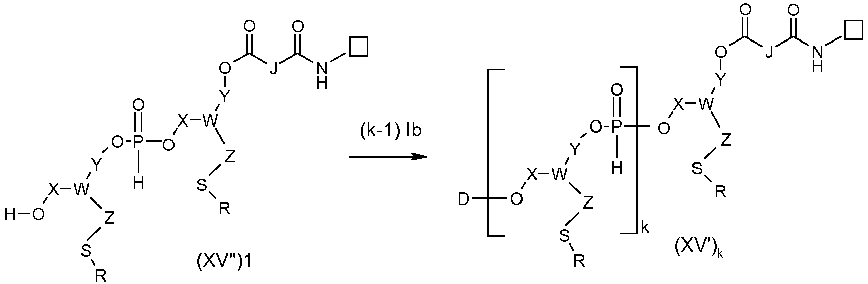

Puis, le composé (XV')ₖ est oxydé, de préférence en présence de diiode et d'eau pour conduire au composé phosphodiester (XV)ₖ.

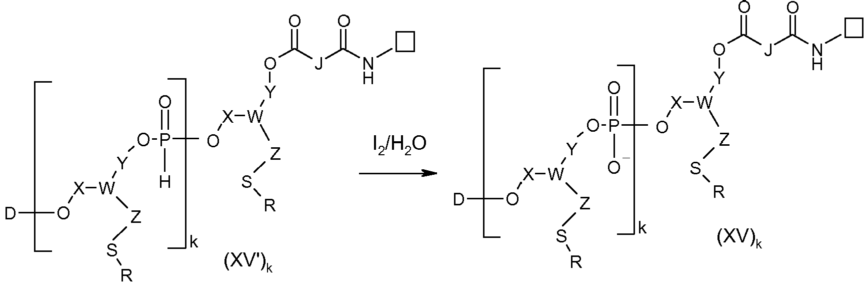

Enfin, une dernière étape éventuelle consiste à déprotéger les composés (XIV)ₖ ou (XV)ₖ pour conduire au même composé (I)ₖ.

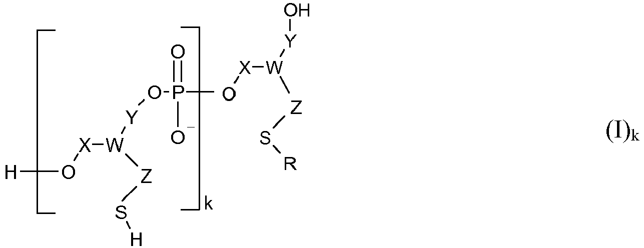

De préférence, l'oligomère résulte de l'oligomérisation de 2 à 12 composés (I), en particulier entre 2 et 8 composés (I), c'est-à-dire que l'oligomère peut comprendre 2, 3, 4, 5, 6, 7 ou 8 composés (I). De préférence, l'oligomère thiol destiné à être greffé sur une surface d'or comprend entre 3 et 8, avantageusement entre 4 et 8 composés (I) et l'oligomère thiol destiné à la conjugaison avec une surface comportant au moins une double liaison carbone-carbone ou une triple liaison carbone-carbone ou des fonctions halogénoacétamide, de préférence des fonctions maléimide ou acrylamide comprend avantageusement entre 2 et 6 composés (I).

Selon un mode de réalisation, l'oligomère est fabriqué uniquement à partir de composés de formule (Ia) ou de composés de formule (Ib). L'oligomérisation s'effectue par réaction entre la fonction alcool déprotégée d'un premier composé (I) et la fonction phosphoramidite ou H-phosphonate d'un second composé (I).

Il est également possible de prévoir la fabrication d'un oligomère à partir d'un mélange de composés (Ia) et de composés (Ib), ce mode de réalisation présentant moins d'intérêt.

De préférence, l'oligomère est fabriqué en utilisant la chimie des phosphoramidites, c'est-à-dire par oligomérisation de composés de type (Ia).

L'oligomérisation peut être effectuée sur support solide ou en solution. De préférence, l'oligomérisation est effectuée sur support solide. En effet, l'oligomérisation en solution implique des étapes de purifications par chromatographie, étapes non économiquement viables, en particulier pour les petites quantités requises dans les applications en diagnostic.

Le support solide greffé par un oligomère d'un composé (Ia) ou d'un composé (Ib) répond à la formule (XVI)ₖ ci-dessous :

(Ic)-(I')ₖ

(XVI)ₖ

dans laquelle :
k représente un entier compris entre 1 et 11,
(Ic) a la même signification que ci-dessus,
(I') représente (I'a) ou (I'b), avec : 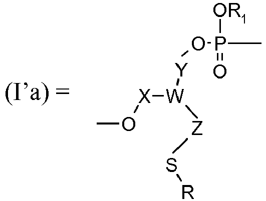
et

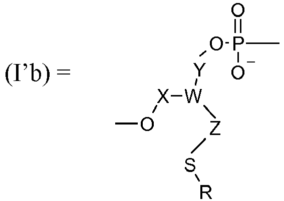

L'oligomère sur support solide formé à partir d'un composé (Ic) et de composés de formule (Ia) répond à la formule (XIV)ₖ suivante : 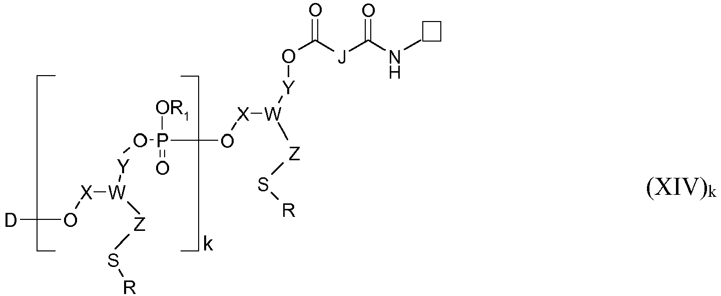 dans laquelle D, X, Y, W, Z, J, R et R₁ ont la même définition que ci-dessus et R peut en outre représenter H, k est un nombre entier compris entre 1 et 11.

L'oligomère sur support solide formé à partir d'un composé (Ic) et de composés de formule (Ib) répond à la formule (XV)ₖ suivante : 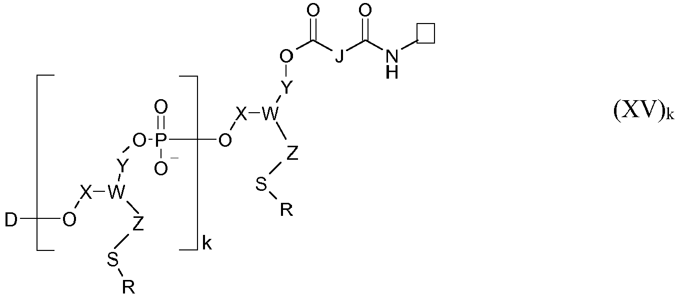 dans laquelle D, X, Y, W, Z, J et R ont la même définition que ci-dessus et R peut en outre représenter H, k est un nombre entier compris entre 1 et 11.

Dans le cas où l'oligomère est formé sur support solide à partir d'un composé (Ic) et de composés (Ib), le composé obtenu (XV)ₖ répond à la même formule que le composé (XIV)ₖ mais dans laquelle R₁ est un atome d'hydrogène après oxydation des liens H-phosphonate diesters.

A l'issue de la réaction d'oligomérisation, on peut prévoir de déprotéger les fonctions thiol par des méthodes classiques et alors R représente H.

### Oligonucléotides modifiés

La présente invention décrit un oligonucléotide modifié, comprenant au moins dix nucléotides et au moins deux composés thiol (I) tels que précédemment décrits.

Dans la présente demande, le terme oligonucléotide désigne une chaîne comprenant de 4 à 100 nucléotides.

Le greffage du composé thiol selon l'invention s'effectue en position 3', dans la chaîne, ou en position 5' d'un oligonucléotide.

Le procédé de préparation d'un tel oligonucléotide modifié comprend au moins :
- une étape de greffage d'un composé (I) sur un oligonucléotide pour donner un 5'-thiol oligonucléotide, ou
- une étape de greffage d'un nucléotide sur un oligomère d'un composé (I) pour donner un 3'-thiol oligonucléotide.

Selon un mode de réalisation, l'oligonucléotide greffé répond à la formule (XIIa) suivante :
□

   -N₁-N₂-...-Nₙ₋₁-Nₙ-(I')_{y}-(M₁-...-Mₘ₋₁-Mₘ)ₚ-(I')_{y'} (XIIa)

   dans laquelle,
   N₁, ...Nₙ représentent indépendamment les uns des autres un nucléotide,
   M₁, ...Mₘ représentent indépendamment les uns des autres un nucléotide,
   (I') représente un composé de formule (I'a) ou (I'b),
   n est un nombre entier allant de 4 à 100,
   m est un nombre entier compris allant de 4 à 100,
   y est un nombre entier allant de 2 à 12,
   p représente 0 ou 1,
   y' est un nombre entier allant de 0 à 12 si p vaut 1 et, y' est égal à 0 si p vaut 0,
   la somme des entiers (y + y') n'étant pas supérieure à 12,
      □ représente un support solide.

L'oligonucléotide modifié (XIIa) présente deux ou plusieurs composés thiol en position 5' d'un oligonucléotide N ou bien dans la chaîne nucléotidique. Il est obtenu par un greffage d'un composé (I) suivi par une élongation de l'oligomère issu de (I) en position 5' d'un oligonucléotide. Puis, dans le cas où p = 1, l'élongation de la chaîne nucléotidique se poursuit. Puis, de la même manière, on peut envisager le greffage d'un ou plusieurs composés (I) supplémentaires en position 5' de l'oligonucléotide M.

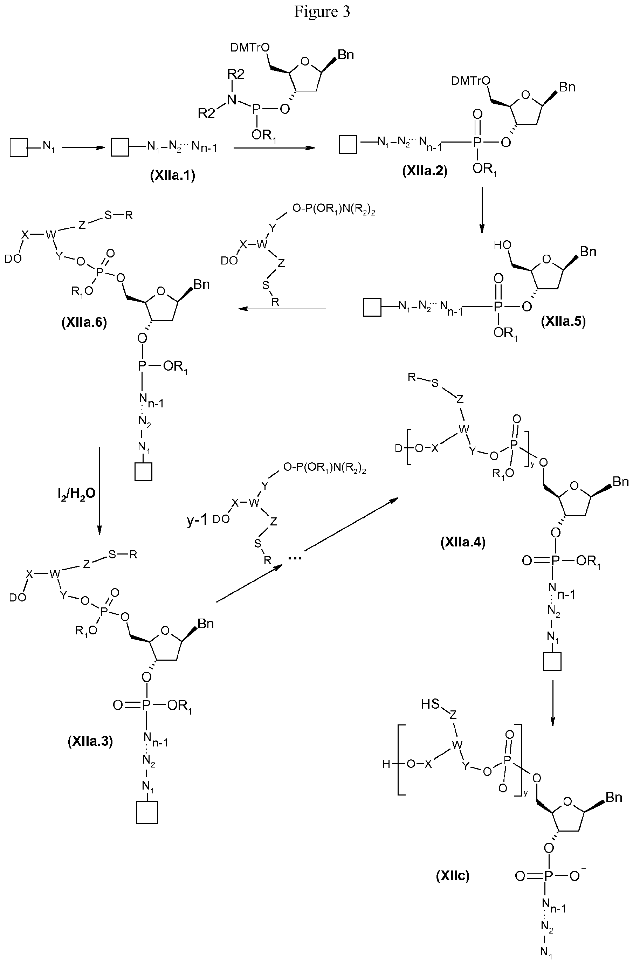

Un schéma d'obtention du composé (XIIa) est décrit sur la figure 3 sur laquelle les composés thiol sont de type (Ia) et p est égal à 0. Les trois premières étapes de fabrication du composé (XIIa) permettent la synthèse d'un oligonucléotide. La synthèse de l'oligonucléotide est effectuée sur support solide par une méthode classique bien connue de l'homme du métier. Lors de la première étape, un premier nucléotide est greffé sur un support solide, puis les autres nucléotides sont greffés suivant des méthodes de synthèse bien connues de l'homme du métier. Le composé suivant est obtenu :
□-N₁-N₂...Nₙ₋₁ ( XIIa.1)

Puis, un autre nucléotide est greffé par une méthode identique pour conduire au composé de formule (XIIa.2).

Lors de l'étape suivante, un composé thiol tel que décrit plus haut, de type (Ia), est greffé en position 5' de l'oligonucléotide (XIIa.2) pour conduire au composé (XIIa.3) :

-N₁-N₂-...-Nₙ₋₁-Nₙ-(I'a) (XIIa.3)

Lors de cette étape, le greffage se fait de manière classique par réaction de la fonction phosphoramidite du composé (Ia) avec la fonction alcool en position 5' du nucléotide terminal du composé (XIIa.2).

Sur le schéma de la figure 3, un exemple de synthèse est décrit avec l'oligomérisation du composé thiol de type (Ia) ; un procédé de synthèse similaire est utilisé pour la synthèse d'oligonucléotides modifiés avec des composés thiol de type (Ib).

Ensuite, l'oligomérisation telle que décrite précédemment, notamment sur les figures 2A et 2B, se poursuit à partir du composé (XIIa.3) ci-dessus, par réaction avec un ou plusieurs composés (Ia) ou (Ib) pour conduire au composé (XIIa.4) de formule :
□

   -N₁-N₂-...-Nₙ₋₁-Nₙ-(I'a)_{y}
ou en formule développée (dans le cas où p = 0) : 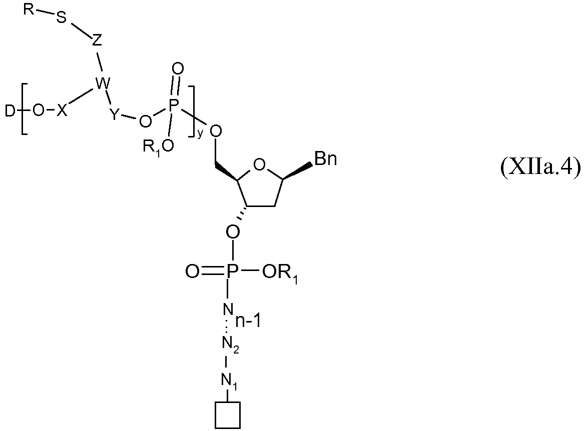 dans laquelle,
D, R, X, Y, W, Z, R₁ ont la même définition que pour le composé (I) et R₁ peut en outre représenter H,
n, y et N₁, N₂, ...Nₙ₋₁ ont la même définition que pour le composé (XIIa),
Bn représente une base classiquement utilisée dans une chaîne nucléotidique.

Dans le cas où l'élongation de l'oligomère s'effectue avec des composés de type (Ib), l'oligonucléotide modifié présente une structure similaire à celle de l'oligonucléotide modifié (XIIa.4) mais dans laquelle R₁ représente H.

Un greffage ultérieur de composés nucléotides M₁, M₂, ...Mₘ est ensuite possible pour aboutir au produit (XIIa) avec p = 1. Dans ces cas, l'élongation s'effectue également sur support solide.

Cette étape de greffage s'effectue de manière classique par des méthodes connues de l'homme du métier.

Selon un autre mode de réalisation, l'oligonucléotide greffé répond à la formule (XIIIa) suivante :

(Ic)-(I')_{y-1}-N₁-N₂-...-Nₙ₋₁-Nₙ-(I')_{y'}-[M₁-M₂-...-Mₘ₋₁-Mₘ]ₚ-(I')_{y"} (XIIIa)

dans laquelle,
N₁, ...Nₙ représentent indépendamment les uns des autres un nucléotide,
M₁, ... Mₘ représentent indépendamment les uns des autres un nucléotide,
(I') représente un composé de formule (I'a) ou (I'b),
n est un nombre entier allant de 4 à 100,
m est un nombre entier allant de 4 à 100,
y est un nombre entier allant de 2 à 12,
y' est un nombre entier allant de 2 à 12,
p vaut 0 ou 1 si y' est différent de 0 et, si y' vaut 0 alors p vaut 0,
y" est un nombre entier allant de 0 à 12 si p vaut 1 et, si p vaut 0 alors y" vaut 0,
la somme des entiers (y+y'+y") n'étant pas supérieure à 12.

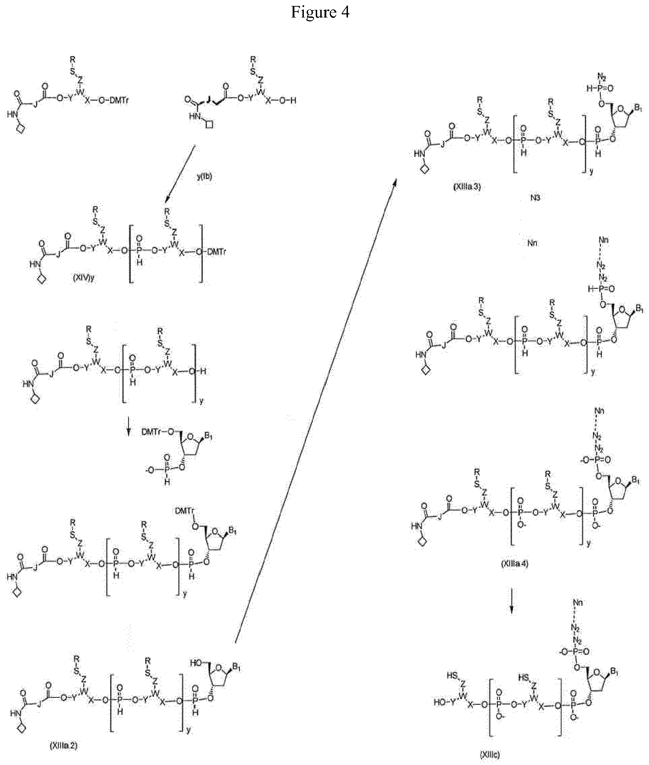

Un schéma représentant un mode de synthèse du composé (XIIIc) est décrit sur la figure 4 en utilisant un oligomère formé à partir d'un composé de type (Ic) où J est un groupement éthyle, et de composés de type (Ib).

La synthèse du composé de formule (XIIIc) comprend une première étape consistant en l'oligomérisation du composé thiol selon l'invention de formule (I) dont le procédé est décrit précédemment pour conduire au composé de formule (XIIIa.1):

(Ic)-(I')_{y-1} (XIIIa.1)

dans laquelle,
(Ic) a la même définition que précédemment,
(I') représente un groupement de type (I'a) ou (I"b), où 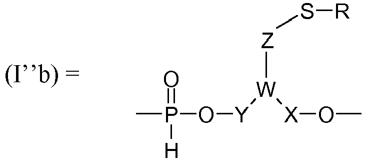
ou en formule développée dans le cas où (I') représente (I"b) :

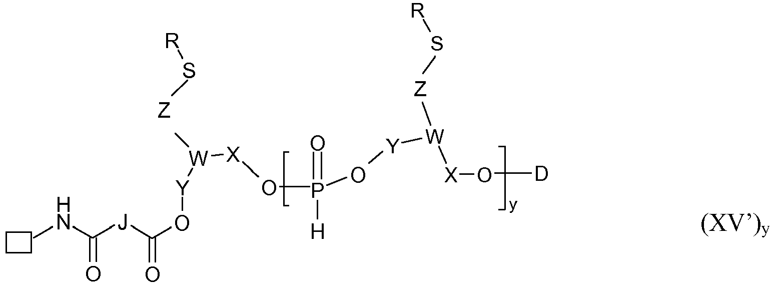

Dans une seconde étape, un premier nucléotide N₁ est greffé sur l'oligomère de formule (XIIIa.1) pour conduire au composé de formule (XIIIa.2) ci-dessous :

(Ic)-(I')_{y-1}-N₁ (XIIIa.2)

Cette étape de greffage s'effectue par réaction entre la fonction alcool déprotégée au bout de la chaîne d'oligomère du composé (XIIIa.1) et la fonction phosphoramidite ou H-phosphonate du premier nucléotide N₁.

L'oligonucléotide modifié est ensuite synthétisé par n'importe quelle méthode connue de l'homme du métier, notamment une méthode classique bien connue de l'homme du métier par réaction entre la fonction alcool en 5' du premier nucléotide présent sur l'oligomère du composé thiol et la fonction phosphoramidite ou H-phosphonate en position 3' d'un second nucléotide. La synthèse se poursuit par des étapes successives similaires d'élongation de la chaîne nucléotidique bien connues de l'homme du métier pour conduire au composé de formule (XIIIa).

Un objet de la présente invention concerne ainsi les composés obtenus à partir des composés de formule (XIIa) et (XIIIa) ci-dessus après coupure de la liaison qui relie l'oligonucléotide modifié au support solide. La coupure de la liaison s'effectue au niveau de la fonction ester.

Ainsi, selon un mode de réalisation décrit dans l'invention, un oligonucléotide modifié non supporté présente la structure (XIIb) suivante :

N₁-N₂-...-Nₙ₋₁-Nₙ-(I'b)_{y}-(M₁-...-Mₘ₋₁-Mₘ)ₚ-(I'b)_{y'} (XIIb)

dans laquelle,
N₁, ...Nₙ représentent indépendamment les uns des autres un nucléotide,
M₁, ...Mₘ représentent indépendamment les uns des autres un nucléotide,
(I'b) représente un composé de formule telle que définie plus haut,
n est un nombre entier allant de 4 à 100,
m est un nombre allant de 4 à 100,
y est un nombre entier allant de 2 à 12,
p représente 0 ou 1,
y' est un nombre entier allant de 0 à 12 si p vaut 1 et, y' est égal à 0 si p vaut 0,
la somme des entiers (y + y') n'étant pas supérieure à 12.

Le retrait du support s'effectue en deux étapes avec premièrement une base forte non nucléophile (piperidine ou DBU) pour éliminer les groupements cyanoéthyle si présents (cas des phosphoramidites) et deuxièmement par un procédé classique connu de l'homme du métier, de préférence par un traitement du composé (XIIa) avec de l'hydroxyde d'ammonium (NH₄OH). Il est nécessaire d'éliminer les groupements cyanoéthyle avant la déprotection des groupements thiol car ils forment de l'acrylonitrile lors de leur élimination qui réagit fortement avec les fonctions thiol.

Selon un autre mode de réalisation décrit dans l'invention, un oligonucléotide modifié non supporté présente la structure (XIIIb) suivante :

(Ic')-(I'b)_{y-1}-N₁-N₂-...-Nₙ₋₁-Nₙ-(I'b)_{y'}-[M₁-M₂-...-Mₘ₋₁-Mₘ]ₚ-(I'b)_{y"} (XIIIb)

dans laquelle,
N₁, ...Nₙ représentent indépendamment les uns des autres un nucléotide,
M₁, ... Mₘ représentent indépendamment les uns des autres un nucléotide,
(Ic') représente le composé obtenu à partir de (Ic) par coupure de la liaison ester avec le support solide, et a de préférence la structure 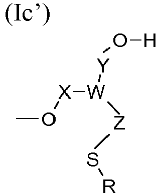
(I'b) représente un composé de formule telle que définie plus haut,
n est un nombre entier allant de 4 à 100,
m est un nombre entier allant de 4 à 100,
y est un nombre entier allant de 2 à 12,
y' est un nombre entier allant de 0 à 12,
p vaut 0 ou 1 si y' est différent de 0 et, si y' vaut 0 alors p vaut 0,
y" est un nombre entier allant de 0 à 12 si p vaut 1 et, si p vaut 0 alors y" vaut 0,
la somme des entiers (y + y' + y") n'est pas supérieure à 12.

Le retrait du support s'effectue par un procédé classique connu de l'homme du métier, de préférence par un traitement du composé (XIIIa) avec de l'hydroxyde d'ammonium (NH₄OH).

Selon la présente invention, dans le cas où p=0, l'oligonucléotide modifié répond à la formule développée (XIIb) : 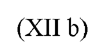 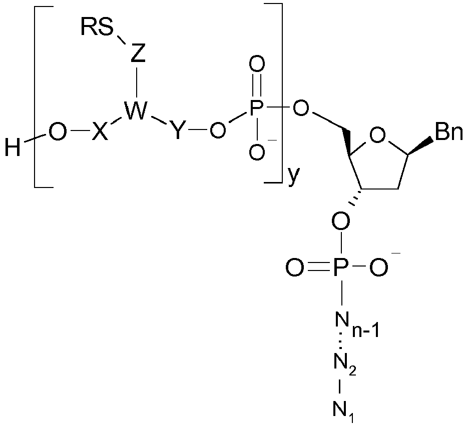 dans laquelle n, y, N₁,..., Nₙ₋₁, X, Y, Z, W et R ont la même définition que ci-dessus, et Bₙ représente la base du n-ième nucléotide.

Selon la présente invention, dans le cas où p=0, l'oligonucléotide modifié répond à la formule développée (XIIIb) : 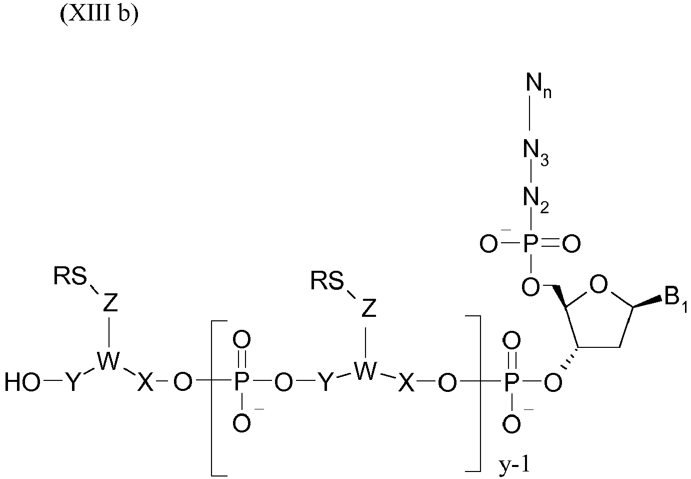 dans laquelle n, y, N₂,..., Nₙ, X, Y, Z, W et R ont la même définition que ci-dessus, et B₁ représente la base du 1er nucléotide.

La présente invention décrit les oligonucléotides modifiés (XIIc) et (XIIIc) obtenus respectivement à partir des composés (XIIa) et (XIIIa) par déprotection de la fonction thiol et coupure de la liaison reliant le composé au support solide (Figures 3 et 4 respectivement).

Dans le cas où l'oligonucléotide est modifié par un ou plusieurs composés thiol de type (Ia), après déprotection de la fonction thiol et de la fonction phosphoramidite du composé (XIIa) par un traitement connu de l'homme du métier, le composé de formule (XIIc) est obtenu :

N₁-N₂-...-Nₙ₋₁-Nₙ-(I")_{y}-(M₁-...-Mₘ₋₁-Mₘ)ₚ-(I")_{y'} (XIIc)

dans laquelle,
N₁, ...Nₙ représentent indépendamment les uns des autres un nucléotide,
M₁, ...Mₘ représentent indépendamment les uns des autres un nucléotide, 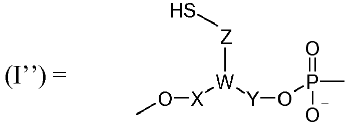
n est un nombre entier allant de 4 à 100,
m est un nombre entier allant de 4 à 100,
y est un nombre entier compris allant de 2 à 12,
p représente 0 ou 1,
y' est un nombre entier allant de 0 à 12 si p vaut 1 et, y' est égal à 0 si p vaut 0,
la somme des entiers (y + y') n'étant pas supérieure à 12.
ou en formule développée dans le cas où p=0 : 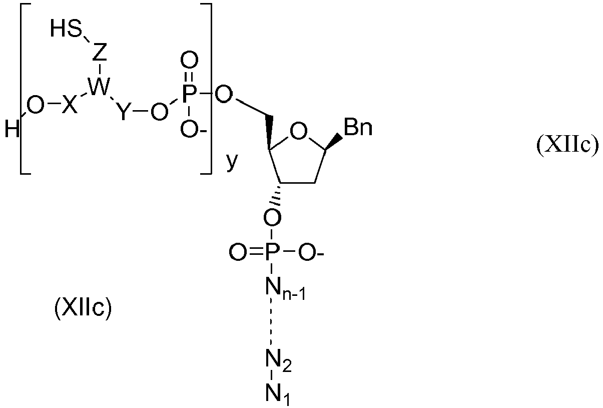 dans laquelle,
X, Y, W, Z, R₁ ont la même définition que pour le composé (I),
N₁, ...Nₙ, n, y ont la même définition que pour le composé (XIIa),
Bn représente une base classiquement utilisée dans une chaîne nucléotidique.

Dans le cas où l'oligonucléotide est modifié par un ou plusieurs composés thiol de type (Ib), après déprotection de la fonction thiol du composé (XIIIa) par un traitement connu de l'homme du métier, le composé de formule (XIIIc) est obtenu :

(Ic')-(I')_{y-1}-N₁-N₂-...-Nₙ₋₁-Nₙ-(I")_{y'}-[M₁-M₂-...-Mₘ₋₁-Mₘ]ₚ-(I")_{y"} (XIIIc)

dans laquelle,
N₁, ...Nₙ représentent indépendamment les uns des autres un nucléotide,
M₁, ... Mₘ représentent indépendamment les uns des autres un nucléotide,
(Ic') représente le composé obtenu à partir de (Ic) par coupure de la liaison ester avec le support solide, 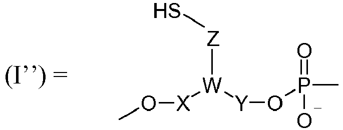
n est un nombre entier allant de 4 à 100,
m est un nombre entier allant de 4 à 100,
y est un nombre entier allant de 2 à 12,
y' est un nombre entier allant de 2 à 12,
p vaut 0 ou 1 si y' est différent de 0 et, si y' vaut 0 alors p vaut 0,
y" est un nombre entier allant de 0 à 12 si p vaut 1 et, si p vaut 0 alors y" vaut 0,
la somme des entiers (y + y' + y") n'étant pas supérieure à 12.
ou en formule développée dans le cas où y' = p = 0 : 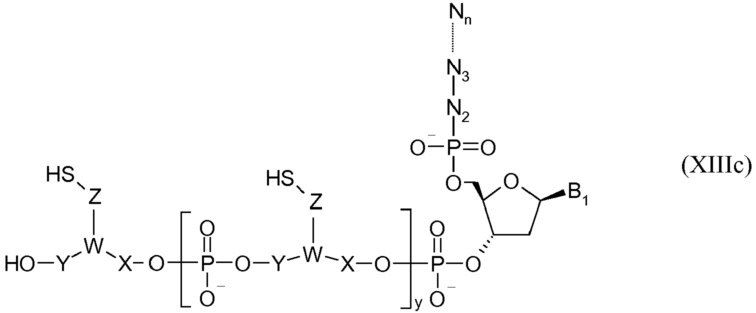 dans laquelle,
X, Y, W, Z ont la même définition que pour le composé (I),
N₂, ...Nₙ, n et y ont la même définition que pour le composé (XIIa),
Bn correspond à une base classiquement utilisée dans une chaîne nucléotidique.

Lors de la synthèse de l'oligonucléotide, la fonction thiol est de préférence protégée. En effet, la fonction thiol peut réagir avec la fonction phosphoramidite entrante.

L'étape de déprotection des fonctions thiol et d'élimination du support solide peut s'effectuer en une seule étape de traitement de l'oligonucléotide modifié (XIIa) ou (XIIIa).

L'élimination du support solide peut également s'effectuer dans une première étape puis la déprotection des fonctions thiol est ensuite effectuée dans une seconde étape.

L'oligonucléotide final (XIIc) (respectivement l'oligonucléotide final (XIIIc)) est obtenu indépendamment du composé thiol de départ, que ce soit à partir du composé (Ia) ou du composé (Ib). En effet, quelque soit le monomère (Ia) ou (Ib) utilisé, l'unité monomère thiol résultant de la réaction d'oligomérisation correspond au composé (I") décrit précédemment.

Les supports greffés de formules (XVI)ₖ décrits ci-dessus permettent d'amorcer la synthèse d'oligonucléotides. On peut notamment prévoir de préparer de façon industrielle ou semi-industrielle des supports solides greffés par des séquences d'oligomères qui seront employées comme séquence polythiol en position 3' d'un oligonucléotide.

Selon un mode de réalisation de l'invention, la séquence de nucléotides (N₁-N₂-...-Nₙ₋₁-Nₙ) et, éventuellement, la séquence de nucléotides (M₁-M₂-...-Mₘ₋₁-Mₘ) de l'oligonucléotide modifié de l'invention, tel que décrit ci-dessus sont spécifiques d'un virus, d'une bactérie ou d'un gène responsable ou impliqué dans une maladie.

Au sens de la présente demande, par « spécifique », il est entendu que la séquence (N₁-N₂-...-Nₙ₋₁-Nₙ) et éventuellement, la séquence (M₁-M₂-...-Mₘ₋₁-Mₘ) sont complémentaires de tout ou partie d'au moins une séquence d'acide nucléique cible comprise dans un gène ou dans le génome d'un virus ou d'une bactérie, et caractéristique de ces derniers. Il est ainsi entendu que l'hybridation de la séquence (N₁-N₂-...-Nₙ₋₁-Nₙ) et éventuellement, de la séquence (M₁-M₂-...-Mₘ₋₁-Mₘ) avec la
partie ou la totalité de la séquence cible qui lui correspond conduit à la formation d'un duplex d'acides nucléiques possédant au plus 2 mésappariements. Dans un mode de réalisation de l'invention, le terme « spécifique » signifie que le duplex formé ne comprend qu'un seul ou deux mésappariements. Avantageusement, le duplex formé ne contient aucun mésappariement. Par « séquence caractéristique d'un gène ou du génome d'un virus ou d'une bactérie », il est entendu une région génomique, chromosomique ou plasmidique d'un organisme, d'un virus ou d'une bactérie, présentant un agencement de nucléotides qui n'est pas retrouvé dans les autres organismes, en raison de variations génétiques uniques.

Dans le cadre de l'invention, les termes « oligonucléotide modifié » et « sonde » sont utilisés indifféremment et désignent un oligonucléotide de 4 à 100 nucléotides et possédant au moins 2 fonctions thiol, tel que décrit ci-dessus. Avantageusement, lorsqu'il est utilisé pour le génotypage, l'oligonucléotide modifié de l'invention comprend de 13 à 20 nucléotides, avantageusement 14 ou 15 nucléotides. La sonde possède donc la capacité de se greffer sur une surface appropriée, telle qu'une surface revêtue d'or ou de groupements maléimide, et possède également la capacité de s'hybrider, *via* sa partie nucléotidique, avec une séquence nucléotidique cible.

L'oligonucléotide modifié de l'invention comprend de 2 à 12 fonctions thiol, en particulier entre 2 et 8 fonctions, c'est-à-dire que l'oligonucléotide modifié peut comprendre 2, 3, 4, 5, 6, 7 ou 8 fonctions. De préférence, l'oligonucléotide modifié destiné à être greffé sur une surface d'or comprend entre 3 et 8, avantageusement entre 4 et 8 fonctions thiol. L'oligonucléotide modifié destiné à la conjugaison avec une surface comportant au moins une double liaison carbone-carbone ou une triple liaison carbone-carbone ou des fonctions halogénoacétamide, de préférence des fonctions maléimide ou acrylamide comprend avantageusement entre 2 et 8 fonctions thiol, et préférablement 4 fonctions thiol.

Le terme « séquence cible », tel qu'utilisé dans le cadre de l'invention, désigne une séquence complémentaire ou partiellement complémentaire à la sonde de l'invention, et qui est amplifiée à partir d'un gène ou du génome d'un virus ou d'une bactérie, et de préférence à partir d'une région caractéristique de ces derniers.

Le terme « virus » désigne une entité biologique nécessitant une cellule-hôte pour se répliquer, et se composant au minimum d'un acide nucléique et de protéines, l'acide nucléique pouvant être de l'ADN et/ou de l'ARN, simple ou double-brin. Les virus comprennent ceux capables de parasiter les procaryotes (par exemple les Myoviridae, les Siphoviridae, les Podoviridae, les Microviridae et les Inoviridae), ceux capables de parasiter les plantes (phytovirus tel que le tobamovirus), les insectes (par exemple les baculovirus), les champignons, et l'homme. Dans le cadre de l'invention, les virus comprennent également les arbovirus. Les arbovirus rassemblent des virus morphologiquement hétérogènes appartenant à plusieurs genres distincts comprenant plus particulièrement les genres *Flavivirus* (famille *Flaviviridaae*), *Alphavirus* (famille *Togaviriadae*), *Coltivirus* (famille *Reoviridae*), *Phlebovirus* (famille *Bunyaviriadae*). Plus largement la famille des *Flaviviridae* comprend notamment les genres *Flavivirus* (virus de la fièvre jaune, virus du Nil occidental (West Nile virus), virus de l'encéphalite à tique (TBE), virus de l'encéphalite japonaise, virus de l'encéphalite de Saint-Louis, virus usutu et virus de la Dengue), *Hepacivirus* (virus de l'hépatite C) et *Pestivirus* (virus de la diarrhée virale bovine (BVD), virus de la peste porcine). La famille des *Togaviridae* comprend notamment les genres *Alphavirus* (virus de Sindbis, virus de Ross River, virus O'nyong'nyong, virus du Chikungunya) et *Rubivirus* (virus Rubella, virus de la rubéole). La famille des *Reoviridae* comprend notamment les virus affectant le système digestif (tel que le *Rotavirus*), ou le système respiratoire. La Famille des *Bunyaviridae* comprend notamment les genres *Hantavirus* (Hantavirus pulmonary syndrome, Korean hemorrhagic fever), *Nairovirus* (*Dugbe virus*), *Orthobunyavirus* (*Bunyamwera virus*), *Phlebovirus* (*Fièvre de la vallée du Rift*) et *Tospovirus.*

Dans le cadre de l'invention, les virus capables d'infecter l'homme comprennent les *Vesiculovirus* (virus de la stomatite vésiculeuse, ..), les *Lyssavirus* (virus de la Rage, virus de la chauve souris australienne,). les *Picornavirus* (*Rhinovirus, Poliovirus,* virus de l'hépatite A...), les *Herpesvirus* (varicelle, zona, cytomegalovirus CMV, virus Epstein Barr EBV ...), les *Orthomyxovirus* (virus de la grippe ...), les *Paramixovirus* (virus de la rougeole, virus des oreillons ...),les *Poxvirus* (virus de la variole ..),*Coronavirus* (SRAS ....), les *Filovirus* (Ebola ...), les *Hepadnavirus* (VHB) et les Retrovirus (HIV, HTLV...).

Le terme « bactérie » désigne tout organisme vivant procaryote se caractérisant par une absence de noyau et d'organites, et pourvu d'une paroi cellulaire. Ce terme comprend notamment les bactéries susceptibles d'être pathogènes et de causer des maladies et/ou des infections chez l'humain, telles que *Corynebacterium diphtheriae* (diphtérie), *Treponema pallidum* (syphilis), *Mycobacterium tuberculosis* (tuberculose), *Mycobacterium leprae* (lèpre), *Neisseria gonorrhoeae* (gonorrhée), les *Rickettsia* (typhus), *Clostridium tetani* (tétanos), *Vibrio cholerae* (choléra), *Pseudomonas aeruginosa* et les *Staphylococcus* (pathogènes opportunistes). Ce terme comprend également les bactéries impliquées dans les risques transfusionnels et dans les infections nosocomiales telles que, par exemple, les bactéries Gram-négatif *Escherichia coli, Klebsiella oxytoca Klebsiella pneumoniae, Serratia marcescens, Yersinia enterocolitica, Enterobacter aerogenes, Acinetobacter baumanii, et Pseudomonas aeruginosa,* ou les bactéries à Gram-positif *Staphylococcus epidermidis, Staphylococcus aureus, Bacillus cereus, Streptococcus pyogenes, Propionibacterium acnes* et *Clostridium perfringens.*

Selon un mode de réalisation de l'invention, la séquence de nucléotides (N₁-N₂-...-Nₙ₋₁-Nₙ) et, éventuellement, la séquence de nucléotides (M₁-M₂-...-Mₘ₋₁-Mₘ) sont choisies parmi :
- les séquences de SEQ ID NO : 1, de SEQ ID NO : 4, de SEQ ID NO : 27, de SEQ ID NO : 28, de SEQ ID NO : 35 ou de SEQ ID NO : 36, spécifiques du virus de l'hépatite C (VHC),
- les séquences de SEQ ID NO : 16, de SEQ ID NO : 17 ou de SEQ ID NO : 40, spécifiques des flavivirus,
- la séquence de SEQ ID NO : 18 ou de SEQ ID NO : 41, spécifique du virus de la Dengue, ou
- la séquence de SEQ ID NO : 19, spécifique du West Nile virus (WNV).

Selon un mode de réalisation de l'invention, la séquence de nucléotides (N₁-N₂-...-Nₙ₋₁-Nₙ) et, éventuellement, la séquence de nucléotides (M₁-M₂-...-Mₘ₋₁-Mₘ) ont une structure de type anomère alpha, anomère béta, linéaire, ou « snail ». Lorsque la séquence de nucléotides a une structure de type anomère béta, l'oligomère modifié de l'invention (dont les thiols sont situés en 5') s'hybride de manière antiparallèle avec la séquence cible. Lorsque la séquence de nucléotides a une structure de type anomère alpha, l'oligomère modifié de l'invention (dont les thiols sont situés en 3') s'hybride de manière parallèle avec la séquence cible.

### Fonctionnalisation de surface

Les oligonucléotides modifiés selon l'invention peuvent être déposés sur un substrat afin de fonctionnaliser la surface de ce substrat. Le substrat peut être métallique ou bien en polymère, conducteur ou non conducteur. Il peut être plan (en partie ou dans sa totalité) ou courbe, et peut avantageusement prendre une forme sphérique.

Selon un mode de réalisation, le substrat est non planaire, par exemple sous forme de microparticules ou nanoparticules. Les oligonucléotides modifiés selon l'invention peuvent alors être greffés sur ces microparticules ou nanoparticules. De préférence, ces particules sont magnétiques. En effet, ces particules magnétiques peuvent alors être facilement amenées à la surface d'une électrode ou au fond des puits d'une microplaque, en vue d'un test de détection, de génotypage ou de séquençage, par application d'un aimant. La mise en oeuvre d'un support non planaire de type microparticule ou nanoparticule dans le cadre de la méthode de détection de l'invention permet avantageusement d'augmenter la superficie de la surface sur laquelle peuvent se greffer les oligonucléotides modifiées de l'invention, et permet ainsi d'augmenter la proportion d'hybridations entre les sondes et les séquences cibles.

Selon un mode de réalisation, le substrat est métallique, par exemple en cuivre ou en titane, et est recouvert partiellement ou sur toute sa surface d'un film d'or ou de platine, de préférence d'or.

Selon un mode de réalisation, le substrat est en polymère, de préférence un polymère conducteur.

Selon un mode de réalisation, le substrat peut comporter des zones de réception recouvertes d'un film d'or ou de platine ou recouvertes de fonctions alcényles, alcynyles ou halogénoacétamide, telles que des fonctions maléimide ou acrylamide, sur lesquelles l'oligonucléotide modifié est déposé.

Selon un mode de réalisation de l'invention, les groupements comportant au moins une double liaison carbone-carbone ou une triple liaison carbone-carbone sont choisi parmi les alcènes activés par une fonction carbonyle en alpha, de préférence les alcènes sont choisis parmi les groupements maléimide et acrylamide.

Selon un mode de réalisation de l'invention, les groupements halogénoacétamide sont choisis parmi les groupements bromoacétamido et iodoacétamido.

Selon un mode de réalisation de l'invention, les groupements comportant au moins une triple liaison carbone-carbone sont choisis parmi les alcynes activés par une fonction carbonyle en alpha, de préférence les alcynes sont choisis parmi les groupements 2-propynamide.

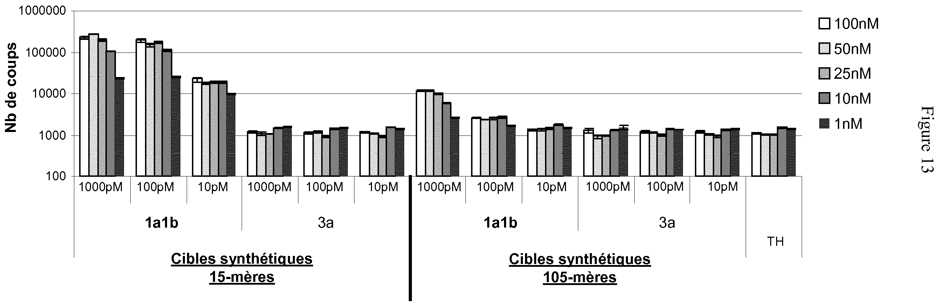

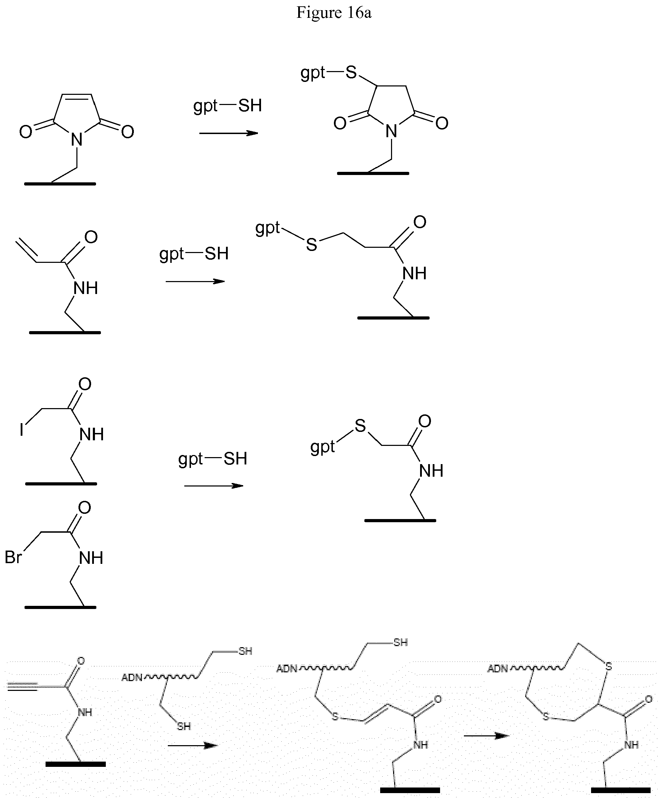

Comme illustré sur la figure 16a, dans laquelle le composé gpt - SH représente l'oligonucléotide modifié selon l'invention, la fonction thiol réagit avec la double liaison carbone-carbone ou la triple liaison carbone-carbone activée par une fonction carbonyle en alpha. Du haut vers le bas de la figure 13a, la première réaction de fonctionnalisation correspond à une surface greffée maléimide, la seconde réaction correspond à une surface greffée acrylamide, la troisième réaction correspond à une surface greffée iodoacétamido ou bromoacétamido et la quatrième réaction correspond à une surface greffée 2-propynamide effectuée dans le cas d'un oligonucléotide modifié selon l'invention comprenant deux composés thiols.

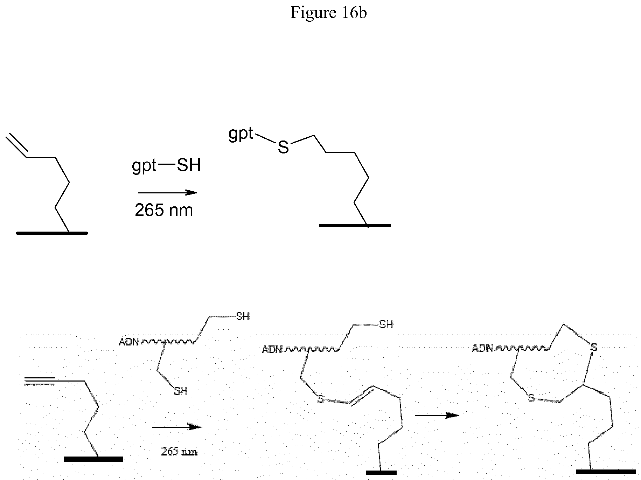

Dans le cas de la figure 16b, la surface est greffée par des groupements alcényles (1^{ère} réaction) et alcynyles (2^{nde} réaction) non activés. La réaction entre la fonction thiol de l'oligonucléotide modifié et les groupements de surface s'effectue à l'aide d'une activation lumineuse (λ = 265 nm). La première réaction de fonctionnalisation est effectuée à l'aide d'un oligonucléotide modifié monothiol schématiquement représenté par gpt - SH et la seconde réaction est effectuée à l'aide d'un oligonucléotide modifié dithiol.

Dans le cas où le support est greffé par des fonctions alcynyles, la fonctionnalisation de surface par un oligonucléotide modifié polythiol est très intéressante car elle conduit à une structure cyclique grâce à deux réactions successives entre une première fonction thiol et la fonction -yne dans une première étape puis entre une deuxième fonction thiol et la fonction -ène résultante dans une seconde étape (figures 16a et 16b).

Selon un mode de réalisation préféré, le substrat est greffé par des groupements maléimide ou acrylamide.

Ainsi, l'invention permet par exemple de fonctionnaliser une surface d'or par création de liaison(s) or-soufre entre la surface du substrat et l'oligonucléotide modifié ou bien de fonctionnaliser une surface greffée par des fonctions maléimide ou acrylamide par création de liaison(s) thioethers.

La fixation des oligonucléotides modifiés à la surface du substrat s'effectue en trempant la surface à traiter dans une solution comprenant l'oligonucléotide modifié selon l'invention tel que décrit plus haut. On prévoit généralement ultérieurement une ou plusieurs étapes de lavage et de séchage. En général, la solution d'oligonucléotides modifiés est à une concentration comprise entre 0,10µM et 500 µM, de préférence entre 0,5 µM et 100 µM lorsque la surface du substrat est en or et entre 50 nM et 200 nM, de préférence entre 75 nM et 150 nM lorsque la surface du substrat est formée de maléimide. Le trempage est suivi d'un lavage pour éliminer ce qui n'a pas réagi.

La présence de plusieurs atomes de soufre sur l'oligonucléotide permet de créer plusieurs liaisons or-soufre, ou plusieurs liaisons thioether, ce qui permet de stabiliser l'oligonucléotide sur la surface.

Un objet de l'invention comprend donc un substrat greffé par au moins un oligonucléotide modifié tel que décrit ci-dessus, ledit substrat comportant au moins une zone de réception revêtue d'une substance tolérant le greffage dudit oligonucléotide modifié. Selon un mode de réalisation de l'invention, le substrat a la forme d'une électrode. Selon un autre mode de réalisation de l'invention, le substrat a la forme d'une microplaque possédant 96 puits ou plus.

Selon un mode de réalisation de l'invention, le substrat greffé selon l'invention est en métal, de préférence en cuivre ou en titane, et comprend au moins une zone de réception revêtue d'un film d'or ou de platine. Selon un autre mode de réalisation de l'invention, le substrat greffé est en plastique, de préférence en polystyrène et la zone de réception est revêtue de groupements maléimide et/ou comporte à sa surface au moins une double liaison carbone-carbone ou une triple liaison carbone-carbone ou des fonctions halogénoacétamide, de préférence des fonctions maléimide ou acrylamide.

Selon un mode de réalisation de l'invention, la densité de greffage de l'oligonucléotide modifié selon l'invention obtenue sur la zone de réception résulte de l'utilisation de solutions de greffage de 10 nM par puits à 500 nM par puits, lorsqu'une plaque 96 puits est utilisée comme substrat. Avantageusement, la densité de greffage de l'oligonucléotide modifié selon l'invention sur la zone de réception du substrat est obtenue en utilisant une solution de greffage de 100 nM par puits lorsqu'une plaque 96 puits est utilisée comme substrat.

### Fixation à des marqueurs ou ligands

Les oligomères modifiés de la présente invention peuvent également être liés par un ou plusieurs points d'attachement à des marqueurs ou des ligands pouvant être par exemple, des marqueurs enzymatiques, chromogènes, fluorogènes, radioactifs, ou chemiluminescents, des métaux, des ions métalliques, des hormones, des protéines, des peptides, des saccharides, oligosaccharides, des agents nucléolytiques ou protéolytiques, des agents de liaison, tels qu'une biotine, un antigène, un haptène, un anticorps ou un récepteur. Avantageusement, ce marqueur ou ligand permet de détecter l'évènement d'hybridation entre l'oligonucléotide modifié de l'invention et une séquence cible représentative d'un gène, d'un virus ou d'une bactérie.

Selon un autre mode de réalisation de l'invention, le marqueur ou ligand se lie à la séquence nucléotidique cible représentative d'un gène, d'un virus ou d'une bactérie, et permet de détecter l'évènement d'hybridation entre l'oligonucléotide modifié de l'invention et la séquence cible.

### Méthode de détection

Un autre objet de la présente invention concerne une méthode de détection d'au moins un acide nucléique cible dans un échantillon biologique, comprenant une étape de détection dudit acide nucléique cible avec au moins une sonde de détection formée par un oligonucléotide modifié tel que décrit ci-dessus.

Selon un mode de réalisation de l'invention, la méthode de détection comprend les étapes :
- d'obtention d'au moins un acide nucléique « source » à partir d'un échantillon biologique,
- de production d'un amplicon par amplification d'un acide nucléique cible à partir de l'acide nucléique source, et
- de détection de l'hybridation entre l'amplicon et au moins une sonde de détection formée par un oligonucléotide modifié tel que décrit ci-dessus.

Par « échantillon biologique», il est entendu toute substance contenant ou suspectée de contenir un acide nucléique, tel que l'ADN ou l'ARN, et obtenue à partir d'un humain, d'un animal, d'un végétal ou de toute composition liquide ou solide. L'échantillon biologique inclut par exemple les échantillons de tissus ou de fluides isolés à partir d'un individu ou d'individus, comprenant mais ne se limitant pas à la peau, le sang, le plasma, le sérum, le fluide spinal, le fluide lymphatique, le fluide synovial, l'urine, les larmes, les cellules sanguines, la moelle osseuse, les organes, les tumeurs, ainsi que les échantillons de constituants de cultures cellulaires *in vitro.* L'échantillon biologique peut être avantageusement traité pour détruire la structure des tissus ou des cellules, afin d'en placer les constituants intracellulaires en solution.

Par « amplicon », il est entendu une molécule d'acide nucléique générée pendant une procédure d'amplification d'une séquence nucléique cible caractéristique du gène, du virus ou de la bactérie contenus dans l'échantillon biologique. Selon un mode de réalisation de l'invention, l'amplicon est généré par la technique de réaction de polymérisation en chaine (en anglais « polymerase chain reaction », ou PCR). L'amplicon utilisé dans la méthode de détection de l'invention possède avantageusement une taille allant de 75 pb à environ 500 pb. Il apparait notamment qu'un des avantages de la méthode de l'invention réside dans la possibilité d'utiliser des amplicons ayant une longueur d'une ou de plusieurs centaines de nucléotides, ce qui se révèle impossible dans les méthodes de détection connues dans l'état de la technique. La méthode de détection de l'invention permet ainsi de sélectionner et d'amplifier des séquences cibles plus grandes dans les gènes, virus ou bactéries.

Selon un mode de réalisation de l'invention, la méthode de détection permet de déterminer le génotype et/ou le sous-type d'un virus présent dans un échantillon biologique. L'amplicon est plus spécifiquement généré par amplification d'une séquence nucléotidique cible, correspondant à une région génomique du virus portant des informations relatives au génotype et/ou au sous-type viral, et la détection est effectuée avec une sonde spécifique d'un génotype et/ou d'un sous-type viral particulier. La méthode de l'invention rend ainsi possible d'effectuer la détection sur la base de séquences cibles contenant plus d'informations sur le type, ou le sous-type viral ou bactérien. Cet avantage résulte directement de l'oligonucléotide modifié selon l'invention, de son mode et de sa capacité de fixation à la zone de fixation du substrat. Avantageusement, il n'est donc plus indispensable de définir des amorces permettant d'amplifier une séquence cible la plus courte possible à partir du génome viral ou bactérien. La méthode de la présente invention permet au contraire de sélectionner des amorces localisées dans des régions conservées dans les familles de virus ou de bactéries visées, et d'amplifier des séquences cibles plus longues. Il en résulte des économies significatives et une facilité d'utilisation accrue dans la mesure où les mêmes amorces peuvent être utilisées pour amplifier une zone génomique retrouvée dans plusieurs types et/ou sous-type viraux ou bactériens, sans craindre de voir les résultats de l'hybridation faussés par la taille de l'amplicon utilisé.

Selon un mode de réalisation de l'invention, l'étape de production de l'amplicon de la méthode de détection de l'invention est réalisée avec un mélange d'amorces nucléotidiques, de préférence choisies parmi les couples d'amorces :
- SEQ ID NO : 8 et SEQ ID NO : 9, lorsque l'amplicon est généré à partir du VHC, quelque soit le génotype viral impliqué. Ce couple d'amorces est générique et permet d'amplifier un amplicon « long » de 401nt à partir de tout génotype de VHC ;
- SEQ ID NO : 10 et SEQ ID NO : 9, un couple permettant la génération d'amplicons « courts » de 191 nt spécifiques du génotype 1a/1b ;
- SEQ ID NO : 29 et SEQ ID NO : 9, un couple permettant la génération d'amplicons « courts » de 108 nt spécifiques du génotype 2 ;
- SEQ ID NO : 8 et SEQ ID NO : 11, un couple permettant la génération d'amplicons « courts » de 143 nt spécifiques du génotype 3a ;
- SEQ ID NO : 8 et SEQ ID NO : 30, un couple permettant la génération d'amplicons « courts » de 175 nt spécifiques du génotype 4a/4d ; et
- SEQ ID NO : 20 et SEQ ID NO : 21, et/ou SEQ ID NO : 22 et SEQ ID NO : 21 lorsque l'amplicon est généré à partir d'un flavivirus.

Selon un mode de réalisation, l'oligonucléotide modifié selon l'invention est utilisé dans la méthode de détection à une densité résultant de la mise en contact d'une solution de greffage allant de 10 nM à 500 nM avec un puits, lorsqu'une plaque 96 puits est utilisée comme substrat. Avantageusement, la solution de greffage de l'oligonucléotide modifié selon l'invention est de 100 nM, lorsqu'une plaque 96 puits est utilisée comme substrat. Lorsque l'oligonucléotide modifié selon l'invention comporte 2 fonctions thiol, la solution de greffage utilisée possède une concentration en oligonucléotide modifié d'au moins 10nM (lorsqu'une plaque 96 puits est utilisée comme substrat) et la concentration de l'amplicon utilisé dans la méthode de détection est d'au moins 100pM. Lorsque l'oligonucléotide modifié selon l'invention comporte 4 fonctions thiol, la solution de greffage utilisée possède une concentration en oligonucléotide modifié d'au moins 10nM par puits et la concentration de l'amplicon utilisé dans la méthode de détection est d'au moins 10pM par puits, lorsqu'une plaque 96 puits est utilisée comme substrat.

La méthode de détection selon l'invention présente un avantage particulier pour le génotypage du VHC, en ce qu'elle permet notamment de distinguer les différents génotypes et sous-types connus du VHC, en mettant en oeuvre un test de détection moléculaire simple.

Les infections liées au virus de l'hépatite C représentent en effet un problème de santé extrêmement important dans la mesure où les individus infectés présentent le risque de développer des maladies du foie, des cirrhoses, et des carcinomes hépatiques primaires, et qu'ils constituent également un réservoir infectieux. Les études épidémiologiques prévoient un triplement du nombre de morts annuelles résultant du VHC au cours des 10 prochaines années si de nouveaux systèmes diagnostiques et thérapeutiques ne sont pas développés.

Caractérisé par une forte variabilité génétique, le VHC est classifié en 6 génotypes majeurs, comprenant plus de 80 sous-types. La détermination précise du génotype et/ou du sous-type infectieux ont des conséquences cruciales pour les stratégies thérapeutiques, et permettent de prédire l'efficacité de la réponse antivirale et de définir la durée de la thérapie, ainsi que le type et le dosage du traitement. L'identification de la classification précise des VHC trouvés chez les individus infectés est également importante pour le suivi épidémiologique, dans le but de surveiller la distribution des souches virales et d'identifier les facteurs de transmission.

De nombreux tests de détection et/ou de quantification d'ARN du VHC disponibles commercialement reposent sur les séquences trouvées dans la région 5' non codante (ou 5'NCR, pour « noncoding region ») qui constitue l'une des régions les plus conservées et les mieux caractérisées du VHC. La région 5'NCR est ainsi choisie comme cible dans les différentes méthodes de génotypage connues et distribuées commercialement, telles que le test INNO LiPA HCV II, de Bayer, le kit Trugene HCV 5'NCR de Bayer /Siemens Healthcare (WO2007/076493), basé sur l'analyse de séquence et le test de mobilité de duplex (« duplex mobility test »), ou un système de détection d'hybridation comprenant une pluralité de typage VHC dessinées dans la région 5'NCR ou dans la région NS5 développé par Roche Molecular Systems (US 2007/014160).

Toutefois, la présence de mutations dans la région 5'NCR conduit à une mauvaise classification des génotypes VHC dans 5 à 8% des cas, entrainant de graves conséquences sur les résultats du traitement. La région 5'NCR ne semble pas non plus présenter un degré de fiabilité suffisant pour identifier les génotypes 2 et 4 du virus.

La méthode de référence pour effectuer le génotypage du VHC réside en outre dans le séquençage de régions spécifiques du VHC, et notamment de la région NS5b du virus, codant pour l'ARN polymérase ARN-dépendante. Toutefois, le séquençage se révèle coûteux et long, et requiert un équipement spécialisé et des opérateurs qualifiés. Il ne se trouve donc pas approprié pour mener des études cliniques à grande échelle.

Il existe donc un besoin pour des méthodes de détection et/ou de génotypage améliorées du VHC permettant de distinguer les différents génotypes et sous-types connus du VHC, en mettant en oeuvre un test moléculaire simple, tel que celui de l'invention.

La présente invention décrit un oligonucléotide possédant une séquence nucléotidique choisie parmi les séquences SEQ ID NO : 1, SEQ ID NO : 4, SEQ ID NO : 27, SEQ ID NO : 28, SEQ ID NO : 16, SEQ ID NO : 17, SEQ ID NO : 18, SEQ ID NO : 19, SEQ ID NO: 35, SEQ ID NO: 36, SEQ ID NO: 40 et SEQ ID NO: 41. Avantageusement, l'oligonucléotide de séquence SEQ ID NO : 1 correspond à une séquence nucléotidique retrouvée spécifiquement chez les VHC de type 1a/1b et permet de détecter spécifiquement ces derniers dans le cadre d'une méthode de détection telle que décrite ci-dessus. Avantageusement, l'oligonucléotide de séquence SEQ ID NO : 27 correspond à une séquence nucléotidique retrouvée spécifiquement chez les VHC de type 2 et permet de détecter spécifiquement ces derniers dans le cadre d'une méthode de détection telle que décrite ci-dessus. Avantageusement, l'oligonucléotide de séquence SEQ ID NO: 35 correspond à une séquence nucléotidique retrouvée spécifiquement chez les VHC de type 2a/2c et permet de détecter spécifiquement ces derniers dans le cadre d'une méthode de détection telle que décrite ci-dessus. Avantageusement, l'oligonucléotide de séquence SEQ ID NO : 36 correspond à une séquence nucléotidique retrouvée spécifiquement chez les VHC de type 2b et permet de détecter spécifiquement ces derniers dans le cadre d'une méthode de détection telle que décrite ci-dessus. Avantageusement, l'oligonucléotide de séquence SEQ ID NO : 4 correspond à une séquence nucléotidique retrouvée spécifiquement chez les VHC de type 3a et permet de détecter spécifiquement ces derniers dans le cadre d'une méthode de détection telle que décrite ci-dessus. Avantageusement, l'oligonucléotide de séquence SEQ ID NO : 28 correspond à une séquence nucléotidique retrouvée spécifiquement chez les VHC de type 4a/4d et permet de détecter spécifiquement ces derniers dans le cadre d'une méthode de détection telle que décrite ci-dessus. Avantageusement, l'oligonucléotide de séquence SEQ ID NO : 16, SEQ ID NO : 17 ou SEQ ID NO : 40 correspond à une séquence nucléotidique retrouvée spécifiquement chez les flavivirus et permet de détecter spécifiquement ces derniers dans le cadre d'une méthode de détection telle que décrite ci-dessus. Avantageusement, l'oligonucléotide de séquence SEQ ID NO: 18 correspond à une séquence nucléotidique retrouvée spécifiquement chez les virus de la Dengue et permet de détecter spécifiquement ces derniers dans le cadre d'une méthode de détection telle que décrite ci-dessus. Avantageusement, l'oligonucléotide de séquence SEQ ID NO : 41 correspond à une séquence nucléotidique retrouvée spécifiquement chez les virus de la Dengue de sérotype 4, et permet de détecter spécifiquement ces derniers dans le cadre d'une méthode de détection telle que décrite ci-dessus. Avantageusement, l'oligonucléotide de séquence SEQ ID NO : 19 correspond à une séquence nucléotidique retrouvée spécifiquement chez les West Nile virus et permet de détecter spécifiquement ces derniers dans le cadre d'une méthode de détection telle que décrite ci-dessus.

### Kits de détection

Un autre objet de l'invention consiste en un kit de détection et/ou de diagnostic comportant au moins un support comportant au moins une zone de réception, sur laquelle est placé au moins un oligonucléotide modifié selon l'invention.

Ainsi, le kit de détection peut être utilisé pour le criblage de molécules biologiquement actives ou pour des tests de diagnostic ou de séquençage. On peut prévoir que le kit de diagnostic comporte plusieurs zones de réception distinctes, sur lesquelles un support solide greffé par des oligonucléotides identiques ou distincts est déposé. Par exemple, des supports solides greffés par des oligonucléotides modifiés identiques ou distincts peuvent être placés sur des zones de réception distinctes d'un substrat recouvert d'un film d'or ou d'un substrat greffé par des fonctions comportant au moins une double liaison carbone-carbone ou une triple liaison carbone-carbone ou des fonctions halogénoacétamide, de préférence des fonctions maléimide ou acrylamide de façon à former un support de test et/ou de diagnostic.

Le support peut notamment être une électrode d'or. Le kit de test peut avantageusement comprendre une cellule électrochimique comportant une électrode de travail, une contre-électrode et une électrode de référence. L'électrode de travail peut être en or, la contre-électrode en platine et l'électrode de référence en argent. L'étude de l'interaction d'un oligonucléotide modifié avec une molécule à tester peut comporter une étape de voltampérométrie cyclique.

Le greffage avec une surface comportant au moins une double liaison carbone-carbone (fonction alcényle) ou une triple liaison carbone-carbone (fonction alcynyle) ou des fonctions halogénoacétamide, de préférence des fonctions maléimide ou acrylamide peut par exemple être utilisé pour des applications dans le domaine du diagnostic en format microplaque et/ou pour la mise en oeuvre de tests de type ELOSA (« Enzyme-Linked OligoSorbent Assay » en anglais ou « dosage d'oligoadsorption par enzyme liée » en français). Au cours de ce type de test, la surface des puits est greffée par des fonctions alcényle, alcynyle ou halogénoacétamide, de préférence des fonctions maléimide ou acrylamide 1. Puis, la surface est mise en contact avec des oligonucléotides modifiés selon l'invention. Ainsi, une ou plusieurs liaisons thioether se forment grâce à la présence d'une ou de plusieurs fonctions thiol sur les oligonucléotides selon l'invention. Puis, le test consiste à mettre en contact un échantillon à tester comprenant une séquence nucléotidique cible avec les puits ainsi fonctionnalisés, pour notamment mesurer l'hybridation des oligonucléotides. La mesure peut par exemple se faire par fluorescence grâce à un marquage des chaînes d' oligonucléotides.

L'invention concerne également l'utilisation d'un oligonucléotide modifié tel que décrit ci-dessus et comportant au moins deux fonctions thiol pour le greffage sur surface comportant au moins une double liaison carbone-carbone ou une triple liaison carbone-carbone ou des fonctions halogénoacétamide, de préférence des fonctions maléimide ou acrylamide. Selon un mode de réalisation, le composé peut comporter trois ou quatre fonctions thiol. L'oligonucléotide modifié immobilisé sur la surface comportant au moins une double liaison carbone-carbone ou une triple liaison carbone-carbone ou des fonctions halogénoacétamide, de préférence des fonctions maléimide ou acrylamide, par l'intermédiaire d'au moins deux fonctions thiol présente une très bonne stabilité pour une utilisation dans un test diagnostic, tel qu'un test de type ELOSA, ainsi qu'une meilleure disponibilité vis-à-vis des cibles. Lorsque le nombre de fonctions thiol dans le composé augmente, le nombre d'hybridations sur l'ensemble du support greffé lors du test de détection augmente. Ainsi, un oligonucléotide modifié présentant quatre fonctions thiol permet d'obtenir une hybridation de la cible plus efficace.

### EXEMPLES

Dans les exemples, on entend par « sonde », une chaîne d'oligonucléotides comprenant au moins un composé thiol selon l'invention destinée à être immobilisée sur une surface.

On entend par « cible », une chaîne d'oligonucléotides destinée à s'hybrider avec la sonde, par exemple, lors d'un test de diagnostic.

### Exemple 1 : Synthèse d'un oligomère

### Synthèse du composé 1-O-(4,4'-diméthoxytrityl)-2-(6-S-acétylthiohexyloxyméthyl)-2-méthylpropane-1,3-diol 4

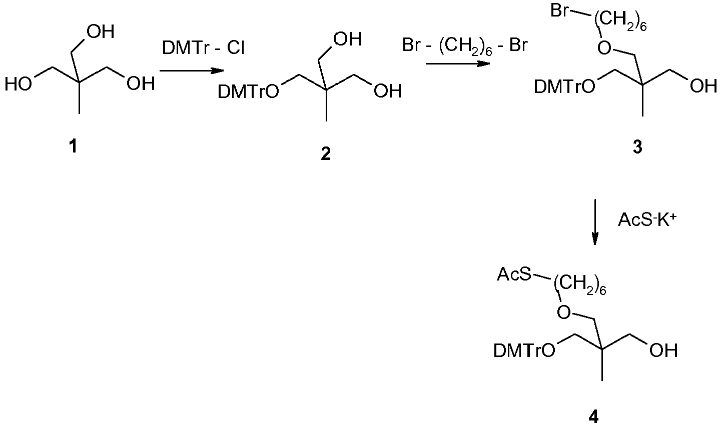

Le composé **3** est obtenu à partir du composé **1** en suivant le protocole décrit dans Pourceau, G., Meyer, A., Vasseur, J. J., and Morvan, F., Journal of Organic Chemistry 74, 2009, 1218-1222.

A une solution de 1-*O*-(4,4'-diméthoxytrityl)-2-(6-bromohexyloxyméthyl)-2-méthyl-1,3-propanediol **3,** (556 mg, 0,95 mmol) et de thioacétate de potassium (162 mg, 1,42 mmol) dans du toluène anhydre (10 mL) on ajoute de l'éther couronne 18-6 (70 mg, 0,26 mmol). Le mélange est agité magnétiquement 2 heures à 50 °C. Après dilution avec du dichlorométhane (150 mL) le mélange est filtré et la phase organique lavée avec de l'eau (2 × 50 mL) puis séchée sur Na₂SO₄. Après évaporation, le brut de réaction est purifié par chromatographie sur silice (0 à 30% d'acétate d'éthyle dans le cyclohexane) pour conduire au produit désiré sous forme d'une huile incolore (413 mg, 75%).

### Synthèse du 1-O-(4,4'-diméthoxytrityl)-2-(6-S-acétylthiohexyloxyméthyl)-2-méthyl-3-O-(2-cyanoéthyl-N,N'-diisopropylphosphoramidite)-propane-1,3-diol 5

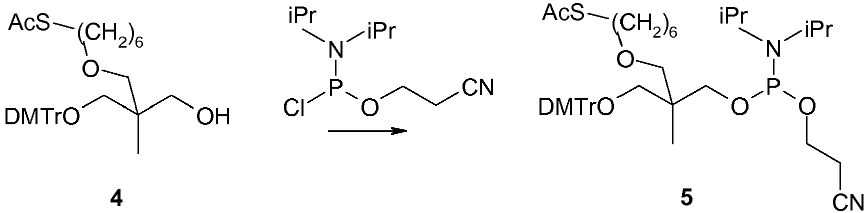

A une solution de 1-*O*-(4,4'-diméthoxytrityl)-2-(6-S-acétylthiohexyloxyméthyl)-2-méthylpropane-1,3-diol **4** (413 mg, 0,71 mmol) et de diisopropyléthylamine (186 µL, 1,06 mmol) dans le dichlorométhane anhydre (10 mL) est ajouté du 2-cyanoéthyl-N,N'-diisopropylchlorophosphoramidite (190 µL, 0,85 mmol). Le mélange est agité magnétiquement une heure à température ambiante. L'excès de réactif est neutralisé par addition de 500 µL d'eau puis le mélange est dilué avec du dichlorométhane (150 mL). La phase organique est lavée avec une solution aqueuse saturée en NaHCO₃ (100 mL), puis séchée sur Na₂SO₄. Après évaporation, le brut est purifié par chromatographie sur silice (0 à 30% d'acétate d'éthyle dans du cyclohexane contenant 4% de triéthylamine) conduisant au composé **5** désiré sous forme d'une huile incolore (400 mg, 72%).

### Synthèse du support solide thiol 6

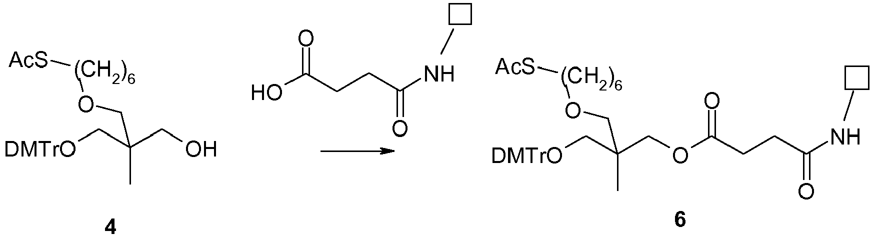

Dans un tube rodé, le 1-O-(4,4'-diméthoxytrityl)-2-(6-S-acétylthiohexyloxyméthyl)-2-méthyl-1,3-propanediol **4** (178 mg, 0,3 mmol) et la dimethylaminopyridine (DMAP 36 mg, 0,3 mmol) sont coévaporés avec 3 mL de pyridine anhydre. Puis on ajoute la succinyl-longue chaine alkylamine -CPG (Controled Pore Glass) (1 g), la pyridine anhydre (5 mL), la triéthylamine anhydre (160mL, 1,2 mmol) et l'éthyldiméthylaminopropyl carbodiimide (EDC, 280 mg, 2,0 mmol). Puis on rince avec 1 mL de pyridine anhydre. Le mélange est agité une nuit.

Le mélange est filtré et lavé avec CH₂Cl₂ (10 mL) puis séché au dessiccateur. Le composé thiol sur support solide est traité par une solution d'anhydride acétique, N-méthylimidazole, 2,6-lutidine dans le THF pendant 3h sous agitation. Le mélange est filtré et lavé avec CH₂Cl₂ (10 mL) puis séché au dessiccateur pour donner le support solide thiol **6** (940 mg) avec une fonctionnalisation de 29 µmol/g.

### Exemple 2 : Préparation d'oligonucléotides modifiés

Trois oligonucléotides comprenant un groupement ferrocène en position 5' et un nombre croissant de composés thiol de type (Ia) selon l'invention (1, 2 et 4 composés thiol) ont été synthétisés sur un synthétiseur d'ADN. Le groupement ferrocène a été utilisé afin de visualiser l'immobilisation de l'oligonucléotide sur la surface d'or par voltampérométrie cyclique. Un, deux ou quatre groupements thiol ont été introduits sur un support solide de type propanediol et la séquence ADN SEQ ID NO : 7 a été greffée. Enfin, une phosphoramidite alpha-thymidine portant un groupement ferrocène a été introduite en position 5' de l'oligonucléotide modifié. La déprotection qui suit s'effectue en deux étapes. D'abord, le milieu est traité avec 10% de pipéridine dans l'acétonitrile pendant 10 minutes afin d'éliminer le groupement cyanoéthyle par une bêta-élimination et l'acrylonitrile résultant est éliminé du milieu par un lavage à l'acétonitrile. Puis, un traitement à l'hydroxyde d'ammonium concentré permet d'éliminer les groupements protecteurs acyles sur les nucléobases et sur les fonctions thiol et permet d'hydrolyser le bras de liaison succinyle (support solide). Ce protocole permet d'éviter l'addition de Michael entre les fonctions thiol déprotégées et l'acrilonitrile. Après évaporation, l'oligonucléotide modifié non supporté est purifié par chromatographie HPLC en phase inverse sur colonne C18.

### Tétrathiol (oligonucléotide modifié par 4 composés thiol)

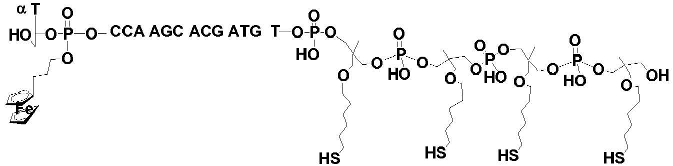

### Dithiol (oligonucléotide modifié par 2 composés thiol)

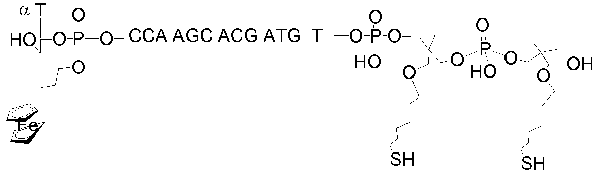

### Monothiol (oligonucléotide modifié par 1 composé thiol)

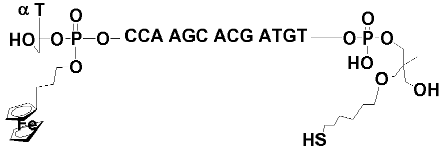

### Greffage et étude de stabilité des oligonucléotides modifiés (tétrathiol, dithiol et monothiol)

Pour cette étude, un potentiostat multivoies VMP3 Biologic (Biologic Science Instruments, Pont de Claix) a été utilisé. Les résultats ont été enregistrés à l'aide du logiciel EC-Lab de Biologic Science Instruments.

La cellule électrochimique est composée d'une électrode d'or de 0,28cm² de surface, d'une contre-électrode en platine et d'une électrode de référence Ag/AgCl.

### Etape 1 : Réduction des groupements thiol

4nmol d'ODN-thiol (oligonucléotides modifiés avec un ou plusieurs composés thiol) sont réduits dans une solution de Tris(2-carboxyethyl)phosphine hydrochlorure (TCEP,HCL, Sigma-Aldrich) (160mM) soit une concentration dans la solution de 20mM en TCEP,HCl, à 20°C, pendant 2h sous argon.

L'ODN est purifié par deux dilutions/centrifugations successives avec une solution de TCEP,HCl 20mM dégazée sur filtres amicon YM3000 (Millipore) 15min à 14000rcf (rcf = Relative Centrifugal Force). Après dilution dans 450µL de tampon phosphate 100mM dégazé, le milieu est de nouveau centrifugé sur filtres amicon YM3000, 30 min, 14000rcf.

Une solution de greffage contenant 4nmol d'ODN, 90mM en phosphate de sodium, 2mM en TCEP,HCl est obtenue.

### Etape 2 : Activation de l'électrode d'or

L'électrode de travail d'or est nettoyée par un premier lavage dans l'acétone pendant 10 minutes aux ultra-sons. Une fois séchée la surface est plongée dans une solution « piranha » (H₂SO₄ 0,7mL, H₂O₂ 0,3mL) pendant 1 minute afin d'éliminer tout résidu organique de la surface.

Enfin l'activation basique de l'électrode consiste à nettoyer l'or en surface par production d'hydrogène à l'électrode par hydrolyse de l'eau dans la soude 0,5M dans les potentiels négatifs (-1,4V vs Ag/AgCl) pendant plusieurs cycles.

### Etape 3 : Greffage de la sonde

Après rinçage, la solution de greffage contenant l'oligonucléotide thiol est mise au contact de l'électrode d'or activée, pendant trois jours sous atmosphère inerte.

Après rinçage, la cellule électrochimique est remplie avec l'électrolyte d'analyse (phosphate de sodium dibasique 10mM, phosphate de potassium monobasique 10mM, perchlorate de sodium 250mM, pH 6,5).

La surface est passivée par une solution de mercaptopropanol 1mM pendant 30 minutes, après rinçage la cellule est mise dans l'électrolyte d'analyse pendant 2h afin de stabiliser la couche greffée.

### Etape 4 : Analyse de la stabilité des composés greffés en surface

Les analyses sont réalisées par voltampérométrie cyclique (CV) à 50mV/s ; entre -0,1V et 0,45V. Les études de stabilité de la couche greffée sont réalisées après une stabilisation du signal électrochimique pendant 2h en cyclant toutes les 30 minutes.

La cellule est remplie d'eau distillée dégazée à 60°C ou 80°C pendant 1 ou 5 minutes, après rinçage la cellule est remplie de 1,5mL d'électrolyte d'analyse phosphate (20mM) perchlorate (250mM). Après 30 minutes de stabilisation, une CV est réalisée.

L'opération est renouvelée autant que nécessaire.

### Résultats

Une comparaison du taux de greffage des 3 oligonucléotides modifiés par des composés thiol (tétrathiol, dithiol et monothiol) a été réalisée. Le taux de greffage a été déterminé par intégration du pic d'oxydation du ferrocène. En effet, la charge d'électron transférée est directement liée au nombre de ferrocènes présents en surface de l'électrode, et donc au nombre de sondes greffées sur l'or.

Les résultats ci-dessous correspondent à la moyenne des taux de greffage de 3 greffages différents pour chaque sonde.

| | molécules/cm² |
|---|---|
| **monothiol** | 5,21E+12 |
| **dithiol** | 5,81E+12 |
| **tétrathiol** | 1,40E+12 |

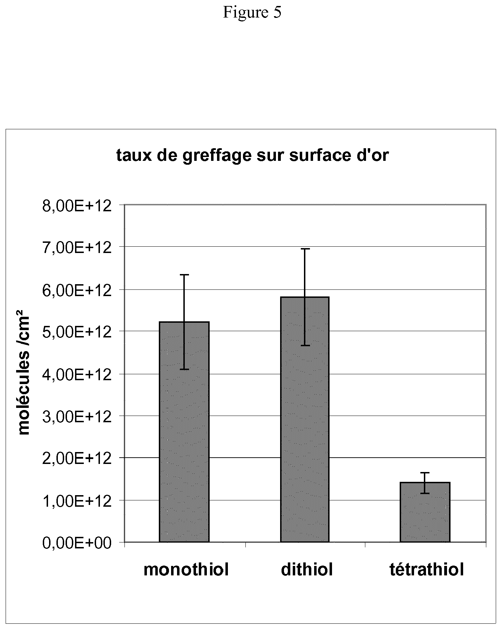

Un histogramme des résultats est représenté sur la figure 5.

Les taux de greffages pour le monothiol et dithiol sont assez comparables, nous pouvons observer un greffage moins important du tétrathiol probablement dû à l'encombrement plus important en accord avec le nombre de bras thiol. Malgré cette différence, le taux de greffage du tétrathiol reste important et il est très reproductible.

Une étude de stabilité des 3 oligonucléotides modifiés par des composés thiol vis-à-vis de la température a été menée dans de l'eau distillée dégazée. L'évolution de la réponse électrochimique a été suivie par voltampérométrie cyclique. Le pourcentage de diminution d'intensité d'oxydation du ferrocène est calculé par rapport au signal obtenu après stabilisation.

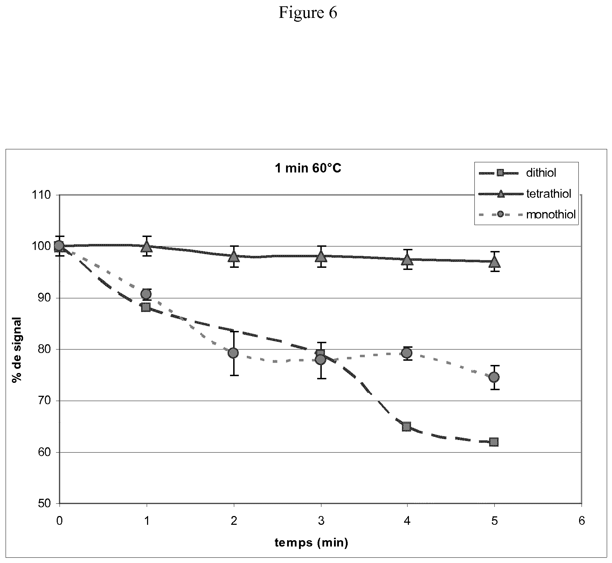

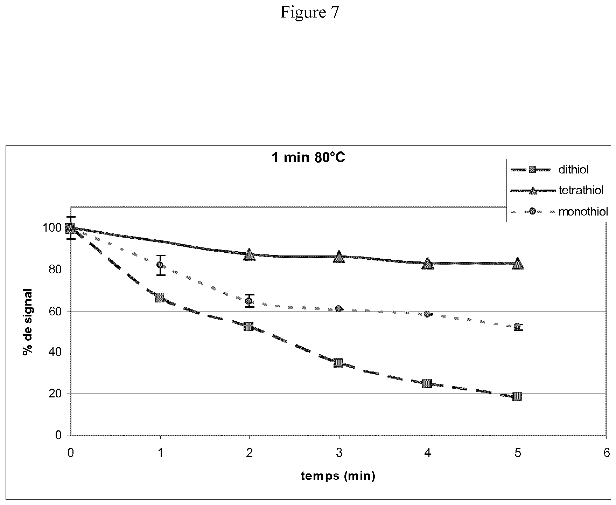

Ces diminutions en fonction du temps sont reportées sur les figures 6 et 7.

La molécule tétrathiol est très stable vis-à-vis de traitements successifs dans de l'eau à 60°C (figure 6). En effet, après 5 fois une minute de ce traitement, il reste 97% du signal de départ, contrairement au monothiol (70% de signal de départ) et dithiol (62%de signal de départ).

A 80°C, la perte de signal est plus importante (figure 7). Après cinq traitements successifs d'une minute, 83% du signal de départ est encore quantifiable pour le tétrathiol. Ainsi, la stabilité du tétrathiol est bien supérieure à celle du monothiol (45% de signal résiduel) et celle du dithiol (18% de signal résiduel).

Cette étude montre le gain notable de stabilité du greffage thiol sur or par l'emploi d'un oligonucléotide modifié par 4 composés thiol (tétrathiol), en comparaison du greffage d'un monothiol ou d'un dithiol. Le manque de stabilité de ce dernier dithiol peut s'expliquer par une possible compétition entre le greffage sur or et la fermeture du cycle pour reformer le pont disulfure intramoléculaire. Il apparaît donc préférable de maintenir un nombre de quatre thiols sur le bras de greffage pour assurer une bonne stabilité vis-à-vis de la température.

### Exemple 3 : Application à la détection du Virus de l'Hépatite C

### Echantillons cibles à tester

### 1) Cibles naturelles : amplicons « VHC (+) »

Des échantillons de plasmas issus de donneurs de sang, dépistés positifs pour la présence du VHC à l'échelle nationale, sont analysés par séquençage. Le génotype et le sous-type viral du VHC infectant chaque donneur sont déterminés.

Des amplicons « VHC (+) » de 401 pb sont produits à partir de la région virale NS5b du VHC, par RT-PCR à partir de chacun des échantillons de plasmas décrits précédemment. L'ARN est extrait à partir de 200µL de plasma humain en utilisant le kit High Pure Viral Nucleic Acid (Roche) selon les recommandations du fabriquant. L'ARN est élué dans 50µL d'eau stérile (DNase/RNase free). Afin d'effectuer l'étape de rétro-transcription (RT), 11 µL d'ARN sont dénaturés à 72°C pendant 10 minutes et rétro-transcrits en présence de 4µL de 5X First Strand Buffer (Invitrogen), 2µL de 10X Hexanucleotide Mix (Roche), 2µL de 10mM dNTP mix (Invitrogen) et 200U de SuperScript® II Reverse Transcriptase (Invitrogen). Les conditions de rétro-transcription sont les suivantes : 10 min à 23°C, 45 min à 37°C, et 10 min à 95°C.

Cinq microlitres d'ADNc sont ensuite amplifiés par PCR (réaction en chaîne par polymérase) en utilisant les amorces :
- « VHCsens biotinylée » (de séquence : [Btn]- TGG GGA TCC CGT ATG ATA CCC GCT GCT TTG A (soit [Btn]-SEQ ID NO : 8)) et
- « VHCanti-sens » (de séquence GGC GGA ATT CCT GGT CAT AGC CTC CGT GAA (SEQ ID NO : 9)) (voir Tamalet C. et al. 2003, Journal of Medical Virology 71:391-398).

La réaction d'amplification est effectuée dans 50µL de mélange réactionnel comprenant : 1X PCR Buffer sans MgCl₂ (Invitrogen), 0,2µM de chaque amorce, 1,5mM MgCl₂ (Invitrogen), 0,2mM dNTP mix (Invitrogen) et 1,3U Taq Polymerase (Invitrogen). Les conditions de PCR sont les suivantes : 5 min à 95°C, 40 cycles (dénaturation: 40 sec, 95°C; hybridation: 40 sec, 56°C; élongation: 50 sec, 72°C), et une extension finale de 10 min à 72°C.

Les amplicons VHC obtenus sont analysés par électrophorèse en gel d'agarose, aliquotés et conservés à -20°C avant utilisation.

Ces amplicons, dont les génotypes viraux d'origine sont connus, sont conservés sous la forme d'une thèque.

Un exemple d'amplicon long de 401pb obtenu à partir d'une souche virale 1a/1b, de référence # PTR6719 a pour séquence :

Un exemple d'amplicon long de 401pb obtenu à partir d'une souche virale 2, de référence # PTR7761 a pour séquence :

Un exemple d'amplicon long de 401pb obtenu à partir d'une souche virale 3a, de référence # PTR9058 a pour séquence :

Un exemple d'amplicon long de 401pb obtenu à partir d'une souche virale 4a/4d, de référence # PTR4162 a pour séquence :

En complément de ces amplicons longs, d'autres amorces ont été dessinées afin de générer des amplicons plus courts à partir de chacun des échantillons de plasmas décrits précédemment. Le même protocole global est suivi pour générer les amplicons « courts », et seules les amorces utilisées sont changées. Les amorces utilisées sont choisies selon le génotype et/ou le sous-type du VHC ayant infecté les patients.

Des amplicons spécifiques des VHC de sous-type 1a/1b, d'une taille de 191 nucléotides sont amplifiés avec les amorces suivantes :
- Amorce sens biotinylée « 1a/1b sens», de séquence 5'-[Btn]-TGA-CRA-CYA-GCT-GYG-GTA-AYA-CCC-T- 3' (soit [Btn]-SEQ ID NO : 10), et
- Amorce antisens « VHCantisens » de SEQ ID NO : 9 (voir plus haut).

Un exemple d'amplicon court de 191 nucléotides obtenu à partir d'une souche virale 1a/1b, de référence # PTR6719 a pour séquence :

Des amplicons spécifiques des VHC de sous-type 2, d'une taille de 108 nucléotides sont amplifiés avec les amorces suivantes :
- Amorce sens biotinylée « VHC 2 sens biotinylée», de séquence 5'-[Btn]-ATG-YTG-GTR-TGC-GGC-GAC-GAC- 3' (soit [Btn]-SEQ ID NO : 29), et
- Amorce antisens « VHCantisens » de SEQ ID NO : 9 (voir plus haut).

Un exemple d'amplicon court de 108 nucléotides obtenu à partir d'une souche virale 2, de référence # PTR7761 a pour séquence :

Des amplicons spécifiques des VHC de sous-type 3a, d'une taille de 143 nucléotides sont amplifiés avec les amorces suivantes :
- Amorce sens biotinylée « VHC sens », de SEQ ID NO : 8 (voir plus haut), et
- Amorce antisens «3a rev » de séquence: 5'-GCA-GTA-AAG-CCG-YTC-CGT-GAG-3' (SEQ ID NO : 11).

Un exemple d'amplicon court de 143 nucléotides obtenu à partir d'une souche virale 3 a, de référence # PTR9058 a pour séquence :

Des amplicons spécifiques des VHC de sous-type 4a/4d, d'une taille de 175 nucléotides sont amplifiés avec les amorces suivantes :
- Amorce sens biotinylée « VHC sens », de SEQ ID NO : 8 (voir plus haut), et
- Amorce antisens «VHC anti-sens 4 rev » de séquence : 5'-AGG-TCT-CCC-YTG-CTG-TTG-TRC-AT-3' (SEQ ID NO : 30).

Un exemple d'amplicon court de 175 nucléotides obtenu à partir d'une souche virale 4a/4d, de référence # PTR4162 a pour séquence :

Comme précédemment, les amplicons VHC « courts » obtenus de 191, 108, 175 et 143 nucléotides sont analysés par électrophorèse en gel d'agarose, aliquotés et conservés à -20°C avant utilisation.

Ces amplicons, dont les génotypes viraux d'origine sont connus, sont conservés sous la forme d'une thèque.

### 2) Cibles synthétiques : oligonucléotides biotinylés 15-mères ou 105-mères

Des oligonucléotides synthétiques 15-mères ou 105-mères biotinylés à l'extrémité 5' ont été synthétisés.

Les oligonucléotides synthétiques 15-mères sont strictement complémentaires aux sondes VHC choisies (voir ci-après), et possèdent les séquences suivantes :
- les oligonucléotides synthétiques 15-mères spécifiques du sous-type 1a/1b ont la séquence : 5' [Btn]- GCT CCR GGA ACT GCA C -3' (soit [Btn]-SEQ ID NO : 2) ;
- les oligonucléotides synthétiques 15-mères spécifiques du sous-type 3a ont la séquence : [Btn]-CTT GAA CCG GAG GCC-3' (soit [Btn]-SEQ ID NO : 5) et
- les oligonucléotides synthétiques 15-mères spécifiques du sous-type 4a/4d ont la séquence : 5' [Btn]-CTA YGT GGG CGG YCC -3' (soit [Btn]-SEQ ID NO : 39).

Des oligonucléotides plus longs comprenant la séquence complémentaire des sondes VHC présentées ci-après, encadrées de part et d'autre par des séquences de 45 nucléotides, soit 105 nucléotides au total, et biotinylés à l'extrémité 5' ont également été synthétisés pour le développement des tests d'hybridation sondes/cibles.

Les oligonucléotides synthétiques 105-mères utilisés possèdent les séquences suivantes :
- les oligonucléotides synthétiques 105-mères spécifiques du sous-type 1a/1b ont la séquence : 5' [Btn]- CCT CAC TTG CTA CAT CAA GGC CCA GGC AGC CTG TCG AGC CGC AGG - GCT CCR GGA ACT GCA C - CAT GCT CGT GTG TGG CGA CGA CTT AGT CGT TAT CTG TGA AAG TGC-3' (soit [Btn]-SEQ ID NO : 3) ; et
- les oligonucléotides synthétiques 105-mères spécifiques du sous-type 3a ont la séquence : 5' [Btn]- GAA CAG GAC ATC AGG GTG GAA GAG GAG ATA TAC CAA TGC TGT AAC - CTT GAA CCG GAG GCC - AGG AAA GTG ATC TCC TCC CTC ACG GAG CGG CTT TAC TGC GGG GGC -3' (soit [Btn]-SEQ ID NO : 6).

### Design des sondes oligonucléotidiques pour la reconnaissance de séquences VHC.

La région NS5b codant l'ARN polymérase du VHC a été ciblée pour le design des sondes oligonucléotidiques de l'invention.

Les séquences de génomes VHC amplifiés dans la région NS5b (banque représentative de 800 échantillons) ont été déterminées et analysées à l'aide des logiciels d'alignement clustalW2 et de phylogénie Mega5 afin d'identifier les zones les plus conservées pour chaque génotype et/ou sous-type du VHC. Des régions distinctes permettant la reconnaissance générique des séquences virales d'un génotype et éventuellement d'un sous-type donné ont été sélectionnées. Une région très conservée permettant la détection spécifique de tous les VHC de génotype viral 1a/1b a ainsi été identifiée et sélectionnée. De la même manière, une région permettant la détection spécifique des VHC de génotype viral 2, 2a/2c, 2b, 3a et 4a/4d a été identifiée et sélectionnée. Des oligonucléotidiques 15-mères complémentaires de chacune des régions choisies ont été dessinés et des oligonucléotides multi-thiols ont ensuite été synthétisés sur la base des séquences choisies pour la reconnaissance spécifique des sous-types 1a/1b, 2, 2a/2c, 2b, 3a et 4a/4d.

La séquence oligonucléotidique retenue pour générer la sonde 1a/1b est : 5'-GTG-CAG-TCC-YGG-AGC (SEQ ID NO : 1). Cette sonde est spécifique des souches de sous-type 1a et 1b.

La séquence oligonucléotidique retenue pour générer la sonde 2 est : 5'-TGG-CTY-TCT-GAG-ATG (SEQ ID NO : 27). Cette sonde est spécifique des souches de sous-type 2.

La séquence oligonucléotidique retenue pour générer la sonde 2a/2c est : 5'-GGA-CTC-CTC-RGT-TCT (SEQ ID NO: 35). Cette sonde est spécifique des souches de sous-type 2a et 2c.

La séquence oligonucléotidique retenue pour générer la sonde 2b est : 5'-TAT-GGA-TTC-TTC-TGT (SEQ ID NO : 36). Cette sonde est spécifique des souches de sous-type 2b.

La séquence oligonucléotidique retenue pour générer la sonde 3a est : 5'-GGC-CTC-CGG-TTC-AAG (SEQ ID NO : 4). Cette sonde est spécifique des souches de sous-type 3 a.

La séquence oligonucléotidique retenue pour générer la sonde 4a/4d est : 5'-GGR-CCG-CCC-ACR-TAG (SEQ ID NO : 28). Cette sonde est spécifique des souches de sous-types 4a et 4d.

### Evaluation des hybridations sondes / cibles par ELOSA (Enzyme-Linked OligoSorbent Assay)

Le greffage de tout type de sondes thiol (oligonucléotides d'anomérie alpha ou béta, sondes linéaires ou tige-boucle (« *snails* ») ...) peut être réalisé selon le protocole optimisé suivant. Les puits de microplaques activées maléimide (Pierce) sont lavés par le tampon WB1 (0,1M Na₂HPO4, 0,15M NaCl, 0,05% Tween 20 (w/v), pH 7,2). La fonctionnalisation des puits est réalisée avec 100nM de sondes multi-thiol (telles que décrites ci-dessus) dans du tampon BB (0,1M Na₂HPO₄, 0,15M NaCl, 10mM EDTA, pH 7,2) pendant 2 heures à température ambiante (TA). Les puits sont ensuite lavés trois fois avec WB1, saturés par une solution de Cystéine-HCl 10 µg.mL⁻¹ dans BB (Pierce) pendant 1 heure à TA, et lavés à nouveau trois fois avec WB1.

Les tests d'hybridation sont réalisés avec des cibles synthétiques courtes 15-mères ou 105-mères ou avec de réels amplicons VHC longs (401 nt) ou plus courts (191 nt ou 143 nt). Les cibles synthétiques et les amplicons sont dilués dans 150 µL de tampon d'hybridation (TH : 0,9M NaCl, 60mM NaH₂PO₄, 6mM EDTA, pH 7,4, Denhardt 5X) avant d'être déposés dans les puits. Une étape supplémentaire de dénaturation de 10 min à 95°C est effectuée sur les amplicons avant transfert dans les micropuits. L'hybridation est réalisée sur la nuit à 37°C. Les puits sont lavés avec du tampon WB2 (0,75M NaCl, 50mM NaH₂PO₄, 5mM EDTA, pH 7,4, SDS 0,1 %) trois fois 2 min à TA et une fois 30 min à 50°C.

L'étape de détection est réalisée après incubation pendant 30 min à TA des puits en présence de 100 µL/puits de Streptavidine-Europium (Perkin Elmer) diluée dans 100 µL de tampon de test (« Assay Buffer », Perkin Elmer). Les puits sont finalement lavés six fois avec du tampon WB3 (WB1X, Perkin Elmer) et 200 µL de tampon de révélation du signal (« Enhancement Buffer », Perkin Elmer) sont ajoutés dans chaque puits pendant 5 min à TA. La fluorescence en temps résolu est mesurée sur un détecteur multi-marquage Victor™ 1420 (« multilabel counter », Perkin Elmer) selon le protocole du fabricant (excitation à 340 nm et émission à 615 nm).

### Résultats 1 : Tests d'hybridation sur sondes linéaires d'anomérie béta possédant 4 fonctions thiol

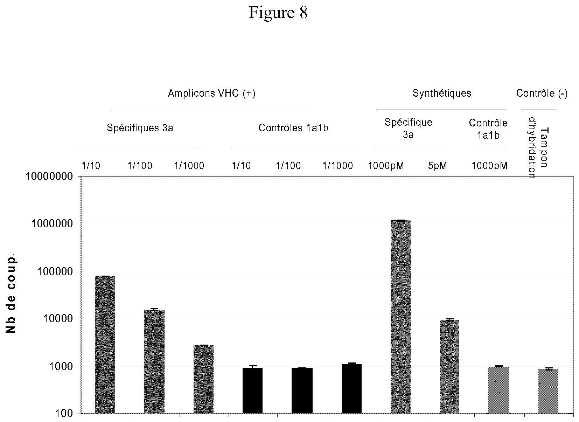

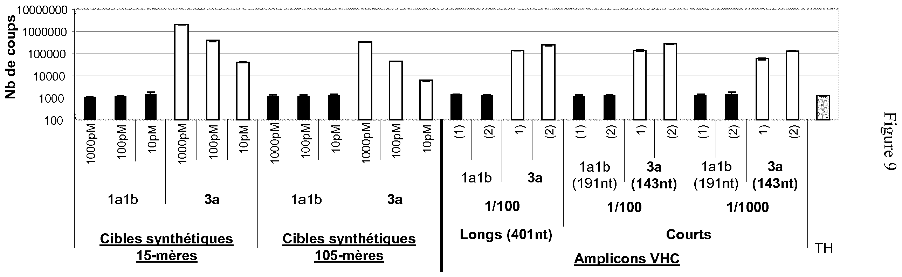

Des sondes 3a linéaires d'anomérie béta possédant 4 fonctions thiol sont greffées, à une densité de 100nM, sur un support couvert de groupements maléimide selon le protocole décrit ci-dessus. Les résultats obtenus avec les sondes 3a sont décrits dans les figures 8, et 9.

Des tests ELOSA sont réalisés en faisant varier la nature et la concentration de la cible utilisée (cibles synthétiques courtes 15-mères et 105-mères, amplicons courts de 143 nt ou 191 nt, amplicons longs de 401 nt), afin de vérifier la spécificité des sondes retenues.

Les résultats présentés dans la figure 8 correspondent à un test ELOSA réalisé avec des amplicons longs spécifiques du génotype VHC 3a, aux dilutions 1/10, 1/100 et 1/1000 et avec des amplicons longs non spécifiques (correspondant au génotype VHC 1a/1b) aux dilutions 1/10, 1/100 et 1/1000. Le contrôle positif d'hybridation est effectué avec une cible synthétique (15-mère) spécifique du génotype 3a à des concentrations de 1000pM et 5pM. Les contrôles négatifs d'hybridation sont effectués avec une cible synthétique (15-mère) non spécifique (correspondant à la séquence cible 1a/1b) à une concentration de 1000pM, ou avec du TH (tampon d'hybridation) seul, ne comprenant pas de cible.

Pour que la sonde 3 a puisse être considérée comme « spécifique » du sous-type 3 a, il s'avère donc nécessaire que les résultats obtenus avec des cibles « 3a » dépassent le bruit de fond obtenu avec les contrôles négatifs, de sorte que l'hybridation sonde/cible puisse être quantifiée.

Les résultats présentés dans la figure 9 correspondent à un test ELOSA réalisé avec les cibles suivantes :
- cible synthétique 15-mère ou 105-mère spécifique 3a, à une concentration de 10pM, 100pM ou de 1000pM,
- cible synthétique 15-mère ou 105-mère non spécifique 1a/1b (contrôle négatif), à une concentration de 10pM, 100pM ou de 1000pM,
- amplicon long spécifique 3a, à la dilution 1/100,
- amplicon long non spécifique 1a/1b, à la dilution 1/100,
- amplicon court spécifique 3a (143 nt), aux dilutions 1/100 et 1/1000,
- amplicon court non spécifique 1a/1b (191 nt), aux dilutions 1/100 et 1/1000,
- TH seul, ne comprenant pas de cible.

Les résultats obtenus dans les figures 8, et 9 montrent que la sonde « 3a » selon la présente invention est non seulement capable de s'hybrider avec des cibles synthétiques spécifiques 3a 15-mères (la cible ayant une concentration de 5pM) ou 105-mères (la cible ayant une concentration de 10pM), mais aussi avec des amplicons longs de 401 nt ou courts de 143 nt obtenus à partir de plasmas génotypés 3a, et donc spécifiques du génotype 3a (à une dilution au 1/1000^{ème}). Ces résultats montrent également que les cibles 1a/1b, non spécifiques de la sonde 3a, ne s'hybrident pas de manière détectable avec cette dernière, indépendamment de leur forme (cible synthétique 15-mère ou 105-mère, amplicon court 191 nt ou long 401 nt), ou de leur concentration (1000pM pour les cibles synthétiques ou dilution au 1/10^{ème} pour les amplicons).

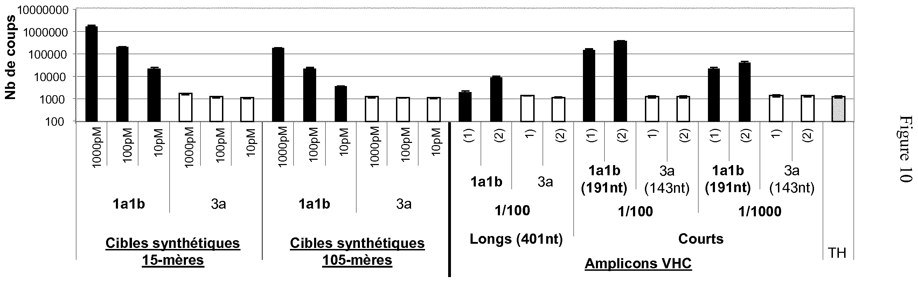

Les résultats présentés dans la figure 10 correspondent à un test ELOSA réalisé avec des sondes 1a/1b linéaires (voir plus haut), d'anomérie béta possédant 4 fonctions thiol, qui sont greffées à une densité de 100nM sur un support couvert de groupements maléimide selon le protocole décrit ci-dessus.

Les résultats de la figure 10 sont obtenus à partir des cibles suivantes :
- cible synthétique 15-mère ou 105-mère spécifique 1a/1b, à une concentration de 10pM, 100pM ou 1000pM,
- cible synthétique 15-mère ou 105-mère non spécifique 3a (contrôle négatif), à une concentration de 10pM, 100pM ou 1000pM,
- amplicon long spécifique du génotype VHC 1a/1b, à la dilution 1/100,
- amplicon long non spécifique 3a, à la dilution 1/100,
- amplicon court (191 nt) spécifique 1a/1b, aux dilutions 1/100 et 1/1000,
- amplicon court (143 nt) non spécifique 3a, aux dilutions 1/100 et 1/1000,
- TH seul, ne comprenant pas de cible.

Les résultats obtenus dans la figure 10 montrent que la sonde 1a/1b selon la présente invention est non seulement capable de s'hybrider avec des cibles synthétiques 15-mères ou 105-mères spécifiques 1a/1b (la cible ayant une concentration de 10pM), mais aussi avec des amplicons courts de 191 nt obtenus à partir de plasmas génotypés 1a/1b, et donc spécifiques du génotype 1a/1b (à une dilution au 1/1000^{ème}), et avec des amplicons longs de 401 nt spécifiques du génotype 1a/1b (à une dilution au 1/100^{ème}). Ces résultats montrent également que les cibles 3a, non spécifiques de la sonde 1a/1b, ne s'hybrident pas de manière détectable avec cette dernière, indépendamment de leur forme (cible synthétique 15-mère ou 105-mère, amplicon court 143 nt ou long 401 nt), ou de leur concentration (1000pM pour les cibles synthétiques ou dilution au 1/10^{ème} pour les amplicons).

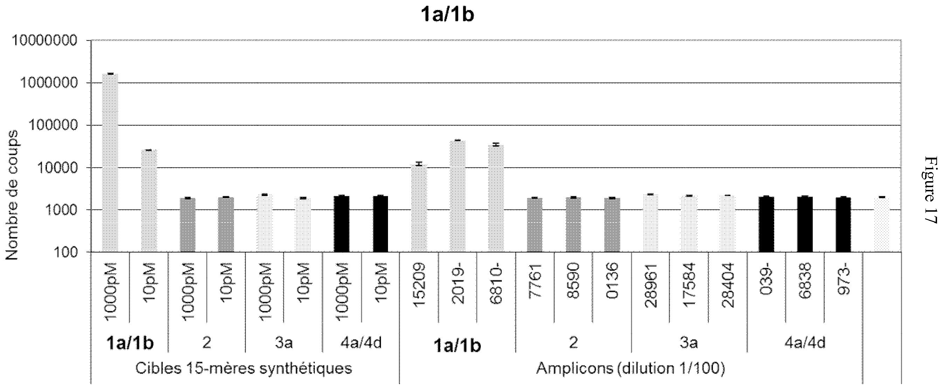

Les résultats présentés dans la figure 17 correspondent à un test ELOSA réalisé avec une sonde 1a/1b linéaire d'anomérie béta possédant 4 fonctions thiol, qui est greffée à une densité de 100nM sur un support couvert de groupements maléimide selon le protocole décrit ci-dessus.

Les résultats de la figure 17 sont obtenus à partir des cibles suivantes :
- cible synthétique 15-mère spécifique 1a/1b à une concentration de 10pM ou 1000pM,
- cible synthétique 15-mère non spécifique 2a/2c, 2b, 3a ou 4a/4d (contrôle négatif), à une concentration de 10pM ou 1000pM,
- amplicon long spécifique du génotype VHC 1a/1b, à la dilution 1/100,
- amplicon long non spécifique 2a/2b/2c, 3a ou 4a/4d à la dilution 1/100,
- TH seul, ne comprenant pas de cible.

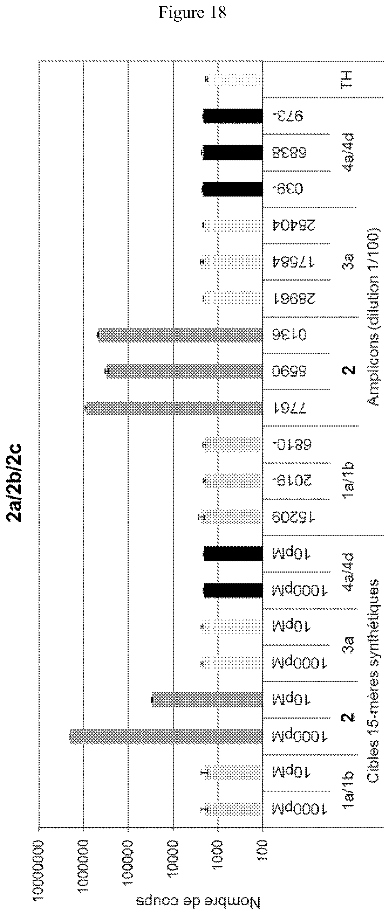

Les résultats présentés dans la figure 18 correspondent à un test ELOSA réalisé avec un mélange de deux sondes (2a/2c de SEQ ID NO : 35 et 2b de SEQ ID NO : 36) linéaires d'anomérie béta possédant 4 fonctions thiol, qui sont greffées à une densité de 50nM chacune sur un support couvert de groupements maléimide selon le protocole décrit ci-dessus.

Les résultats de la figure 18 sont obtenus à partir des cibles suivantes :
- cibles synthétiques 15-mères spécifiques 2a/2c et 2b à une concentration de 10pM ou 1000pM,
- cible synthétique 15-mère non spécifique 1a/1b, 3a ou 4a/4d (contrôle négatif), à une concentration de 10pM ou 1000pM,
- amplicon long spécifique du génotype VHC 2a/2b/2c, à la dilution 1/100,
- amplicon long non spécifique 1a/1b, 3a ou 4a/4d à la dilution 1/100,
- TH seul, ne comprenant pas de cible.

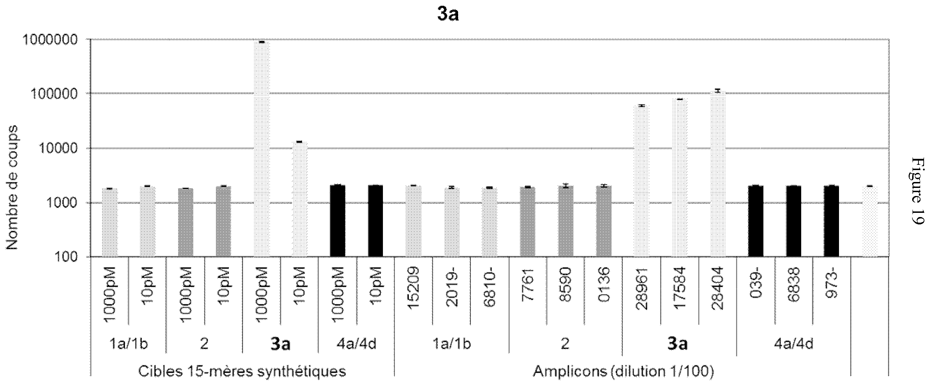

Les résultats présentés dans la figure 19 correspondent à un test ELOSA réalisé avec une sonde 3a linéaire d'anomérie béta possédant 4 fonctions thiol, qui est greffée à une densité de 100nM sur un support couvert de groupements maléimide selon le protocole décrit ci-dessus.

Les résultats de la figure 19 sont obtenus à partir des cibles suivantes :
- cible synthétique 15-mère spécifique 3a à une concentration de 10pM ou 1000pM,
- cible synthétique 15-mère non spécifique 1a/1b, 2a/2b/2c ou 4a/4d (contrôle négatif), à une concentration de 10pM ou 1000pM,
- amplicon long spécifique du génotype VHC 3a, à la dilution 1/100,
- amplicon long non spécifique 1a/1b, 2a/2b/2c ou 4a/4d à la dilution 1/100,
- TH seul, ne comprenant pas de cible.

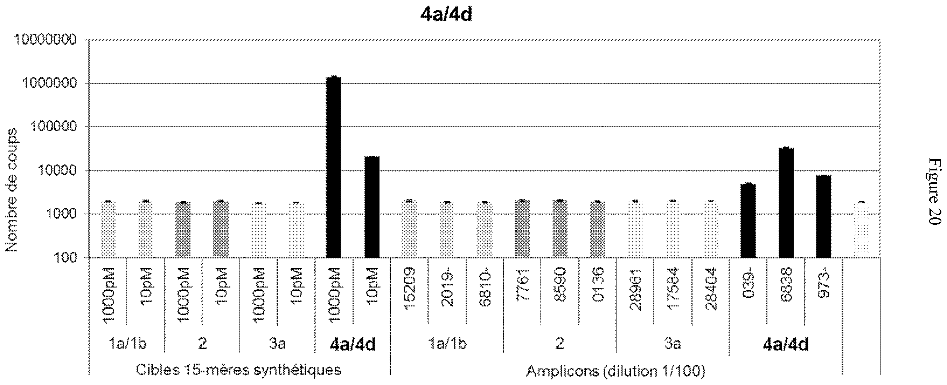

Les résultats présentés dans la figure 20 correspondent à un test ELOSA réalisé avec une sonde 4a/4d linéaire d'anomérie béta possédant 4 fonctions thiol, qui est greffée à une densité de 100nM sur un support couvert de groupements maléimide selon le protocole décrit ci-dessus.

Les résultats de la figure 20 sont obtenus à partir des cibles suivantes :
- cible synthétique 15-mère spécifique 4a/4d à une concentration de 10pM ou 1000pM,
- cible synthétique 15-mère non spécifique 1a/1b, 2a/2b/2c ou 3a (contrôle négatif), à une concentration de 10pM ou 1000pM,
- amplicon long spécifique du génotype VHC 4a/4d, à la dilution 1/100,
- amplicon long non spécifique 1a/1b, 2a/2b/2c ou 3a à la dilution 1/100,
- TH (tampon d'hybridation) seul, ne comprenant pas de cible.

Pour les tests effectués en utilisant des amplicons, les résultats de 3 essais indépendants réalisés à partir de souches virales d'origines différentes, mais du même sous-type, sont présentés dans les figures 17 à 20.

Les résultats obtenus dans les figures 17 à 20 montrent que chacune des sondes 1a/1b, 2a//2c, 2b, 3a ou 4a/4d selon la présente invention est non seulement capable de s'hybrider avec des cibles synthétiques 15-mères qui lui sont spécifiques (la cible ayant une concentration de 10pM), mais aussi avec des amplicons spécifiques du même génotype (à une dilution au 1/100^{ème}). Ces résultats montrent également que les cibles non spécifiques ne s'hybrident pas de manière détectable.

### Résultats 2 : Etude de l'effet du nombre de fonctions thiol

Des tests ELOSA sont également réalisés en faisant varier le nombre de fonctions thiol dans la sonde utilisée et en testant différentes natures et concentrations de cibles (cible synthétique courte 15-mère ou 105-mère, amplicons courts de 143 nt ou 191 nt, amplicons longs de 401 nt).

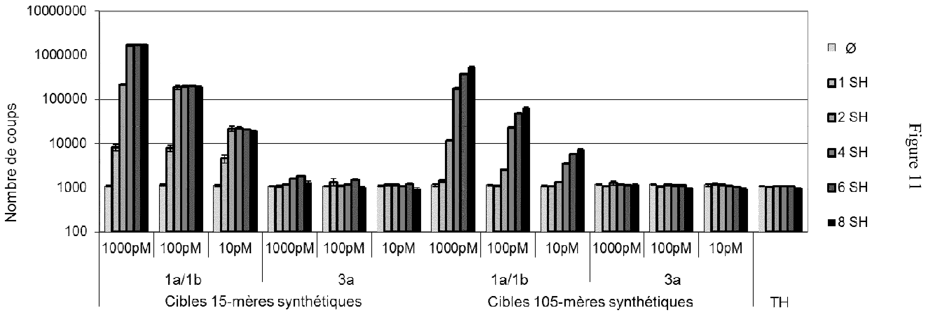

Les résultats présentés dans la figure 11 correspondent à un test ELOSA réalisé avec des sondes 1a/1b linéaires (voir plus haut), d'anomérie béta possédant 1, 2, 4, 6 ou 8 fonctions thiol, qui sont greffées à une densité de 100nM sur un support couvert de groupements maléimide selon le protocole décrit ci-dessus. Les résultats de la figure 11 sont obtenus à partir des cibles suivantes :
- cible synthétique 15-mère ou de 105-mère spécifique 1a/1b, à une concentration de 10pM, 100pM ou 1000pM,
- cible synthétique 15-mère ou 105-mère non spécifique 3a (contrôle négatif), à une concentration de 10pM, 100pM ou 1000pM,
- TH seul, ne comprenant pas de cible.

Chaque condition d'hybridation sonde/cible est testée en triplicat dans une microplaque. Les essais sont reproduits trois fois intégralement de l'étape de greffage des sondes à la détection de l'hybridation (sur 3 microplaques différentes). Les résultats exposés dans la demande correspondent donc à une moyenne de 9 mesures.

Les résultats obtenus dans la figure 11 montrent que l'hybridation d'une cible synthétique 15-mère spécifique 1a/1b avec la sonde 1a/1b est détectable à partir de 10pM de cible, dès la présence d'un thiol. La détection de la cible synthétique 105-mère spécifique 1a/1b nécessite, quant à elle, une concentration en cible de 100pM lorsque la sonde comprend 2 fonctions thiol ou de 10pM lorsque la sonde comprend 4, 6 ou 8 fonctions thiol. Aucune hybridation détectable n'est observée en présence des cibles 3a, non spécifiques de la sonde 1a/1b, indépendamment de leur taille (15-mères ou 105-mères) ou de leur concentration (concentration maximale testée : 1000pM). Les résultats montrent ainsi que l'ajout de fonctions thiol dans la sonde oligonucléotidique greffée améliore les performances de détection des cibles visées. La présence de 4 fonctions thiol dans la sonde utilisée permet de détecter toutes les cibles synthétiques (15-mères ou 105-mères) à une concentration de 10pM.

### Résultats 3 : Etude de l'effet de la densité de greffage

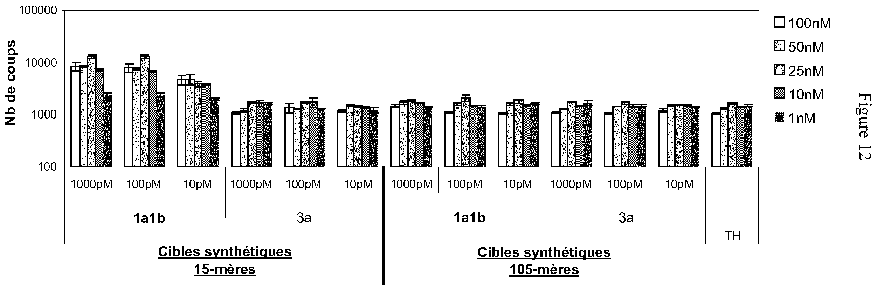

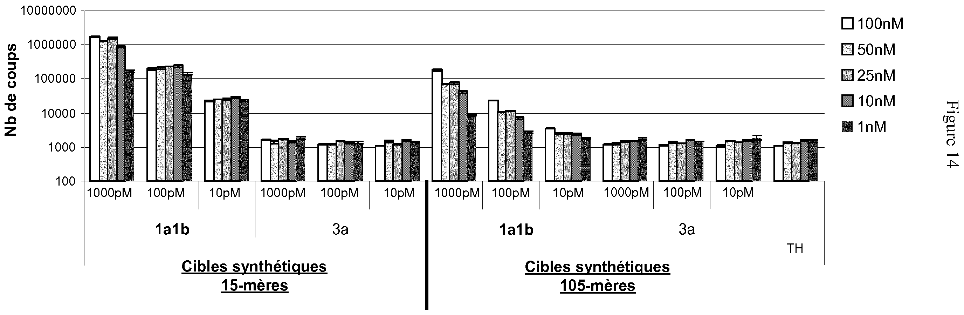

Les résultats présentés dans les figures 12, 13 et 14 correspondent à des tests ELOSA réalisés avec des sondes 1a/1b linéaires (voir plus haut), d'anomérie béta possédant 1, 2 ou 4 fonctions thiol, qui sont greffées à des densités de 1, 10, 25, 50 ou 100nM sur un support couvert de groupements maléimide selon le protocole décrit ci-dessus. La figure 12 illustre les résultats obtenus pour la sonde monothiol, c'est-à-dire comprenant un seul composé thiol selon l'invention. La figure 13 illustre les résultats obtenus pour la sonde dithiol, c'est-à-dire comprenant deux composés thiol selon l'invention, et la figure 14 illustre les résultats obtenus pour la sonde tétrathiol, c'est-à-dire comprenant quatre composés thiol selon l'invention.

Dans chaque cas, les tests d'hybridation sont réalisés avec les cibles suivantes :
- cible synthétique 15-mère ou 105-mère spécifique 1a/1b, à une concentration de 1000pM, de 100pM ou 10pM,
- cible synthétique 15-mère ou 105-mère non spécifique 3a (contrôle négatif), à une concentration de 1000pM, de 100pM ou 10pM,
- TH seul, ne comprenant pas de cible.

Les résultats présentés dans les figures 12, 13 et 14 confirment la spécificité d'hybridation des sondes 1a/1b avec les cibles 1a/1b, dans la mesure où il n'est pas observé d'hybridation détectable en présence des cibles 3a, indépendamment de la concentration de ces dernières.

Ces résultats confirment (1) que la présence d'un seul thiol dans la sonde utilisée ne permet pas de détecter les cibles ayant une taille de 105 nt, (2) qu'à partir de 2 fonctions thiol, il devient possible de détecter l'hybridation entre la sonde et la cible de 105 nt (en particulier à partir de 100pM de cible), (3) que les meilleurs résultats sont obtenus avec la sonde tétrathiol.

D'autre part, il apparaît que la densité de greffage de la sonde monothiol n'influe pas beaucoup sur les signaux d'hybridation quelles que soient les cibles (15-mères et 105-mères), tandis que l'hybridation est dose-dépendante de la densité de greffage dans le cas des sondes dithiol et tétrathiol. Les meilleurs signaux d'hybridation sondes/cibles (15-mères et 105-mères) sont obtenus avec la sonde tétrathiol greffée à 100nM.

### Evaluation des hybridations sondes/cibles sur électrode à surface d'or

Un test d'hybridation a été réalisé en greffant une séquence sonde spécifique 1a/1b sur une électrode à surface d'or.

Une sonde tétrathiol portant un oligonucléotide de séquence SEQ ID NO :1 a été greffée sur l'électrode de travail à surface d'or d'une cellule. L'hybridation de cibles 105-mères spécifiques 1a/1b (de SEQ ID NO : 3) a été évaluée, pour 3 concentrations différentes de cibles : 1pM, 10pM et 100pM. Un contrôle négatif est effectué avec une cible 105-mères spécifique 3a de SEQ ID NO : 6 (témoin négatif).

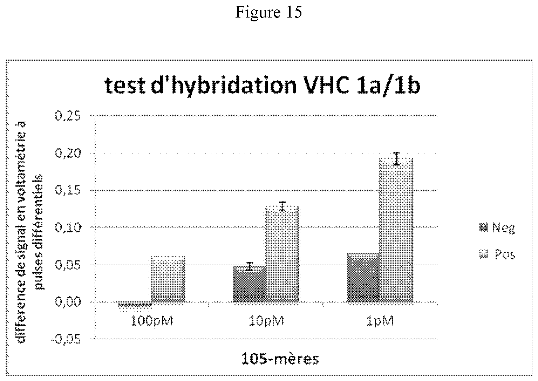

La réaction d'hybridation est suivie par voltamétrie à pulses différentiels. Les résultats obtenus sont présentés en figure 15.

Les valeurs en ordonnée correspondent aux valeurs normalisées de variation de courant.

Les résultats obtenus montrent que le test d'hybridation par électrochimie est sensible et spécifique à une concentration de cibles de 1pM. La variation de signal apparait également être plus forte à 1pM que lorsque le test est mené à 100pM. A plus forte concentration en cible, un effet d'adsorption non spécifique des cibles sur la surface de l'électrode réduit l'efficacité de la réaction de reconnaissance. La méthode électrochimique ne permet pas un suivi quantitatif de la réaction d'hybridation. Néanmoins, elle fournit une réponse oui/non spécifique d'une très grande sensibilité.

### Exemple 4 : Application à la détection de flavivirus, tels que les virus de la Dengue et du West Nile

Des séquences connues des virus de la Dengue (sérotypes 1, 2, 3 et 4) et du West Nile ont été extraites de la base de données GenBank et analysées. Le tableau 1 recense les numéros d'accession GenBank_des séquences de virus de la Dengue et du West Nile ayant servi à réaliser les alignements :

**Tableau 1**

| Virus | Numéro d'accession GenBank des séquences |
|---|---|
| Dengue 1 (8 souches) | FJ882517, FJ882552, EU482480, FJ639683, HM181967, AY726549, GU131762, GU131982 |
| Dengue 2 (17 souches) | GQ398257, HM582109, HM582110, HM582116, HM582117, AY744150, AY744149, AY744148, AY744147, EU056812, AF169686, AF169683, AF169687, AF169682, GU131896, DQ181803, EU073981 |
| Dengue 3 (34 souches) | GQ252678, GU131951, EU482566, GQ868574, GQ868617, FJ882577, GU131868, FJ024465, HM181974, HM181973, GQ199870, AY770511, GU370053, GQ199888, GU131939, GU131937, GU131934, HM631854, GU131905, GU189648, EU660409, DQ109373, DQ109368, DQ109310, AY676351, AY676350, FJ744740, FJ744730, DQ109400, DQ109348, GQ868593, AB214880, AY744677, EF629370 |
| Dengue 4 (20 souches) | AY618992, AY618990, AY858049, AY618993, GQ398256, AF289029, EU854301, EU073983, GU289913, AF326573, FJ882597, GQ199885, GQ199884, GQ199882, GQ868582, GQ868584, AY762085, AY618989, AY618988, EF457906 |
| West Nile (15 souches) | GQ851607, GQ851606, AY369441, AY490240, AY274504, GQ851602, GQ851603, DQ256376, DQ318019, EF429199, EF429200, JN858070, AY277251, FJ159131, FJ159129 |

### Design des sondes oligonucléotidiques pour la reconnaissance de séquences Dengue ou West Nile.

Les alignements et les comparaisons de séquences effectués à l'aide des logiciels clustalW2 et Mega5 ont permis de définir 4 régions, parmi lesquelles :
- 2 sont conservées parmi toutes les souches des virus Dengue et West Nile, ainsi que parmi d'autres flavivirus tels que les virus de l'encéphalite japonaise, de l'encéphalite à tique, de la fièvre jaune et le virus usutu (liste non-exhaustive),
- 1 est conservée parmi les virus de la Dengue,
- 1 est conservée parmi les virus West Nile.

Ces régions ont été utilisées pour dessiner des sondes selon la présente invention, dans le but de permettre la détection générique des flavivirus, ou la détection spécifique des virus de la Dengue, du sous-type 4 de la Dengue ou du virus de West Nile, dans un échantillon biologique.

Les sondes 1 à 3 présentées dans le tableau 2 ci-après permettent ainsi de détecter les flavivirus de manière générale (incluant les flavivirus de la Dengue et du West Nile). La sonde 4 est spécifique des virus de la Dengue, quelque soit le sérotype. La sonde 5 est spécifique du sérotype 4 des virus de la Dengue. La sonde 6 est spécifique des virus West Nile.

**Tableau 2**

| Sonde | Séquence | Spécificité | Taille | SEQ ID |
|---|---|---|---|---|
| Sonde 1 | 5'-GCT CCC ARC CAC AT-3' | Générique | 14-mère | SEQ ID NO: 16 |
| Sonde 2 | 5'-AAC CAT CTR TCT TC-3' | Générique | 14-mère | SEQ ID NO: 17 |
| Sonde 3 | 5'-AGC CAC ATG WAC CA -3' | Générique | 14-mère | SEQ ID NO: 40 |
| Sonde 4 | 5'-CTT CYC CTT CYA CTC-3' | Dengue | 15-mère | SEQ ID NO: 18 |
| Sonde 5 | 5'-CAC TCC ACT CCA TGA-3' | Dengue 4 | 15-mère | SEQ ID NO: 41 |
| Sonde 6 | 5'-CKC CTC CTG ART TCT-3' | West Nile Virus | 15-mère | SEQ ID NO: 19 |

### Design de cibles pour tester la spécificité de reconnaissance des sondes 1 à 6

Des amplicons englobant chacune des 6 régions identifiées sont préparés en effectuant une PCR simple, à partir des séquences mentionnées dans le tableau 1, en utilisant les amorces et le protocole précédemment décrits par Scaramozzino et al. (Scaramozzino N. et al. Comparison of Flavivirus universal primer pairs and developement of a rapid, highly sensitive heminested reverse transcription-PCR assay for détection of flaviviruses targeted to a conserved région of the NS5 gene sequences. 2001. Journal of Clinical Microbiology 39: 1922-1927).

L'amplification primaire par PCR est ainsi réalisée avec :
- l'amorce sens MAMD: [Btn]-5'-AAC-ATG-ATG-GGR-AAR-AGR-GAR-AA-3' (soit [Btn]- SEQ ID NO : 20), et
- l'amorce antisens cFD2 (SEQ ID NO : 21) : 5'-GTG-TCC-CAG-CCG-GCG-GTG-TCA-TCA-GC -3'.

Cette amplification conduit à l'obtention d'un amplicon primaire de 263 pb.

Lorsque la charge virale est très faible, une PCR secondaire est effectuée en utilisant comme matrice les produits d'amplification résultant de la première PCR. Cette PCR « semi-nichée » permet avantageusement un gain significatif de sensibilité.

Un exemple d'amplicon de 263 pb obtenu après amplification à partir de la souche de virus de Dengue référencée sous le numéro FJ882517 a pour séquence : 5'-

Un exemple d'amplicon de 263 pb obtenu après amplification à partir de la souche de virus West Nile référencée sous le numéro EF429200/H442 a pour séquence : 5'-

Une amplification secondaire par PCR peut éventuellement être ensuite réalisée conformément au protocole publié par Scaramozzino et al., en utilisant les amplicons primaires ci-dessus comme matrice avec :
- l'amorce sens FS778 : [Btn] 5'- AAR-GGH-AGY-MCD-GCH-ATH-TGG-T- 3' (soit [Btn]- SEQ ID NO: 22), et
- l'amorce antisens cFD2 (SEQ ID NO : 21) : 5'-GTG-TCC-CAG-CCG-GCG-GTG-TCA-TCA-GC -3'.

Cette amplification conduit à l'obtention d'un amplicon secondaire de 215 pb.

Un exemple d'amplicon de 215 pb obtenu après amplification à partir de la souche de virus de Dengue référencée sous le numéro FJ882517 a pour séquence : 5'-

Un exemple d'amplicon de 215 pb obtenu après amplification à partir de la souche de virus West Nile référencée sous le numéro EF429200/H442 a pour séquence : 5'-

Dans un autre mode de réalisation, les amplicons sont préparés à partir d'ARN viraux de flavivirus, de Dengue ou de virus West Nile par RT-PCR asymétrique. Des ARNs viraux sont extraits à partir de virus Dengue (1-4) et West Nile (d'origine américaine NY ou africaine Af) produits *in vitro.* Des amplicons de 263 pb sont produits à partir de la région virale NS5 des flavivirus, par RT-PCR à partir de chacun des échantillons d'ARN décrits précédemment. Afin d'effectuer l'étape de rétro-transcription (RT), 5µL d'ARN sont dénaturés à 72°C pendant 10 minutes et rétro-transcrits en présence de 4µL de 5X First Strand Buffer (Invitrogen), 2µL de 10X Hexanucleotide Mix (Roche), 2µL de 10mM dNTP mix (Invitrogen) et 200U de SuperScript® II Reverse Transcriptase (Invitrogen) dans un volume total de 20µL. Les conditions de rétro-transcription sont les suivantes : 10 min à 25°C, 60 min à 42°C, et 10 min à 95°C. Cinq microlitres d'ADNc sont ensuite amplifiés par PCR (réaction en chaîne par polymérase) en utilisant les amorces :
- sens flavivirus « MAMD » biotinylée (de séquence : [Btn]- AAC ATG ATG GGR AAR AGR GAR AA (soit [Btn]-SEQ ID NO : 20)) et
- anti-sens flavivirus « cFD2 » (de séquence GTG TCC CAG CCG GCG GTG TCA TCA GC (SEQ ID NO : 21)) (voir Scaramozzino N. et al. 2001, Journal of Clinical Microbiology 39:1922-1927).

La réaction d'amplification asymétrique est effectuée dans 50µL de mélange réactionnel comprenant : 1X PCR Buffer sans MgCl₂ (Invitrogen), 0,2µM de MAMD, 0.02µM de cFD2, 1,5mM MgCl₂ (Invitrogen), 0,2mM dNTP mix (Invitrogen) et 1,5U Taq Polymerase (Invitrogen). Les conditions de PCR sont les suivantes : 5 min à 95°C, 40 cycles (dénaturation: 40 sec, 94°C; hybridation: 40 sec, 53°C; élongation: 50 sec, 72°C), et une extension finale de 10 min à 72°C. Les amplicons obtenus sont analysés par électrophorèse en gel d'agarose, aliquotés et conservés à -20°C avant utilisation. Ces amplicons, dont les génotypes viraux d'origine sont connus, sont conservés sous la forme d'une thèque.

Des oligonucléotides synthétiques 15-mères biotinylés à l'extrémité 5' ont été synthétisés. Ces oligonucléotides sont strictement complémentaires aux sondes choisies ci-dessus, c'est-à-dire à la sonde 3 générique pour détecter les flavivirus, à la sonde générique pour détecter le virus West Nile et à la sonde spécifique du sérotype 4 du virus de la Dengue. Ces oligonucléotides synthétiques possèdent les séquences suivantes :
- Les oligonucléotides synthétiques 14-mères spécifiques des flavivirus ont la séquence : 5' [Btn]- TGG TWC ATG TGG CT-3' (soit [Btn]-SEQ ID NO : 43) ;
- Les oligonucléotides synthétiques 15-mères spécifiques du virus West Nile ont la séquence : 5' [Btn]- AGA AYT CAG GAG GMG -3' (soit [Btn]-SEQ ID NO : 42) et
- Les oligonucléotides synthétiques 15-mères spécifiques du sérotype 4 du virus de la Dengue ont la séquence : 5' [Btn]- TCA TGG AGT GGA GTG -3' (soit [Btn]-SEQ ID NO : 44).

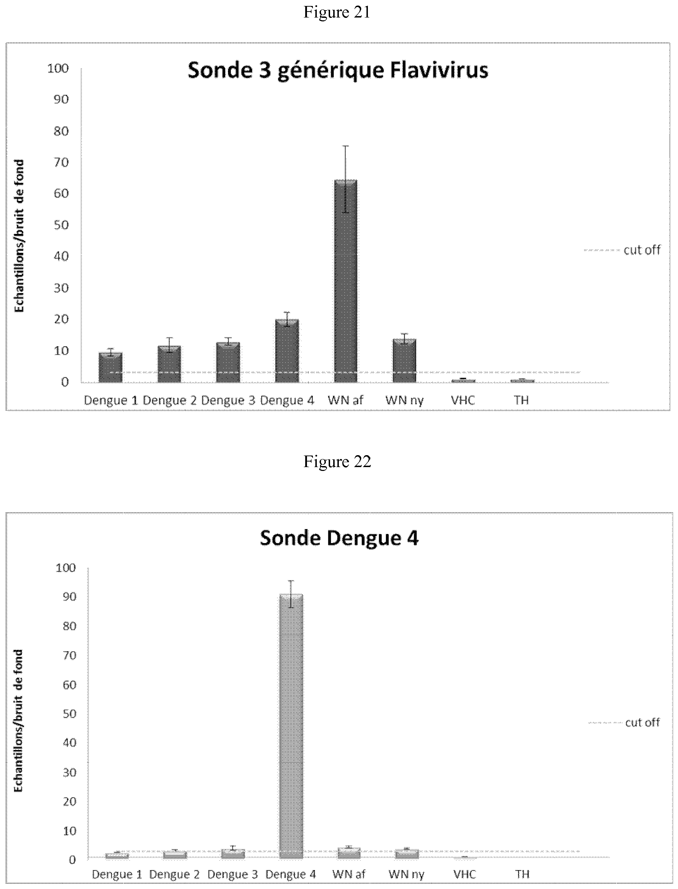

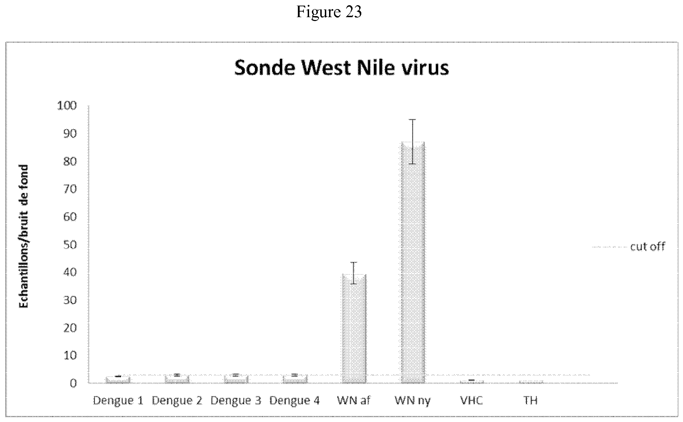

Des tests d'hybridations sont effectués avec la sonde 3 reconnaissant de manière générique les flavivirus (voir figure 21), la sonde 5 reconnaissant spécifiquement le sérotype 4 de la Dengue (voir figure 22) et la sonde 6 reconnaissant le virus West Nile (voir figure 23). Les sondes, possédant 4 fonctions thiol sont greffées à une densité de 200nM sur un support couvert de groupements maléimide selon le protocole décrit plus haut. Des tests ELOSA sont ensuite réalisés en utilisant des amplicons préparés à partir de sérotypes 1, 2, 3 ou 4 de la Dengue, de virus West Nile d'origine américaine (NY) ou Africaine (Af), ou de virus de l'hépatite C (amplicon long 3a), à une dilution de 1/10 pour la sonde 3 et de 1/50 pour les sondes 5 et 6 afin de vérifier la spécificité des sondes retenues. Dans les graphiques des figures 21, 22 et 23, la valeur « échantillons/bruit de fond » est obtenu en calculant le ratio entre le nombre de coups mesuré pour chaque échantillon et le nombre de coups mesuré pour le témoin (TH), correspondant au tampon d'hybridation seul, ne comprenant pas de cible. Les amplicons de Dengue et de virus West Nile utilisés dans ces tests ont été obtenus par la méthode de RT-PCR asymétrique décrite ci-dessus.

Les résultats présentés dans les figures 21, 22 et 23 montrent que la sonde générique flavivirus, la sonde spécifique Dengue 4 et la sonde générique West Nile virus ne s'hybrident de manière significative qu'avec les amplicons qui correspondent aux virus concernés.

## Revendications

1. Oligonucléotide modifié répondant à l'une des formules : 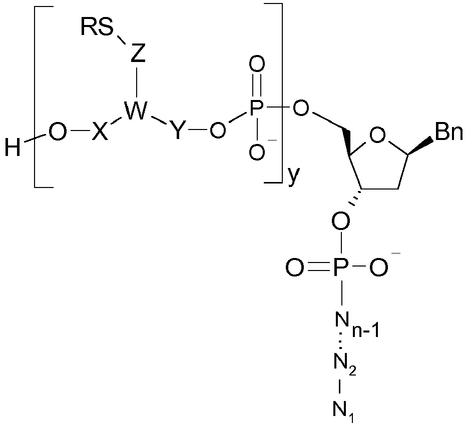 ou 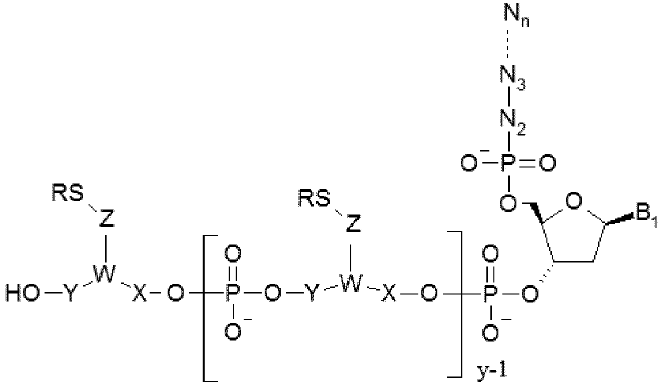 dans lesquelles :
NI, ..., Nn représentent indépendamment les uns des autres un nucléotide,
n est un nombre entier allant de 4 à 100,
y est un nombre entier allant de 2 à 12,
X est choisi parmi les groupements alkyles linéaires ou ramifiés en C1-C12, aminoalkyles en C1-C12, alkoxy en C1-C12, cycloalkyles en C3-C12, cyclohétéroalkyles oxygénés ou azotés en C3-C12,
Y est choisi parmi les groupements alkyles linéaires ou ramifiés en C1-C12, aminoalkyles en C1-C12, alkoxy en C1-C12, cycloalkyles en C3-C12, cyclohétéroalkyles oxygénés ou azotés en C3-C12,
Z est choisi parmi les groupements alkoxy en C1-C12, cyclohétéroalkyles oxygénés ou azotés en C3-C12, NCO-alkyles en C1-C12, CON-alkyles en C1-C12,
W est choisi parmi les groupements alcanes tri-yles en C1-C12, les groupements aryles tri-yles en C6-C18 et les groupements aralcanes tri-yles en C6-C18, et
R est H ou est choisi parmi les groupements acyles en C1-C12, S-alkyles en C1-C12, S-aryles en C6-C12, S-2-pyridine, S-hétéroalkyles oxygénés ou azotés en C1-C12, S-cycloalkyles en C3-C12, S-cyclohétéroalkyles oxygénés ou azotés en C3-C12.
Bₙ représente la base du n-ième nucléotide,
B₁ représente la base du 1er nucléotide.

2. Oligonucléotide modifié selon la revendication 1, dans lequel la séquence de nucléotides (N₁-N₂-...-Nₙ₋₁-Nₙ) est spécifique d'un virus, d'une bactérie ou d'un gène responsable ou impliqué dans une maladie.

3. Oligonucléotide modifié selon la revendication 2, dans lequel la séquence de nucléotides (N₁-N₂-...-Nₙ₋₁-Nₙ) est choisie parmi :
- les séquences de SEQ ID NO : 1, de SEQ ID NO : 4, de SEQ ID NO : 27, de SEQ ID NO : 28, de SEQ ID NO : 35 ou de SEQ ID NO : 36 spécifiques du virus de l'hépatite C (VHC),
- les séquences de SEQ ID NO : 16, de SEQ ID NO : 17 ou de SEQ ID NO : 40, spécifiques des flavivirus,
- la séquence de SEQ ID NO : 18 ou de SEQ ID NO : 41, spécifique des virus de la Dengue, ou
- la séquence de SEQ ID NO : 19, spécifique des virus West Nile (WNV).

4. Oligonucléotide modifié selon l'une des revendications 1 à 3, dans lequel la séquence de nucléotides (N₁-N₂-...-Nₙ₋₁-Nₙ) a une structure de type anomère alpha, anomère béta, linéaire, ou « *snail* ».

5. Substrat greffé par au moins un oligonucléotide modifié selon les revendications 1 à 4, ledit substrat comportant au moins une zone de réception revêtue d'une substance tolérant le greffage dudit oligonucléotide modifié.

6. Substrat greffé selon la revendication 5, dans lequel :
- ladite zone de réception est revêtue d'un film d'or ou de platine, et ledit substrat est en métal, de préférence en cuivre ou en titane, ou
- ladite zone de réception comporte à sa surface au moins une double liaison carbone-carbone ou une triple liaison carbone-carbone ou des fonctions halogénoacétamide, de préférence des fonctions maléimide ou acrylamide, et ledit substrat est en plastique, de préférence en polystyrène.

7. Substrat greffé selon la revendication 5 ou 6, dans lequel ledit substrat est non planaire, et est de préférence une microparticule ou une nanoparticule.

8. Méthode de détection d'au moins un acide nucléique cible dans un échantillon biologique, comprenant une étape de :
- détection dudit acide nucléique cible avec au moins une sonde de détection formée par un oligonucléotide modifié selon l'une des revendications 1 à 4.

9. Méthode de détection selon la revendication 8, comprenant les étapes :
- d'obtention d'au moins un acide nucléique source à partir dudit échantillon biologique,
- de production d'un amplicon par amplification dudit acide nucléique cible à partir de l'acide nucléique source, et
- de détection de l'hybridation dudit amplicon avec au moins une sonde de détection formée par un oligonucléotide modifié selon l'une des revendications 1 à 4.

10. Méthode selon la revendication 9, pour déterminer le génotype et/ou le sous-type d'un virus présent dans un échantillon biologique, dans laquelle
- l'amplicon est généré par amplification d'une séquence nucléotidique cible, correspondant à une région génomique du virus portant des informations relatives au génotype et/ou au sous-type viral, et
- la détection est effectuée avec une sonde spécifique d'un génotype et/ou d'un sous-type viral.

11. Méthode selon la revendication 10, dans laquelle l'étape de production de l'amplicon est réalisée en amplifiant une séquence nucléotidique cible correspondant à une région génomique du virus portant des informations relatives au génotype et/ou au sous-type viral, avec un mélange d'amorces nucléotidiques, de préférence choisies parmi les couples d'amorces :
- SEQ ID NO : 8 et SEQ ID NO : 9, lorsque l'amplicon est généré à partir de tout génotype du VHC;
- SEQ ID NO : 10 et SEQ ID NO : 9, lorsque l'amplicon est généré à partir d'un VHC de génotype 1a/1b ;
- SEQ ID NO : 29 et SEQ ID NO : 9, lorsque l'amplicon est généré à partir d'un VHC de génotype 2 ;
- SEQ ID NO : 8 et SEQ ID NO : 11 lorsque l'amplicon est généré à partir d'un VHC de génotype 3a ;
- SEQ ID NO : 8 et SEQ ID NO : 30, lorsque l'amplicon est généré à partir d'un VHC de génotype 4a/4b ;
- SEQ ID NO : 20 et SEQ ID NO : 21, ou SEQ ID NO : 22 et SEQ ID NO : 21 lorsque l'amplicon est généré à partir d'un flavivirus.

12. Kit de détection d'au moins un acide nucléique cible dans un échantillon biologique comprenant :
- au moins un oligonucléotide modifié selon l'une des revendications 1 à 4 et au moins un substrat comportant au moins une zone de réception revêtue d'une substance tolérant le greffage dudit oligonucléotide modifié, ladite zone de réception étant de préférence revêtue d'or, de platine ou comportant à sa surface au moins une double liaison carbone-carbone ou une triple liaison carbone-carbone ou des fonctions halogénoacétamide, de préférence des fonctions maléimide ou acrylamide, ou
- au moins un substrat greffé selon l'une des revendications 5 à 7.

13. Utilisation d'un oligonucléotide modifié selon l'une des revendications 1 à 4, ou d'un substrat greffé selon l'une des revendications 5 à 7 pour détecter au moins un acide nucléique cible dans un échantillon biologique.

14. Utilisation selon la revendication 13, pour le diagnostic, le génotypage ou le séquençage de souches virales, de préférence de virus des hépatites, ou d'arbovirus de préférence les *Flaviviridae*, les *Togaviridae* ou les *Bunhyaviridae*.

15. Utilisation selon la revendication 14, pour le diagnostic, le génotypage ou le séquençage du VHC, du VHB, des virus de la Dengue ou du virus West Nile.

## Patentansprüche

1. Modifiziertes Oligonukleotid, mit einer der folgenden Formeln 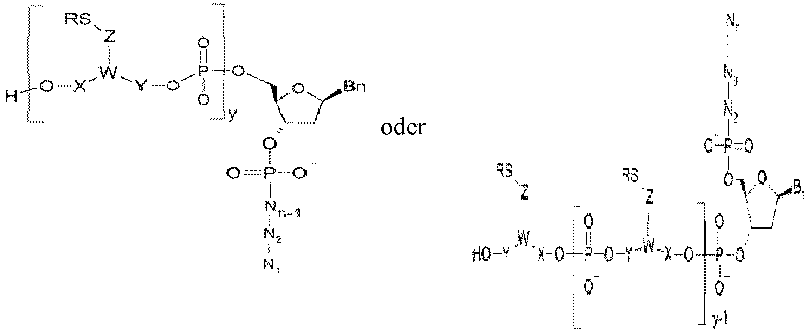 wobei:
N1, ..., Nn unabhängig voneinander ein Nukleotid darstellen,
n eine ganze Zahl von 4 bis 100 ist,
y eine ganze Zahl von 2 bis 12 ist,
X ausgewählt ist aus linearen oder verzweigten C₁-C₁₂ Alkylgruppen, C₁-C₁₂ Aminoalkylgruppen, C₁-C₁₂ Alkoxygruppen, C₃-C₁₂ Cycloalkylgruppen, sauerstoffhaltigen oder stickstoffhaltigen C₃-C₁₂ Cycloheteroalkylgruppen,
Y ausgewählt ist aus linearen oder verzweigten C₁-C₁₂ Alkylgruppen, C₁-C₁₂ Aminoalkylgruppen, C₁-C₁₂ Alkoxygruppen, C₃-C₁₂ Cycloalkylgruppen, sauerstoffhaltigen oder stickstoffhaltigen C₃-C₁₂ Cycloheteroalkylgruppen,
Z ausgewählt ist aus C₁-C₁₂ Alkoxygruppen, sauerstoffhaltigen oder stickstoffhaltigen C₃-C₁₂ Cycloheteroalkylgruppen, C₁-C₁₂ NCO-Alkyl--gruppen, C₁-C₁₂ CON-Alkylgruppen,
W ausgewählt ist aus C₁-C₁₂ Alkan-tri-yl-gruppen, C₆-C₁₈ Aryl-tri-yl-Gruppen und C₆-C₁₈ Aralkan-tri-yl-Gruppen, und
R gleich H ist oder ausgewählt ist aus C₁-C₁₂ Acylgruppen, C₁-C₁₂ S-Alkylgruppen, C₆-C₁₂ S-Arylgruppen, S-2-Pyridingruppen, sauerstoffhaltigen oder stickstoffhaltigen C₁-C₁₂ S-Heteroalkylgruppen, C₃-C₁₂ S-Cycloalkylgruppen, sauerstoffhaltigen oder stickstoffhaltigen C₃-C₁₂ S-Cycloheteroalkylgruppen,
Bₙ die Base des n-ten Nukleotids darstellt,
B₁ die Base des ersten Nukleotids darstellt.

2. Modifiziertes Oligonukleotid nach Anspruch 1, bei dem die Nukleotidsequenz (N₁-N₂-...-Nₙ₋₁-Nₙ) spezifisch für ein Virus, ein Bakterium oder ein Gen ist, das verantwortlich für eine Erkrankung oder daran beteiligt ist.

3. Modifiziertes Oligonukleotid nach Anspruch 2, wobei die Sequenz der Nukleotide (N₁-N₂-...-Nₙ₋₁-Nₙ) ausgewählt ist aus:
- den Sequenzen von SEQ ID NO: 1, SEQ ID NO: 4, SEQ ID NO: 27, SEQ ID NO: 28, SEQ ID NO: 35 oder SEQ ID NO: 36, die spezifisch für das Hepatitis C Virus (HCV) sind,
- den Sequenzen von SEQ ID NO: 16, SEQ ID NO: 17 oder SEQ ID NO: 40, die spezifisch für Flaviviren sind,
- der Sequenz von SEQ ID NO: 18 oder SEQ ID NO: 41, die spezifisch für Dengue-Viren sind, oder
- der Sequenz von SEQ ID NO: 19, die spezifisch für das West-Nil-Virus (WNV) ist.

4. Modifiziertes Oligonukleotid nach einem der Ansprüche 1 bis 3, wobei die Nukleotidsequenz (N₁-N₂ -...- Nₙ₋₁-Nn) die Struktur vom Typ alpha-Anomer, beta-Anomer, linear oder "Schnecke" aufweist.

5. Träger, der mit mindestens einem modifizierten Oligonukleotid nach den Ansprüchen 1 bis 4 gepfropft ist, wobei der Träger mindestens einen Rezeptorbereich umfasst, der mit einer Substanz beschichtet ist, welche die Pfropfung des modifizierten Oligonukleotids toleriert.

6. Gepfropfter Träger nach Anspruch 5, wobei:
- der Rezeptorbereich mit einer Folie aus Gold oder Platin beschichtet ist und der Träger aus Metall besteht, vorzugsweise aus Kupfer oder Titan, oder
- der Rezeptorbereich an seiner Oberfläche mindestens eine Kohlenstoff-Kohlenstoff Doppelbindung oder eine Kohlenstoff-Kohlenstoff Dreifachbindung oder Halogenacetamid-Funktionen umfasst, vorzugsweise Maleimid- oder Acrylamid-Funktionen und der Träger aus Kunststoff ist, vorzugsweise aus Polystyrol.

7. Gepfropfter Träger nach Anspruch 5 oder 6, wobei der Träger nicht eben ist und vorzugsweise ein Mikropartikel oder ein Nanopartikel ist.

8. Verfahren zum Nachweis mindestens einer Zielnukleinsäure in einer biologischen Probe, das den folgenden Schritt umfasst:
- Nachweis der Zielnukleinsäure mit mindestens einer Nachweissonde, die von einem modifizierten Oligonukleotid nach einem der Ansprüche 1 bis 4 gebildet wird.

9. Nachweisverfahren nach Anspruch 8, das die folgenden Schritte umfasst:
- Erhalten mindestens einer ursprünglichen Nukleinsäure aus der biologischen Probe,
- Herstellung eines Amplikons durch Amplifikation der Zielnukleinsäure aus der ursprünglichen Nukleinsäure und
- Nachweis der Hybridisierung des Amplikons mit wenigstens einer Nachweissonde, die von einem modifizierten Oligonukleotid nach einem der Ansprüche 1 bis 4 gebildet wird.

10. Verfahren nach Anspruch 9 zum Bestimmen des Genotyps und/oder Subtyps eines Virus, der in einer biologischen Probe vorliegt, wobei
- das Amplikon durch Amplifikation einer Zielnukleotidsequenz erzeugt wird, die einer genomischen Region des Virus entspricht, welche Informationen über den Genotyp und/oder den viralen Subtyp trägt, und
- der Nachweis mit einer Sonde erfolgt, die für einen bestimmten Genotyp und/oder einen viralen Subtyp spezifisch ist.

11. Verfahren nach Anspruch 10, wobei der Schritt des Herstellens des Amplikons durch Amplifizieren einer Zielnukleotidsequenz, die einer genomischen Region des Virus entspricht, welche Informationen über den Genotyp und/oder den viralen Subtyp trägt, mit einem Gemisch von Nukleotid-Primern erfolgt, vorzugsweise ausgewählt aus den folgenden Primerpaaren:
- SEQ ID NO: 8 und SEQ ID NO: 9, wenn das Amplikon aus einem beliebigen HCV Genotyp erzeugt wird;
- SEQ ID NO: 10 und SEQ ID NO: 9, wenn das Amplikon aus einem HCV vom Genotyp 1a/1b erzeugt wird;
- SEQ ID NO: 29 und SEQ ID NO: 9, wenn das Amplikon aus einem HCV vom Genotyp 2 erzeugt wird;
- SEQ ID NO: 8 und SEQ ID NO: 11, wenn das Amplikon aus einem HCV vom Genotyp 3a erzeugt wird;
- SEQ ID NO: 8 und SEQ ID NO: 30, wenn das Amplikon aus einem HCV vom Genotyp 4a/4b erzeugt wird;
- SEQ ID NO: 20 und SEQ ID NO: 21 oder SEQ ID NO: 22 und SEQ ID NO: 21, wenn das Amplikon aus einem Flavivirus erzeugt wird.

12. Kit zum Nachweisen mindestens einer Zielnukleinsäure in einer biologischen Probe, der Folgendes umfasst:
- mindestens ein modifiziertes Oligonukleotid nach einem der Ansprüche 1 bis 4 und mindestens einen Träger, der mindestens einen Rezeptorbereich umfasst, der mit einer Substanz beschichtet ist, welche das Pfropfen des modifizierten Oligonukleotids toleriert, wobei der Rezeptorbereich vorzugsweise mit Gold beschichtet ist, mit Platin oder an seiner Oberfläche mindestens eine Kohlenstoff-Kohlenstoff Doppelbindung oder eine Kohlenstoff-Kohlenstoff Dreifachbindung oder Halogenacetamid-Funktionen aufweist, vorzugsweise Maleimid- oder Acrylamid-Funktionen, oder
- mindestens einen gepfropften Träger nach einem der Ansprüche 5 bis 7.

13. Verwendung eines modifizierten Oligonukleotids nach einem der Ansprüche 1 bis 4, oder eines gepfropften Trägers nach einem der Ansprüche 5 bis 7 zum Nachweisen mindestens einer Zielnukleinsäure in einer biologischen Probe.

14. Verwendung nach Anspruch 13 zur Diagnose, zur Genotypisierung oder zur Sequenzierung von Virusstämmen, vorzugsweise von Hepatitisviren oder Arboviren, vorzugsweise *Flaviviridae, Togaviridae* oder *Bunhyaviridae.*

15. Verwendung nach Anspruch 14 zur Diagnose, zur Genotypisierung oder zur Sequenzierung von HCV, HBV, Dengue-Viren oder des West-Nil-Virus.

## Claims

1. Modified oligonucleotide corresponding to one of the formulae: 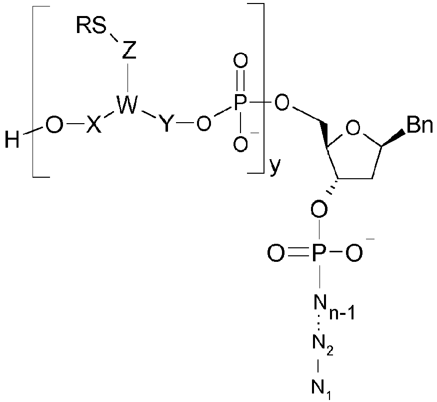 or 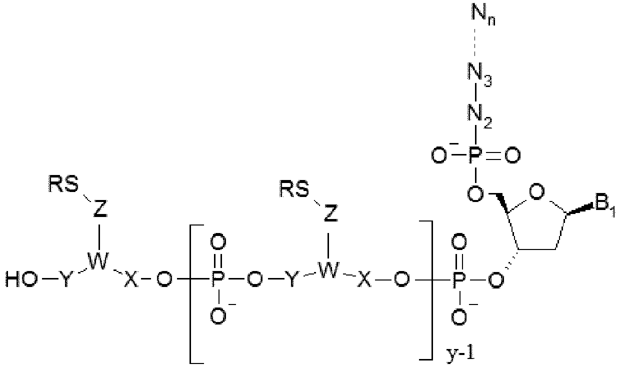 in which,
N1, ..., Nn represent, independently of one another, a nucleotide,
n is an integer ranging from 4 to 100,
y is an integer ranging from 2 to 12,
X is selected from the linear or branched C1-C12 alkyl groups, C1-C12 aminoalkyl groups, C1-C12 alkoxy groups, C3-C12 cycloalkyl groups, oxygen-containing or nitrogen-containing C3-C12 cycloheteroalkyl groups,
Y is selected from the linear or branched C1-C12 alkyl groups, C1-C12 aminoalkyl groups, C1-C12 alkoxy groups, C3-C12 cycloalkyl groups, oxygen-containing or nitrogen-containing C3-C12 cycloheteroalkyl groups,
Z is selected from the C1-C12 alkoxy groups, oxygen-containing or nitrogen-containing C3-C12 cycloheteroalkyl groups, C1-C12 NCO-alkyl groups, C1-C12 CON-alkyl groups,
W is selected from the C1-C12 alkane triyl groups, the C6-C18 aryl triyl groups and the C6-C18 aralkane triyl groups, and
R is H or is selected from the C1-C12 acyl, C1-C12 S-alkyl, C6-C12 S-aryl, S-2-pyridine, oxygen-containing or nitrogen-containing C1-C12 S-heteroalkyl, C3-C12 S-cycloalkyl, oxygen-containing or nitrogen-containing C3-C12 S-cycloheteroalkyl groups.
Bₙ represents the base of the n-th nucleotide,
B₁ represents the base of the 1^{st} nucleotide.

2. Modified oligonucleotide according to claims 1, in which the nucleotide sequence (N₁-N₂-...-Nₙ₋₁-Nₙ) is specific to a virus, a bacterium or a gene responsible for or involved in a disease.

3. Modified oligonucleotide according to claim 2, in which the nucleotide sequence (N₁-N₂-...-Nₙ₋₁-Nₙ) is selected from:
- the sequences SEQ ID NO: 1, SEQ ID NO: 4, SEQ ID NO: 27, SEQ ID NO: 28, SEQ ID NO: 35 or SEQ ID NO: 36 specific to the hepatitis C virus (HCV),
- the sequences SEQ ID NO: 16, SEQ ID NO: 17 or SEQ ID NO: 40, specific to the flaviviruses,
- the sequence SEQ ID NO: 18 or SEQ ID NO: 41, specific to the dengue viruses, or
- the sequence SEQ ID NO: 19, specific to the West Nile viruses (WNV).

4. Modified oligonucleotide according to one of claims 1 to 3, in which the nucleotide sequence (N₁-N₂-...-Nₙ₋₁-Nₙ) has a structure of the alpha anomer, beta anomer, linear, or "snail" type.

5. Substrate grafted with at least one modified oligonucleotide according to claims 1 to 4, said substrate comprising at least one receiving zone coated with a substance that tolerates the grafting of said modified oligonucleotide.

6. Grafted substrate according to claim 5, in which:
- said receiving zone is coated with a gold or platinum film, and said substrate is of metal, preferably of copper or titanium, or
- said receiving zone comprises on its surface at least one carbon-carbon double bond or carbon-carbon triple bond or haloacetamide functions, preferably maleimide or acrylamide functions, and said substrate is of plastic, preferably of polystyrene.

7. Grafted substrate according to claim 5 or 6, in which said substrate is non-planar, and is preferably a microparticle or a nanoparticle.

8. Method for detecting at least one target nucleic acid in a biological sample, comprising a step of:
- detecting said target nucleic acid with at least one detection probe formed by a modified oligonucleotide according to one of claims 1 to 4.

9. Detection method according to claim 8, comprising the steps of:
- obtaining at least one source nucleic acid from said biological sample,
- producing an amplicon by the amplification of said target nucleic acid from the source nucleic acid, and
- detecting the hybridization of said amplicon with at least one detection probe formed by a modified oligonucleotide according to one of claims 1 to 4.

10. Method according to claim 9, for determining the genotype and/or subtype of a virus present in a biological sample, in which
- the amplicon is generated by the amplification of a target nucleotide sequence, corresponding to a genomic region of the virus bearing information relating to the viral genotype and/or subtype, and
- detection is carried out with a probe specific to a viral genotype and/or subtype.

11. Method according to claim 10, in which the step of production of the amplicon is carried out by amplifying a target nucleotide sequence corresponding to a genomic region of the virus bearing information relating to the viral genotype and/or subtype, with a mixture of nucleotide primers, preferably selected from the primer pairs:
- SEQ ID NO: 8 and SEQ ID NO: 9, when the amplicon is generated starting from any genotype of HCV;
- SEQ ID NO: 10 and SEQ ID NO: 9, when the amplicon is generated starting from an HCV of genotype 1a/1b;
- SEQ ID NO: 29 and SEQ ID NO: 9, when the amplicon is generated starting from an HCV of genotype 2;
- SEQ ID NO: 8 and SEQ ID NO: 11, when the amplicon is generated starting from an HCV of genotype 3a;
- SEQ ID NO: 8 and SEQ ID NO: 30, when the amplicon is generated starting from an HCV of genotype 4a/4b;
- SEQ ID NO: 20 and SEQ ID NO: 21, or SEQ ID NO: 22 and SEQ ID NO: 21 when the amplicon is generated from a flavivirus.

12. Kit for detecting at least one target nucleic acid in a biological sample comprising:
- at least one modified oligonucleotide according to one of claims 1 to 4 and at least one substrate comprising at least one receiving zone coated with a substance that tolerates the grafting of said modified oligonucleotide, said receiving zone preferably being coated with gold, with platinum or comprising on its surface at least one carbon-carbon double bond or carbon-carbon triple bond or haloacetamide functions, preferably maleimide or acrylamide functions, or
- at least one grafted substrate according to one of claims 5 to 7.

13. Use of a modified oligonucleotide according to one of claims 1 to 4, or of a grafted substrate according to one of claims 5 to 7 for detecting at least one target nucleic acid in a biological sample.

14. Use according to claim 13, for diagnostics, genotyping or sequencing of viral strains, preferably of hepatitis viruses, or of arboviruses, preferably the *Flaviviridae,* the *Togaviridae* or the *Bunhyaviridae.*

15. Use according to claim 14, for diagnostics, genotyping or sequencing of HCV, HBV, dengue viruses or West Nile virus.


### SEQUENCE LISTING

<110> Etablissement FranÃ§ais du Sang (EFS)
   Centre National de la Recherche Scientifique (CNRS)
   UniversitÃ© de Montpellier 1
   UniversitÃ© Claude Bernard Lyon 1
<120> Oligonucleotides modifies comprenant des fonctions thiol et leur utilisation pour la détection d'acides nucléiques
<130> 33740 EFN
<160> 44
<170> BiSSAP 1.0
<210> 1
   <211> 15
   <212> DNA
   <213> artificial sequences
<220>
   <221> source
   <222> 1..15
   <223> /mol_type="DNA"
   /note="Sonde VHC la/lb"
   /organism="artificial sequences"
<400> 1
   gtgcagtccy ggagc 15
<210> 2
   <211> 15
   <212> DNA
   <213> artificial sequences
<220>
   <221> source
   <222> 1..15
   <223> /mol_type="DNA"
   /note="Cible VHC 1a/1b 15-mÃ¨res"
   /organism="artificial sequences"
<400> 2
   gctccrggac tgcac 15
<210> 3
   <211> 105
   <212> DNA
   <213> artificial sequences
<220>
   <221> source
   <222> 1..105
   <223> /mol_type="DNA"
   /note="Cible VHC 1a/1b 105-mÃ¨res"
   /organism="artificial sequences"
<400> 3
   cctcacttgc tacatcaagg cccaggcagc ctgtcgagcc gcagggctcc gggactgcac 60
   catgctcgtg tgtggcgacg acttagtcgt tatctgtgaa agtgc 105
<210> 4
   <211> 15
   <212> DNA
   <213> artificial sequences
<220>
   <221> source
   <222> 1..15
   <223> /mol_type="DNA"
   /note="Sonde VHC 3a"
   /organism="artificial sequences"
<400> 4
   ggcctccggt tcaag 15
<210> 5
   <211> 15
   <212> DNA
   <213> artificial sequences
<220>
   <221> source
   <222> 1..15
   <223> /mol_type="DNA"
   /note="Cible VHC 3a 15-mÃ¨res"
   /organism="artificial sequences"
<400> 5
   cttgaaccgg aggcc 15
<210> 6
   <211> 105
   <212> DNA
   <213> artificial sequences
<220>
   <221> source
   <222> 1..105
   <223> /mol_type="DNA"
   /note="Cible VHC 3a 105-mÃ¨res"
   /organism="artificial sequences"
<400> 6
   gaacaggaca tcagggtgga agaggagata taccaatgct gtaaccttga accggaggcc 60
   aggaaagtga tctcctccct cacggagcgg ctttactgcg ggggc 105
<210> 7
   <211> 13
   <212> DNA
   <213> artificial sequences
<220>
   <221> source
   <222> 1..13
   <223> /mol_type="DNA"
   /note="Acide nuclÃ©ique test greffage"
   /organism="artificial sequences"
<400> 7
   ccaagcacga tgt 13
<210> 8
   <211> 31
   <212> DNA
   <213> artificial sequences
<220>
   <221> source
   <222> 1..31
   <223> /mol_type="DNA"
   /note="Amorce VHC sens biotinylÃ©e"
   /organism="artificial sequences"
<400> 8
   tggggatccc gtatgatacc cgctgctttg a 31
<210> 9
   <211> 30
   <212> DNA
   <213> artificial sequences
<220>
   <221> source
   <222> 1..30
   <223> /mol_type="DNA"
   /note="Amorce VHC anti-sens"
   /organism="artificial sequences"
<400> 9
   ggcggaattc ctggtcatag cctccgtgaa 30
<210> 10
   <211> 25
   <212> DNA
   <213> artificial sequences
<220>
   <221> source
   <222> 1..25
   <223> /mol_type="DNA"
   /note="Amorce VHC 1a/1b sens biotinylÃ©e"
   /organism="artificial sequences"
<400> 10
   tgacracyag ctgyggtaay accct 25
<210> 11
   <211> 21
   <212> DNA
   <213> artificial sequences
<220>
   <221> source
   <222> 1..21
   <223> /mol_type="DNA"
   /note="Amorce VHC anti-sens 3a rev "
   /organism="artificial sequences"
<400> 11
   gcagtaaagc cgytccgtga g 21
<210> 12
   <211> 401
   <212> DNA
   <213> Hepatitis C virus
<220>
   <221> source
   <222> 1..401
   <223> /mol_type="DNA"
   /note="Amplicon VHC long 401nt 1a/1b #PTR6719"
   /organism="Hepatitis C virus"
<400> 12
   tggggatccc gtatgatacc cgctgctttg actcaacggt cactgagaat gacatccgtg      60
   ttgaggagtc aatttaccaa tgttgtgacc tagcccccga agccagacag gccataaggt     120
   cgctcacaga gcggctttac atcgggggtc ccctgactaa ttcaaaaggg cagaactgcg     180
   gctatcgccg gtgccgcgcg agcggtgtgc tgacgaccag ctgcggtaat accctcacat     240
   gttacttgaa ggcctctgcg gcctgtcgag ctgcaaagct ccaggactgc acaatgctcg     300
   tgtgcggaga cgaccttgtc gttatctgtg aaagcgcggg aacccargag gatgcggcga     360
   gcctacgagt cttcacggag gctatgacca ggaattccgc c                         401
<210> 13
   <211> 191
   <212> DNA
   <213> Hepatitis C virus
<220>
   <221> source
   <222> 1..191
   <223> /mol_type="DNA"
   /note="Amplicon VHC court 191nt 1a/1b #PTR6719"
   /organism="Hepatitis C virus"
<400> 13
   tgacgaccag ctgcggtaat accctcacat gttacttgaa ggcctctgcg gcctgtcgag      60
   ctgcaaagct ccaggactgc acaatgctcg tgtgcggaga cgaccttgtc gttatctgtg     120
   aaagcgcggg aacccargag gatgcggcga gcctacgagt cttcacggag gctatgacca     180
   ggaattccgc c                                                          191
<210> 14
   <211> 401
   <212> DNA
   <213> Hepatitis C virus
<220>
   <221> source
   <222> 1..401
   <223> /mol_type="DNA"
   /note="Amplicon VHC long 401nt 3a #PTR9058"
   /organism="Hepatitis C virus"
<400> 14
   tggggatccc gtatgatacc cgctgctttg actcgactgt cactgaacag gatatcaggg 60
   tggaagagga gatataccaa tgctgtaatc ttgaaccgga ggccaggaag gtgatctcct 120
   ccctcacgga gcggctttac tgcgggggtc ctatgttcaa cagcaaaggg gcccagtgtg 180
   gttatcgccg ttgccgtgct agtggagttc tacctaccag cttcggcaat acaatcactt 240
   gctacatcaa ggccacagcg gctgcaaggg ccgcaggcct ccggaacccg gactttcttg 300
   tctgcggaga cgatctagtc gtggtggctg agagtgacgg cgtcgacgag gatggggcgg 360
   ccctgagagc cttcacggag gctatgacca ggaattccgc c 401
<210> 15
   <211> 143
   <212> DNA
   <213> Hepatitis C virus
<220>
   <221> source
   <222> 1..143
   <223> /mol_type="DNA"
   /note="Amplicon VHC court 143nt 3a #PTR9058"
   /organism="Hepatitis C virus"
<400> 15
   tggggatccc gtatgatacc cgctgctttg actcgactgt cactgaacag gatatcaggg 60
   tggaagagga gatataccaa tgctgtaatc ttgaaccgga ggccaggaag gtgatctcct 120
   ccctcacgga gcggctttac tgc 143
<210> 16
   <211> 14
   <212> DNA
   <213> artificial sequences
<220>
   <221> source
   <222> 1..14
   <223> /mol_type="DNA"
   /note="Sonde gÃ©nÃ©rique 1 flavivirus"
   /organism="artificial sequences"
<400> 16
   gctcccarcc acat 14
<210> 17
   <211> 14
   <212> DNA
   <213> artificial sequences
<220>
   <221> source
   <222> 1..14
   <223> /mol_type="DNA"
   /note="Sonde gÃ©nÃ©rique 2 flavivirus"
   /organism="artificial sequences"
<400> 17
   aaccatctrt cttc 14
<210> 18
   <211> 15
   <212> DNA
   <213> artificial sequences
<220>
   <221> source
   <222> 1..15
   <223> /mol_type="DNA"
   /note="Sonde spÃ©cifique 3 Dengue"
   /organism="artificial sequences"
<400> 18
   cttcyccttc yactc 15
<210> 19
   <211> 15
   <212> DNA
   <213> artificial sequences
<220>
   <221> source
   <222> 1..15
   <223> /mol_type="DNA"
   /note="Sonde spÃ©cifique 4 West Nile "
   /organism="artificial sequences"
<400> 19
   ckcctcctga rttct 15
<210> 20
   <211> 23
   <212> DNA
   <213> artificial sequences
<220>
   <221> source
   <222> 1..23
   <223> /mol_type="DNA"
   /note="Amorce sens flavivirus MAMD "
   /organism="artificial sequences"
<400> 20
   aacatgatgg graaragrga raa 23
<210> 21
   <211> 26
   <212> DNA
   <213> artificial sequences
<220>
   <221> source
   <222> 1..26
   <223> /mol_type="DNA"
   /note="Amorce anti-sens flavivirus cFD2"
   /organism="artificial sequences"
<400> 21
   gtgtcccagc cggcggtgtc atcagc 26
<210> 22
   <211> 22
   <212> DNA
   <213> artificial sequences
<220>
   <221> source
   <222> 1..22
   <223> /mol_type="DNA"
   /note="Amorce sens flavivirus FS778"
   /organism="artificial sequences"
<400> 22
   aargghagym cdgchathtg gt 22
<210> 23
   <211> 263
   <212> DNA
   <213> Dengue virus 1
<220>
   <221> source
   <222> 1..263
   <223> /mol_type="DNA"
   /note="Amplicon Dengue 1 263nt FJ882517"
   /organism="Dengue virus 1"
<400> 23
   aacatgatgg ggaagagaga gaaaaaacta ggagagttcg gaaaggcaaa aggaagtcgt 60
   gcaatatggt acatgtggct gggagcacgc tttctagagt tcgaagctct tggtttcatg 120
   aacgaagatc actggttcag cagagagaat tcactcagcg gagtggaagg agaaggactc 180
   cacaaacttg gatatatact cagagacata tcaaagattc cagggggaaa catgtatgca 240
   gatgacacag ccggatggga cac 263
<210> 24
   <211> 215
   <212> DNA
   <213> Dengue virus 1
<220>
   <221> source
   <222> 1..215
   <223> /mol_type="DNA"
   /note="Amplicon Dengue 1 215nt FJ882517"
   /organism="Dengue virus 1"
<400> 24
   aaaggaagtc gtgcaatatg gtacatgtgg ctgggagcac gctttctaga gttcgaagct      60
   cttggtttca tgaacgaaga tcactggttc agcagagaga attcactcag cggagtggaa     120
   ggagaaggac tccacaaact tggatatata ctcagagaca tatcaaagat tccaggggga     180
   aacatgtatg cagatgacac agccggatgg gacac                                215
<210> 25
   <211> 263
   <212> DNA
   <213> West Nile virus
<220>
   <221> source
   <222> 1..263
   <223> /mol_type="DNA"
   /note="Amplicon WNV 263nt EF429200H442"
   /organism="West Nile virus"
<400> 25
   aacatgatgg gaaagagaga gaagaagcct ggagagttcg gcaaggctaa aggcagcaga      60
   gccatctggt tcatgtggct gggggctcgt ttcctggagt ttgaagctct cggattcctc     120
   aatgaagacc actggctggg taggaagaac tcaggaggag gagttgaagg cttaggactg     180
   cagaagcttg ggtacatctt gaaggaagtt gggacaaagc ctggaggaaa gatttacgcc     240
   gatgataccg caggctggga cac                                             263
<210> 26
   <211> 215
   <212> DNA
   <213> West Nile virus
<220>
   <221> source
   <222> 1..215
   <223> /mol_type="DNA"
   /note="Amplicon WNV 215nt EF429200H442"
   /organism="West Nile virus"
<400> 26
   aaaggcagca gagccatctg gttcatgtgg ctgggggctc gtttcctgga gtttgaagct      60
   ctcggattcc tcaatgaaga ccactggctg ggtaggaaga actcaggagg aggagttgaa     120
   ggcttaggac tgcagaagct tgggtacatc ttgaaggaag ttgggacaaa gcctggagga     180
   aagatttacg ccgatgatac cgcaggctgg gacac                                215
<210> 27
   <211> 15
   <212> DNA
   <213> artificial sequences
<220>
   <221> source
   <222> 1..15
   <223> /mol_type="DNA"
   /note="Sonde VHC 2"
   /organism="artificial sequences"
<400> 27
   tggctytctg agatg 15
<210> 28
   <211> 15
   <212> DNA
   <213> Hepatitis C virus
<220>
   <221> source
   <222> 1..15
   <223> /mol_type="DNA"
   /note="Sonde VHC 4a/4d"
   /organism="Hepatitis C virus"
<400> 28
   ggrccgccca crtag 15
<210> 29
   <211> 21
   <212> DNA
   <213> artificial sequences
<220>
   <221> source
   <222> 1..21
   <223> /mol_type="DNA"
   /note="Amorce VHC2 sens biotinylÃ©e"
   /organism="artificial sequences"
<400> 29
   atgytggtrt gcggcgacga c 21
<210> 30
   <211> 23
   <212> DNA
   <213> artificial sequences
<220>
   <221> source
   <222> 1..23
   <223> /mol_type="DNA"
   /note="Amorce VHC anti-sens 4 rev"
   /organism="artificial sequences"
<400> 30
   aggtctcccy tgctgttgtr cat 23
<210> 31
   <211> 401
   <212> DNA
   <213> Hepatitis C virus
<220>
   <221> source
   <222> 1..401
   <223> /mol_type="DNA"
   /note="AmpliconVHC long 2 401nt #PTR7761"
   /organism="Hepatitis C virus"
<400> 31
   tggggatccc gtatgatacc cgctgctttg actcaactgt cactgagaga gacatcagaa      60
   ccgaggagtc catataccag gcctgctccc taaccgagga ggctcgcacc gccatacact     120
   cgctgactga gaggctatac gtgggagggc ccatgctcaa tagcaaaggc cagacctgcg     180
   ggtacaggcg ttgccgcgcc agcggggtgc tcaccactag catgggaaac accattacgt     240
   gctatgtgaa agctctagcg gcatgcaagg ccgcagggat agtagcgccc acgatgctgg     300
   tatgcggcga cgacctggtc gtcatctcag aaagccaggg gactgaggag gacgagcgga     360
   acctgagagt cttcacggag gctatgacca ggaattccgc c                          401
<210> 32
   <211> 401
   <212> DNA
   <213> Hepatitis C virus
<220>
   <221> source
   <222> 1..401
   <223> /mol_type="DNA"
   /note="Amplicon VHC long 4a/4d 401nt #PTR4162"
   /organism="Hepatitis C virus"
<400> 32
   tggggatccc gtatgatacc cgctgctttg actccactgt aaccgaaaga gacatcaggg      60
   tcgaggagga ggtctatcag tgttgtgacc tagagcccga agcccgcaag gtaatatccg     120
   ccctcacaga gagactctac gtgggcggtc ccatgtacaa cagcagggga gacctttgcg     180
   gaactcgacg gtgccgtgca agcggcgtat tcaccaccag ctttgggaac acactgacgt     240
   gctatcttaa ggccagcgcg gccatcaggg ctgcaggcct aaaggactgc accatgctgg     300
   tctgtggcga cgacttagtc gttatcgctg aaagcgatgg cgtggaggag gacaaccgtg     360
   cgctcagagc cttcacggag gctatgacca ggaattccgc c                          401
<210> 33
   <211> 108
   <212> DNA
   <213> Hepatitis C virus
<220>
   <221> source
   <222> 1..108
   <223> /mol_type="DNA"
   /note="Amplicon VHC court 108nt 2 #PTR7761"
   /organism="Hepatitis C virus"
<400> 33
   atgctggtat gcggcgacga cctggtcgtc atctcagaaa gccaggggac tgaggaggac 60
   gagcggaacc tgagagtctt cacggaggct atgaccagga attccgcc 108
<210> 34
   <211> 175
   <212> DNA
   <213> Hepatitis C virus
<220>
   <221> source
   <222> 1..175
   <223> /mol_type="DNA" /note="Amplicon VHC court 4a/4d 175nt #PTR4162" /organism="Hepatitis C virus"
<400> 34
   tggggatccc gtatgatacc cgctgctttg actccactgt aaccgaaaga gacatcaggg 60
   tcgaggagga ggtctatcag tgttgtgacc tagagcccga agcccgcaag gtaatatccg 120
   ccctcacaga gagactctac gtgggcggtc ccatgtacaa cagcagggga gacct 175
<210> 35
   <211> 15
   <212> DNA
   <213> artificial sequences
<220>
   <221> source
   <222> 1..15
   <223> /mol_type="DNA"
   /note="Sonde VHC 2a/2c"
   /organism="artificial sequences"
<400> 35
   ggactcctcr gttct 15
<210> 36
   <211> 15
   <212> DNA
   <213> artificial sequences
<220>
   <221> source
   <222> 1..15
   <223> /mol_type="DNA" /note="Sonde VHC 2b" /organism="artificial sequences"
<400> 36
   tatggattct tctgt 15
<210> 37
   <211> 15
   <212> DNA
   <213> artificial sequences
<220>
   <221> source
   <222> 1..15
   <223> /mol_type="DNA"
   /note="Cible VHC 2a/2c 15-mÃ¨res biotinylÃ©e"
   /organism="artificial sequences"
<400> 37
   agaacygagg agtcc 15
<210> 38
   <211> 15
   <212> DNA
   <213> artificial sequences
<220>
   <221> source
   <222> 1..15
   <223> /mol_type="DNA"
   /note="Cible VHC 2b 15-mÃ¨res biotinylÃ©e"
   /organism="artificial sequences"
<400> 38
   acagaagaat ccata 15
<210> 39
   <211> 15
   <212> DNA
   <213> artificial sequences
<220>
   <221> source
   <222> 1..15
   <223> /mol_type="DNA"
   /note="Cible VHC 4a/4d 15-mÃ¨res biotinylÃ©e"
   /organism="artificial sequences"
<400> 39
   ctaygtgggc ggycc 15
<210> 40
   <211> 14
   <212> DNA
   <213> artificial sequences
<220>
   <221> source
   <222> 1..14
   <223> /mol_type="DNA"
   /note="Sonde gÃ©nÃ©rique 3 flavivirus"
   /organism="artificial sequences"
<400> 40
   agccacatgw acca 14
<210> 41
   <211> 15
   <212> DNA
   <213> artificial sequences
<220>
   <221> source
   <222> 1..15
   <223> /mol_type="DNA"
   /note="Sonde spÃ©cifique Dengue 4"
   /organism="artificial sequences"
<400> 41
   cactccactc catga 15
<210> 42
   <211> 15
   <212> DNA
   <213> artificial sequences
<220>
   <221> source
   <222> 1..15
   <223> /mol_type="DNA"
   /note="Cible WNV 15-mÃ¨res biotinylÃ©e"
   /organism="artificial sequences"
<400> 42
   agaaytcagg aggmg 15
<210> 43
   <211> 13
   <212> DNA
   <213> artificial sequences
<220>
   <221> source
   <222> 1..13
   <223> /mol_type="DNA"
   /note="Cible gÃ©nÃ©rique 3 flavivirus 15-mÃ¨res biotinylÃ©e"
   /organism="artificial sequences"
<400> 43
   tggwcatgtg gct 13
<210> 44
   <211> 15
   <212> DNA
   <213> artificial sequences
<220>
   <221> source
   <222> 1..15
   <223> /mol_type="DNA"
   /note="Cible spÃ©cifique Dengue 4 15-mÃ¨res biotinylÃ©e"
   /organism="artificial sequences"
<400> 44
   tcatggagtg gagtg 15